# EUROPEAN PATENT SPECIFICATION

(11) **EP 1 044 001 B1**
(45) Date of publication and mention of the grant of the patent: **06.07.2005**
(21) Application number: 98963893.7
(22) Date of filing: 14.12.1998
(51) Int. Cl.: A61K 31/495, A61K 31/445, C07D 417/14, C07D 401/14, C07D 401/06, C07D 471/04, A61P 19/10

(54) **INTEGRIN RECEPTOR ANTAGONISTS**
INTEGRINREZEPTOR ANTAGONISTEN
ANTAGONISTES DU RECEPTEUR DE L'INTEGRINE

(30) Priority: 17.12.1997 US 69909 P; 06.04.1998 GB 9807384; 27.04.1998 US 83250 P; 13.07.1998 US 92630 P; 21.07.1998 GB 9815803
(43) Date of publication of application: 18.10.2000
(73) Proprietor: MERCK & CO., INC., Rahway, New Jersey 07065 (US)
(72) Inventor: DUGGAN, Mark, E., Rahway, NJ 07065 (US); PERKINS, James, J., Rahway, NJ 07065 (US); MEISSNER, Robert, S., Rahway, NJ 07065 (US)
(74) Representative: Horgan, James Michael Frederic
(86) International application number: PCT/US1998/026485
(87) International publication number: WO 1999/030713

(56) References cited:
- US-A- 4 929 618
- RICOUART J.C. et al., "Design of Potent Protein Kinase Inhibitors Using the Bisubstrate Approach", J. MED. CHEM., 1991, Vol. 34, No. 1, pages 73-78. XP002918324

## Description

### CROSS-REFERENCE TO RELATED APPLICATIONS

The present invention is related to U.S. provisional applications Serial No. 60/069,909, filed December 17, 1997; 60/083,250, filed April 27, 1998; and 60/092,630, filed July 13, 1998; the contents of each of which are hereby incorporated by reference.

### FIELD OF THE INVENTION

The present invention relates to compounds and derivatives thereof, their synthesis, and their use as integrin receptor antagonists. More particularly, the compounds of the present invention are antagonists of the integrin receptors ανβ3, ανβ5, and/or ανβ6 and are useful for inhibiting bone resorption, treating and preventing osteoporosis, and inhibiting vascular restenosis, diabetic retinopathy, macular degeneration, angiogenesis, atherosclerosis, inflammation, wound healing, viral disease, tumor growth, and metastasis.

### BACKGROUND OF THE INVENTION

It is believed that a wide variety of disease states and conditions can be mediated by acting on integrin receptors and that integrin receptor antagonists represent a useful class of drugs. Integrin receptors are heterodimeric transmembrane proteins through which cells attach and communicate with extracellular matrices and other cells *(See* S.B. Rodan and G.A. Rodan, "Integrin Function In Osteoclasts", *Journal of Endocrinology,* Vol. 154, S47- S56 (1997), which is incorporated by reference herein in its entirety).

In one aspect of the present invention, the compounds herein are useful for inhibiting bone resorption. Bone resorption is mediated by the action of cells known as osteoclasts. Osteoclasts are large multinucleated cells of up to about 400 mm in diameter that resorb mineralized tissue, chiefly calcium carbonate and calcium phosphate, in vertebrates. Osteoclasts are actively motile cells that migrate along the surface of bone, and can bind to bone, secrete necessary acids and proteases, thereby causing the actual resorption of mineralized tissue from the bone. More specifically, osteoclasts are believed to exist in at least two physiological states, namely, the secretory state and the migratory or motile state. In the secretory state, osteoclasts are flat, attach to the bone matrix via a tight attachment zone (sealing zone), become highly polarized, form a ruffled border, and secrete lysosomal enzymes and protons to resorb bone. The adhesion of osteoclasts to bone surfaces is an important initial step in bone resorption. In the migratory or motile state, the osteoclasts migrate across bone matrix and do not take part in resorption until they again attach to bone.

Integrins are involved in osteoclast attachment, activation and migration. The most abundant integrin in osteoclasts, e.g., in rat, chicken, mouse and human osteoclasts, is an integrin receptor known as ανβ3, which is thought to interact in bone with matrix proteins that contain the RGD sequence. Antibodies to ανβ3 block bone resorption *in vitro* indicating that this integrin plays a key role in the resorptive process. There is increasing evidence to suggest that ανβ3 ligands can be used effectively to inhibit osteoclast mediated bone resorption *in vivo* in mammals.

The current major bone diseases of public concern are osteoporosis, hypercalcemia of malignancy, osteopenia due to bone metastases, periodontal disease, hyperparathyroidism, periarticular erosions in rheumatoid arthritis, Paget's disease, immobilization-induced osteopenia, and glucocorticoid-induced osteoporosis. All of these conditions are characterized by bone loss, resulting from an imbalance between bone resorption, i.e. breakdown, and bone formation, which continues throughout life at the rate of about 14% per year on the average. However, the rate of bone turnover differs from site to site; for example, it is higher in the trabecular bone of the vertebrae and the alveolar bone in the jaws than in the cortices of the long bones. The potential for bone loss is directly related to turnover and can amount to over 5% per year in vertebrae immediately following menopause, a condition which leads to increased fracture risk.

In the United States, there are currently about 20 million people with detectable fractures of the vertebrae due to osteoporosis. In addition, there are about 250,000 hip fractures per year attributed to osteoporosis. This clinical situation is associated with a 12% mortality rate within the first two years, while 30% of the patients require nursing home care after the fracture.

Individuals suffering from all the conditions listed above would benefit from treatment with agents which inhibit bone resorption.

Additionally, ανβ3 ligands have been found to be useful in treating and/or inhibiting restenosis, i.e. recurrence of stenosis after corrective surgery on the heart valve, atherosclerosis, diabetic retinopathy, macular degeneration, and angiogenesis, i.e. formation of new blood vessels. Moreover, it has been postulated that the growth of tumors depends on an adequate blood supply, which in turn is dependent on the growth of new vessels into the tumor; thus, inhibition of angiogenesis can cause tumor regression in animal models (*See* Harrison's Principles of Internal Medicine, 12th ed., 1991, which is incorporated by reference herein in its entirety). Therefore, ανβ3 antagonists which inhibit angiogenesis can be useful in the treatment of cancer by inhibiting tumor growth (*See* e.g., Brooks *et al., Cell, 79*:1157-1164 (1994), which is incorporated by reference herein in its entirety).

Moreover, compounds of this invention can also inhibit neovascularization by acting as antagonists of the integrin receptor, ανβ5. A monoclonal antibody for ανβ5 has been shown to inhibit VEGF-induced angiogenesis in rabbit cornea and the chick chorioallantoic membrane model (*See* M.C. Friedlander, *et al., Science 270,* 1500-1502, (1995), which is incorporated by reference herein in its entirety). Thus, compounds that antagonize ανβ5 are useful for treating and preventing macular degeneration, diabetic retinopathy, tumor growth, and metastasis.

Additionally, compounds of the instant invention can inhibit angiogenesis and inflammation by acting as antagonists of the integrin receptor, αvβ6, which is expressed during the later stages of wound healing and remains expressed until the wound is closed (*See* Christofidou-Solomidou, et al., "Expression and Function of Endothelial Cell αv Integrin Receptors in Wound-Induced Human Angiogenesis in Human Skin/SCID Mice Chimeras, American Journal of Pathology, Vol. 151, No. 4, pp. 975-983 (October 1997), which is incorporated by reference herein in its entirety). It is postulated that αvβ6 plays a role in the remodeling of the vasculature during the later stages of angiogenesis. Also, αvβ6 participates in the modulation of epithelial inflammation and is induced in response to local injury or inflammation (*See* Xiao-Zhu Huang, et al., "Inactivation of the Integrin β6 Subunit Gene Reveals a Role of Epithelial Integrins in Regulating Inflammation in the Lungs and Skin," Journal of Cell Biology, Vol. 133, No.4, pp. 921-928 (May 1996), which is incorporated by reference herein in its entirety). Accordingly, compounds that antagonize αvβ6 are useful in treating or preventing cancer by inhibiting tumor growth and metastasis.

In addition, certain compounds of this invention antagonize both the ανβ3 and ανβ5 receptors. These compounds, referred to as "dual ανβ3/ανβ5 antagonists," are useful for inhibiting bone resorption, treating and preventing osteoporosis, and inhibiting vascular restenosis, diabetic retinopathy, macular degeneration, angiogenesis, atherosclerosis, inflammation, tumor growth, and metastasis.

In addition, certain compounds of this invention are useful as mixed αvβ3, αvβ5, and αvβ6 receptor antagonists.

It is therefore an object of the present invention to provide compounds which are useful as integrin receptor antagonists.

It is another object of the present invention to provide compounds which are useful as ανβ3 receptor antagonists.

It is another object of the present invention to provide compounds which are useful as ανβ5 receptor antagonists.

It is another object of the present invention to provide compounds which are useful as αvβ6 receptor antagonists.

It is another object of the present invention to provide compounds which are useful as dual ανβ3/ανβ5 receptor antagonists.

It is another object of the present invention to provide compounds which are useful as mixed αvβ3, αvβ5, and αvβ6 receptor antagonists.

It is another object of the present invention to provide pharmaceutical compositions comprising integrin receptor antagonists.

It is another object of the present invention to provide methods for making the pharmaceutical compositions of the present invention.

It is another object of the present invention to provide methods for eliciting an integrin receptor antagonizing effect in a mammal in need thereof by administering the compounds and pharmaceutical compositions of the present invention.

It is another object of the present invention to provide compounds and pharmaceutical compositions useful for inhibiting bone resorption, restenosis, atherosclerosis, inflammation, viral disease, diabetic retinopathy, macular degeneration, angiogenesis, tumor growth, and metastasis.

It is another object of the present invention to provide compounds and pharmaceutical compositions useful for treating osteoporosis.

It is another object of the present invention to provide methods for inhibiting bone resorption, restenosis, atherosclerosis, inflammation, viral disease, diabetic retinopathy, macular degeneration, angiogenesis, tumor growth, and metastasis.

It is another object of the present invention to provide methods for treating osteoporosis.

These and other objects will become readily apparent from the detailed description which follows.

### SUMMARY OF THE INVENTION

The present invention relates to compounds of the formula 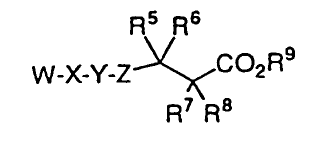 wherein W is selected from the group consisting of 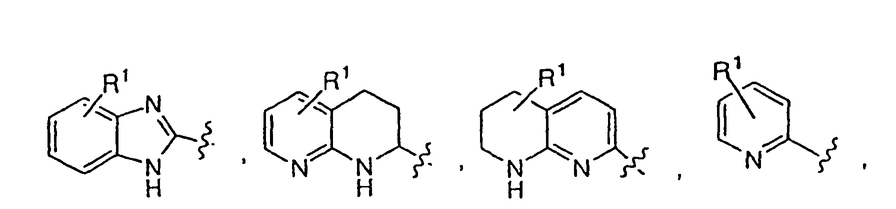 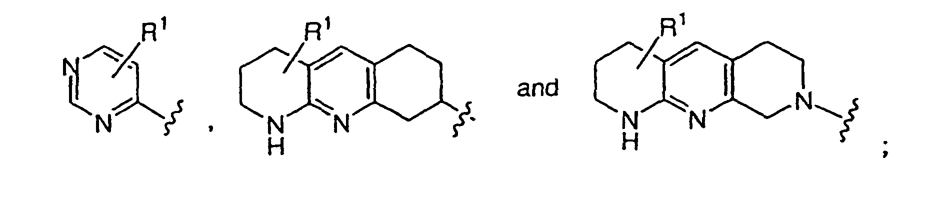
X is -(CH₂)ᵥ-, wherein any methylene (CH₂) carbon atom is either unsubstituted or substituted with one or two R¹ substitutents;
Y is selected from the group consisting of

   -(CH₂)ₘ-,

   -(CH₂)ₘ-O-(CH₂)ₙ-,

   -(CH₂)ₘ-NR⁴-(CH₂)ₙ-,

   -(CH₂)ₘ-S-(CH₂)ₙ-,

   -(CH₂)ₘ-SO-(CH₂)ₙ-,

   -(CH₂)ₘ-SO₂-(CH₂)ₙ-,

   -(CH₂)ₘ-O-(CH₂)ₙ-O-(CH₂)ₚ-,

   -(CH₂)ₘ-O-(CH₂)ₙ-NR⁴-(CH₂)ₚ -,

   -(CH₂)ₘ-NR⁴-(CH₂)ₙ-NR⁴-(CH₂)ₚ -,

   -(CH₂)ₘ-O-(CH₂)ₙ-S-(CH₂)ₚ -,

   -(CH₂)ₘ-S-(CH₂)ₙ-S-(CH₂)ₚ -,

   -(CH₂)ₘ-NR⁴-(CH₂)ₙ-S-(CH₂)ₚ -,

   -(CH₂)ₘ-NR⁴-(CH₂)ₙ-O-(CH₂)ₚ -,

   -(CH₂)ₘ-S-(CH₂)ₙ-O-(CH₂)ₚ -,

   and

   -(CH₂)ₘ-S-(CH₂)ₙ-NR⁴-(CH₂)ₚ -,

   wherein any methylene (CH₂) carbon atom in Y, other than in R⁴, can be substituted by one or two R³ substituents;
Z is selected from the group consisting of 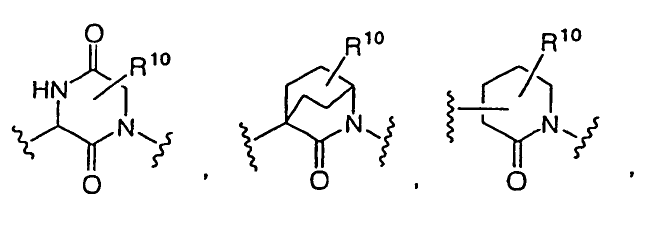 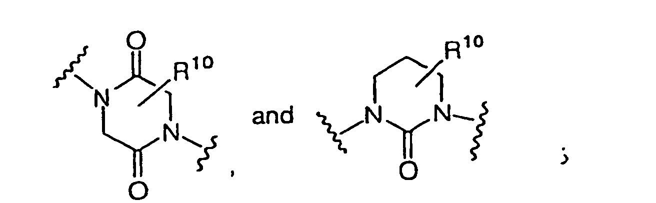
R¹ is selected from the group consisting of
   hydrogen,
   halogen,
   C₁₋₁₀ alkyl,
   C₃₋₈ cycloalkyl,
   C₃₋₈ cycloheteroalkyl,
   hydroxy,
   nitro,
   cyano,
   trifluoromethyl, and
   trifluoromethoxy;
each R³ is independently selected from the group consisting of
   hydrogen,
   fluoro,
   trifluoromethyl,
   aryl,
   C₁₋₈ alkyl,
   arylC₁₋₆ alkyl
   hydroxyl,
   oxo,
   arylaminocarbonyl,
   aryl C₁₋₅ alkylaminocarbonyl,
   aminocarbonyl, and
   aminocarbonyl C₁₋₆ alkyl;
each R⁴ is independently selected from the group consisting of
   hydrogen,
   aryl,
   C₃₋₈ cycloalkyl,
   C₁₋₈ alkyl,
   C₁₋₈ alkylcarbonyl,
   arylcarbonyl,
   C₁₋₆ alkylsulfonyl,
   arylsulfonyl,
   arylC₁₋₆alkylsulfonyl,
   arylC₁₋₆alkylcarbonyl,
   C₁₋₈alkylaminocarbonyl,
   arylC₁₋₅alkylaminocarbonyl,
   arylC₁₋₈alkoxycarbonyl, and
   C₁₋₈alkoxycarbonyl;
R⁵ and R⁶ are each independently selected from the group consisting of
   hydrogen,
   aryl,
   C₁₋₈ alkyl,
   aryl-C≡C-(CH₂)ₜ-,
   aryl C₁₋₆ alkyl,
   CH₂=CH-(CH₂)ₜ-, and
   HC≡C-(CH₂)ₜ-;
R⁷ and R⁸ are each independently selected from the group consisting of
   hydrogen,
   aryl,
   C₁₋₈ alkylcarbonylamino,
   arylcarbonylamino,
   C₁₋₈ alkylsulfonylamino,
   arylsulfonylamino,
   C₁₋₈ alkylsulfonylamino C₁₋₆ alkyl,
   arylsulfonylamino C₁₋₆ alkyl,
   aryl C₁₋₆ alkylsulfonylamino,
   aryl C₁₋₆ alkylsulfonylamino C₁₋₆ alkyl,
   C₁₋₈ alkoxycarbonylamino,
   C₁₋₈ alkoxycarbonylamino C₁₋₈ alkyl,
   aryloxycarbonylamino C₁₋₈ alkyl,
   aryl C₁₋₈ alkoxycarbonylamino,
   aryl C₁₋₈ alkoxycarbonylamino C₁₋₈ alkyl,
   C₁₋₈ alkylcarbonylamino C₁₋₆ alkyl,
   arylcarbonylamino C₁₋₆ alkyl,
   aryl C₁₋₆ alkylcarbonylamino,
   aryl C₁₋₆ alkylcarbonylamino C₁₋₆ alkyl,
   aminocarbonylamino C₁₋₆ alkyl,
   (C₁₋₈ alkyl)ₚaminocarbonylamino,
   (C₁₋₈ alkyl)ₚaminocarbonylamino C₁₋₆ alkyl,
   (aryl)ₚaminocarbonylamino C₁₋₆ alkyl,
   (aryl C₁₋₈ alkyl)ₚaminocarbonylamino,
   (aryl C₁₋₈ alkyl)ₚaminocarbonylamino C₁₋₆ alkyl,
   aminosulfonylamino C₁₋₆ alkyl,
   (C₁₋₈ alkyl)ₚaminosulfonylamino,
   (C₁₋₈ alkyl)ₚaminosulfonylamino C₁₋₆ alkyl,
   (aryl)paminosulfonylamino C₁₋₆ alkyl,
   (aryl C₁₋₈ alkyl)ₚaminosulfonylamino,
   (aryl C₁₋₈ alkyl)ₚaminosulfonylamino C₁₋₆ alkyl,
   C₁₋₆ alkylthiocarbonylamino,
   C₁₋₆ alkylthiocarbonylamino C₁₋₆ alkyl,
   arylthiocarbonylamino C₁₋₆ alkyl,
   aryl C₁₋₆ alkylthiocarbonylamino, and
   aryl C₁₋₆ alkylthiocarbonylamino C₁₋₆ alkyl;
R⁹ is selected from the group consisting of
   hydrogen, methyl, and ethyl;
R¹⁰ is selected from the group consisting of
   hydrogen, and
   C₁₋₈ alkyl;
   each m is independently an integer from 0 to 6;
   each n is independently an integer from 0 to 6;
   each p is independently an integer from 0 to 2;
   each t is an integer from 0 to 3;
   v is independently an integer from 0 to 6; and
   "aryl," as used herein, refers to a monocyclic or polycyclic system comprising at least one aromatic ring, wherein the monocylic or polycyclic system contains 0, 1, 2, 3, or 4 heteroatoms chosen from N, O, or S, and wherein the monocylic or polycylic system is either unsubstituted or substituted with one or more groups independently selected from hydrogen, halogen, C₁₋₁₀ alkyl, C₃₋₈ cycloalkyl, aryl, aryl C₁₋₈ alkyl, amino, amino C₁₋₈ alkyl, C₁₋₃ acylamino, C₁₋₃ acylamino C₁₋₈ alkyl, C₁₋₆ alkylamino, C₁₋₆ alkylamino C₁₋₈ alkyl, C₁₋₆ dialkylamino, C₁₋₆ dialkylamino-C₁₋₈ alkyl, C₁₋₄ alkoxy, C₁₋₄ alkoxy C₁₋₆ alkyl, hydroxycarbonyl, hydroxycarbonyl C₁₋₆ alkyl, C₁₋₅ alkoxycarbonyl, C₁₋₃ alkoxycarbonyl C₁₋₆ alkyl, hydroxycarbonyl C₁₋₆ alkyloxy, hydroxy, hydroxy C₁₋₆ alkyl, cyano, trifluoromethyl, oxo or C₁₋₅ alkylcarbonyloxy;
and the pharmaceutically acceptable salts thereof.

The present invention also relates to pharmaceutical compositions comprising the compounds of the present invention and a pharmaceutically acceptable carrier.

The present invention also relates to methods for making the pharmaceutical compositions of the present invention.

The present invention also relates to methods for eliciting an integrin receptor antagonizing effect in a mammal in need thereof by administering the compounds and pharmaceutical compositions of the present invention.

The present invention also relates to methods for inhibiting bone resorption, restenosis, atherosclerosis, inflammation, viral disease, diabetic retinopathy, macular degeneration, angiogenesis, wound healing, tumor growth, and metastasis by administering the compounds and pharmaceutical compositions of the present invention.

The present invention also relates to methods for treating osteoporosis by administering the compounds and pharmaceutical compositions of the present invention.

### DETAILED DESCRIPTION OF THE INVENTION

The present invention relates to compounds useful as integrin receptor antagonists. Representative compounds of the present invention are described by the following chemical formula: 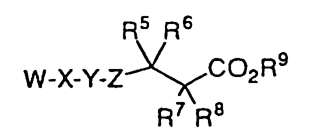 wherein W, X, Y, Z and R⁵-R⁹ are as defined above.

In the compounds of the present invention,

W is selected from the group consisting of

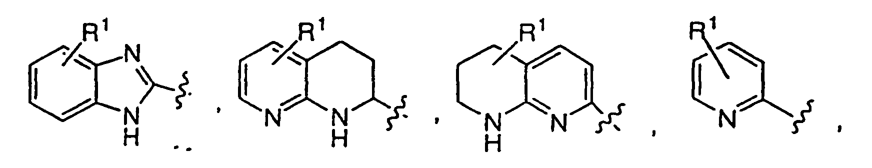

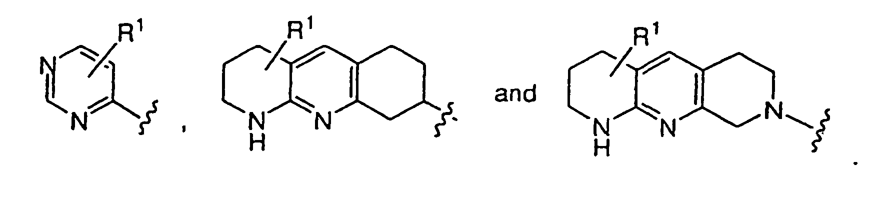

Most preferably W is

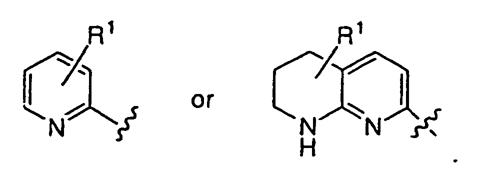

In the compounds of the present invention, X is -(CH₂)ᵥ-, wherein any methylene (CH₂) carbon atom is either unsubstituted or substituted with one or two R¹ substituents.

More preferably X is a direct bond, that is, v is 0.

In the compounds of the present invention, Y is preferably selected from the group consisting of

-(CH₂)ₘ-,

-(CH₂)ₘ-O-(CH₂)ₙ-,

-(CH₂)ₘ-NR⁴-(CH₂)ₙ-,

-(CH₂)ₘ-S-(CH₂)ₙ-,

-(CH₂)ₘ-SO-(CH₂)ₙ-,

-(CH₂)ₘ-SO₂-(CH₂)ₙ-,

-(CH2)m-O-(CH2)n-O-(CH2)p-,

-(CH2)m-O-(CH2)n-NR⁴-(CH2)p-,

-(CH2)m-NR⁴-(CH2)n-NR⁴-(CH2)p-,

and

-(CH2)m-NR⁴-(CH2)n-O-(CH2)p-,

wherein any methylene (CH₂) carbon atom in Y, other than in R⁴, can be substituted by one or two R³ substituents.

More preferably Y is selected from the group consisting of

(CH₂)ₘ, (CH₂)ₘ-S-(CH₂)ₙ, and (CH₂)ₘ-NR⁴-(CH₂)ₙ,

wherein any methylene (CH₂) carbon atom in Y, other than in R⁴, can be substituted by one or two R³ substituents.

In the compounds of the present invention,

Z is selected from the group consisting of

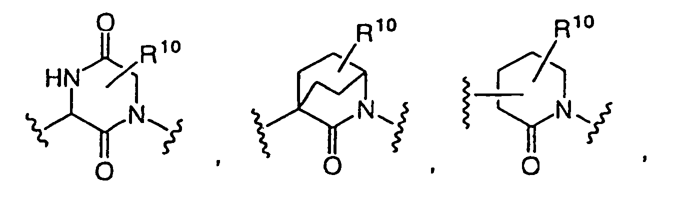

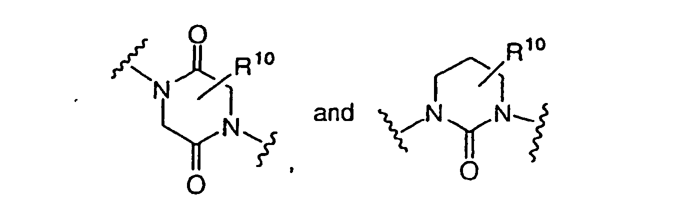

Most preferably Z is

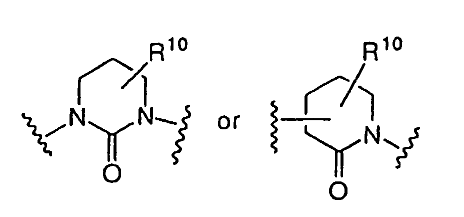

In the compounds of the present invention, R¹ is selected from the group consisting of hydrogen, halogen, C₁₋₁₀ alkyl, C₃₋₈ cycloalkyl, C₃₋₈ cycloheteroalkyl, hydroxy, nitro, cyano, trifluoromethyl, and trifluoromethoxy.

More preferably, R¹ is selected from the group consisting of hydrogen, halogen, C₁₋₁₀ alkyl, C₃₋₈ cycloalkyl, trifluoromethyl, and trifluoromethoxy.

In the compounds of the present invention, R³ is selected from the group consisting of
hydrogen,
fluoro,
trifluoromethyl,
aryl,
C₁₋₈ alkyl,
arylC₁₋₆ alkyl
hydroxyl,
oxo,
arylaminocarbonyl,
aryl C₁₋₅ alkylaminocarbonyl,
aminocarbonyl, and
aminocarbonyl C₁₋₆ alkyl.

More preferably, R³ is selected from the group consisting of
fluoro,
aryl,
C₁₋₈ alkyl,
arylC₁₋₆ alkyl
hydroxyl,
oxo, and
arylaminocarbonyl.

In the compounds of the present invention, R⁴ is selected from the group consisting of
hydrogen,
aryl,
C₃₋₈ cycloalkyl,
C₁₋₈ alkyl,
C₁₋₈ alkylcarbonyl,
arylcarbonyl,
C₁₋₆ alkylsulfonyl,
arylsulfonyl,
arylC₁₋₆alkylsulfonyl,
arylC₁₋₆alkylcarbonyl,
C₁₋₈alkylaminocarbonyl,
arylC₁₋₅alkylaminocarbonyl,
arylC₁₋₈alkoxycarbonyl, and
C₁₋₈alkoxycarbonyl.

More preferably, R⁴ is selected from the group consisting of hydrogen,
C₁₋₈ alkyl,
C₁₋₈ alkylcarbonyl,
arylcarbonyl,
arylC₁₋₆alkylcarbonyl,
C₁₋₆ alkylsulfonyl,
arylsulfonyl, and
arylC₁₋₆alkylsulfonyl.

R⁵ and R⁶ are each independently selected from the group consisting of
hydrogen,
aryl,
C₁₋₈ alkyl,
aryl-C≡C-(CH₂)ₜ-,
aryl C₁₋₆ alkyl,
CH₂=CH-(CH₂)ₜ-, and
HC≡C-(CH₂)ₜ-.

In a class of the present invention, R⁶ is hydrogen and R⁵ is selected from the group consisting of
hydrogen,
aryl,
C₁₋₈ alkyl,
aryl-C≡C-(CH₂)ₜ-,
aryl C₁₋₆ alkyl,
CH₂=CH-(CH₂)ₜ-, and
HC≡C-(CH₂)ₜ-.

In a subclass of this class of the present invention, R⁶, R⁷, and R⁸ are each hydrogen and R⁵ is selected from the group consisting of
hydrogen,
aryl,
C₁₋₈ alkyl,
aryl-C≡C-(CH₂)ₜ-,
aryl C₁₋₆ alkyl,
CH₂=CH-(CH₂)ₜ-, and
HC≡C-(CH₂)ₜ-.

In the present invention, R⁷ and R⁸ are each independently selected from the group consisting of
hydrogen,
aryl,
C₁₋₈ alkylcarbonylamino,
arylcarbonylamino,
C₁₋₈ alkylsulfonylamino,
arylsulfonylamino,
C₁₋₈ alkylsulfonylamino C₁₋₆ alkyl,
arylsulfonylamino C₁₋₆ alkyl,
aryl C₁₋₆ alkylsulfonylamino,
aryl C₁₋₆ alkylsulfonylamino C₁₋₆ alkyl,
C₁₋₈ alkoxycarbonylamino,
C₁₋₈ alkoxycarbonylamino C₁₋₈ alkyl,
aryloxycarbonylamino C₁₋₈ alkyl,
aryl C₁₋₈ alkoxycarbonylamino,
aryl C₁₋₈ alkoxycarbonylamino C₁₋₈ alkyl,
C₁₋₈ alkylcarbonylamino C₁₋₆ alkyl,
arylcarbonylamino C₁₋₆ alkyl,
aryl C₁₋₆ alkylcarbonylamino,
aryl C₁₋₆ alkylcarbonylamino C₁₋₆ alkyl,
aminocarbonylamino C₁₋₆ alkyl,
(C₁₋₈ alkyl)ₚaminocarbonylamino,
(C₁₋₈ alkyl)ₚaminocarbonylamino C₁₋₆ alkyl,
(aryl)ₚaminocarbonylamino C₁₋₆ alkyl,
(aryl C₁₋₈ alkyl)ₚaminocarbonylamino,
(aryl C₁₋₈ alkyl)ₚaminocarbonylamino C₁₋₆ alkyl,
aminosulfonylamino C₁₋₆ alkyl,
(C₁₋₈ alkyl)ₚaminosulfonylamino,
(C₁₋₈ alkyl)ₚaminosulfonylamino C₁₋₆ alkyl,
(aryl)ₚaminosulfonylamino C₁₋₆ alkyl,
(aryl C₁₋₈ alkyl)ₚaminosulfonylamino,
(aryl C₁₋₈ alkyl)ₚaminosulfonylamino C₁₋₆ alkyl,
C₁₋₆ alkylthiocarbonylamino,
C₁₋₆ alkylthiocarbonylamino C₁₋₆ alkyl,
arylthiocarbonylamino C₁₋₆ alkyl,
aryl C₁₋₆ alkylthiocarbonylamino, and
aryl C₁₋₆ alkylthiocarbonylamino C₁₋₆ alkyl.

In a class of the present invention, R⁸ is hydrogen and R⁷ is selected from the group consisting of consisting of
hydrogen,
aryl,
C₁₋₈ alkylcarbonylamino,
aryl C₁₋₆ alkylcarbonylamino,
arylcarbonylamino,
C₁₋₈ alkylsulfonylamino,
aryl C₁₋₆ alkylsulfonylamino,
arylsulfonylamino,
C₁₋₈ alkoxycarbonylamino,
aryl C₁₋₈ alkoxycarbonylamino,
arylaminocarbonylamino,
(C₁₋₈ alkyl)ₚaminocarbonylamino,
(aryl C₁₋₈ alkyl)ₚaminocarbonylamino,
(C₁₋₈ alkyl)ₚaminosulfonylamino, and
(aryl C₁₋₈ alkyl)ₚaminosulfonylamino.

In a subclass of this class of the present invention, R⁵, R⁶, and R⁸ are each hydrogen and R⁷ is selected from the group consisting of
hydrogen,
aryl,
C₁₋₈ alkylcarbonylamino,
aryl C₁₋₆ alkylcarbonylamino,
arylcarbonylamino,
C₁₋₈ alkylsulfonylamino,
aryl C₁₋₆ alkylsulfonylamino,
arylsulfonylamino,
C₁₋₈ alkoxycarbonylamino,
aryl C₁₋₈ alkoxycarbonylamino,
arylaminocarbonylamino,
(C₁₋₈ alkyl)ₚaminocarbonylamino,
(aryl C₁₋₈ alkyl)ₚaminocarbonylamino,
(C₁₋₈ alkyl)ₚaminosulfonylamino, and
(aryl C₁₋₈ alkyl)ₚaminosulfonylamino.

In the compounds of the present invention, R⁹ is selected from the group consisting of hydrogen, methyl, and ethyl.

More preferably, R⁹ is hydrogen.

In the compounds of the present invention, R¹⁰ is selected from the group consisting of hydrogen and C₁₋₈ alkyl.

More preferably R¹⁰ is hydrogen.

In the compounds of the present invention, m is preferably an integer from 0 to 4, more preferably from 0 to 3.

In the compounds of the present invention, n is preferably an integer from 0 to 4, more preferably from 0 to 3.

In the compounds of the present invention, t is preferably an integer from 0 to 2, more preferably from 0 to 1.

In the compounds of the present invention, v is preferably 0.

In certain embodiments of the present invention the compounds have the formula with the following designated stereochemistry: 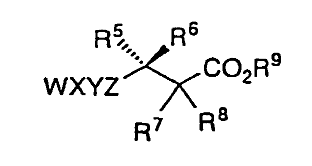 wherein the substituents W, X, Y, Z, R¹, R³, R⁴, R⁵, R⁶, R⁷, R⁸, R⁹, and R¹⁰, and the subscripts m, n, p, t, and v are as described above.

Illustrative but nonlimiting examples of compounds of the present invention that are useful as integrin receptor antagonists are the following:
ethyl 3(S)-(2,3-dihydro-benzofuran-6-yl)-3-{2-oxo-3-[3-(5,6,7,8-tetrahydro-[1,8]naphthyridin-2-yl)-propyl]-tetrahydro-pyrimidin-1-yl}-propionate;
ethyl 3(S)-(3-fluorophenyl)-3-(2-oxo-3(S or R)-[3-(5,6,7,8-tetrahydro-[1,8]naphthyridin-2-yl)-propyl]-piperidin-1-yl)-propionate;
ethyl 3(S)-(3-fluorophenyl)-3-(2-oxo-3(R or S)-[3-(5,6,7,8-tetrahydro-[1,8]naphthyridin-2-yl)-propyl]-piperidin-1-yl)-propionate;
3(S)-(2,3-dihydro-benzofuran-6-yl)-3-{2-oxo-3-[3-(5,6,7,8-tetrahydro-[1,8]naphthyridin-2-yl)-propyl]-tetrahydro-pyrimidin-1-yl}-propionic acid;
3(S)-(3-fluorophenyl)-3-(2-oxo-3(S or R)-[3-(5,6,7,8-tetrahydro-[1,8]naphthyridin-2-yl)-propyl]-piperidin-1-yl)-propionic acid;
3(S)-(3-fluorophenyl)-3-(2-oxo-3(R or S)-[3-(5,6,7,8-tetrahydro-[1,8]naphthyridin-2-yl)-propyl]-piperidin-1-yl)-propionic acid;
and the pharmaceutically acceptable salts thereof.

Further illustrative of the present invention are the compounds selected from the group consisting of:
3(S)-(2,3-dihydro-benzofuran-6-yl)-3-{2-oxo-3-[3-(5,6,7,8-tetrahydro-[1,8]naphthyridin-2-yl)-propyl]-tetrahydro-pyrimidin-1-yl}-propionic acid;
3(S)-(3-fluorophenyl)-3-(2-oxo-3(S or R)-[3-(5,6,7,8-tetrahydro-[1,8]naphthyridin-2-yl)-propyl]-piperidin-1-yl)-propionic acid;
3(S)-(3-fluorophenyl)-3-(2-oxo-3(R or S)-[3-(5,6,7,8-tetrahydro-[1,8]naphthyridin-2-yl)-propyl]-piperidin-1-yl)-propionic acid;
and the pharmaceutically acceptable salts thereof.

For use in medicine, the salts of the compounds of this invention refer to non-toxic "pharmaceutically acceptable salts." Other salts may, however, be useful in the preparation of the compounds according to the invention or of their pharmaceutically acceptable salts. Salts encompassed within the term "pharmaceutically acceptable salts" refer to non-toxic salts of the compounds of this invention which are generally prepared by reacting the free base with a suitable organic or inorganic acid. Representative salts include the following: acetate, benzenesulfonate, benzoate, bicarbonate, bisulfate, bitartrate, borate, bromide, calcium, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, oxalate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts.

The compounds of the present invention can have chiral centers and occur as racemates, racemic mixtures, diastereomeric mixtures, and as individual diastereomers, or enantiomers with all isomeric forms being included in the present invention. Therefore, where a compound is chiral, the separate enantiomers or diastereomers, substantially free of the other, are included within the scope of the invention; further included are all mixtures of the two enantiomers. Also included within the scope of the invention are polymorphs and hydrates of the compounds of the instant invention.

The present invention includes within its scope prodrugs of the compounds of this invention. In general, such prodrugs will be functional derivatives of the compounds of this invention which are readily convertible *in vivo* into the required compound. Thus, in the methods of treatment of the present invention, the term "administering" shall encompass the treatment of the various conditions described with the compound specifically disclosed or with a compound which may not be specifically disclosed, but which converts to the specified compound *in vivo* after administration to the patient. Conventional procedures for the selection and preparation of suitable prodrug derivatives are described, for example, in "Design of Prodrugs," ed. H. Bundgaard, Elsevier, 1985, which is incorporated by reference herein in its entirety. Metabolites of these compounds include active species produced upon introduction of compounds of this invention into the biological milieu.

The term "therapeutically effective amount" shall mean that amount of a drug or pharmaceutical agent that will elicit the biological or medical response of a tissue, system, animal or human that is being sought by a researcher or clinician.

The term "integrin receptor antagonist," as used herein, refers to a compound which binds to and antagonizes either the αvβ3 receptor, the αvβ5 receptor, or the αvβ6 receptor, or a compound which binds to and antagonizes combinations of these receptors (for example, a dual αvβ3/αvβ5 receptor antagonist).

The term "bone resorption," as used herein, refers to the process by which osteoclasts degrade bone.

The term "alkyl" shall mean straight or branched chain alkanes of one to ten total carbon atoms, or any number within this range (i.e., methyl, ethyl, 1-propyl, 2-propyl, n-butyl, s-butyl, t-butyl, etc.).

The term "alkenyl" shall mean straight or branched chain alkenes of two to ten total carbon atoms, or any number within this range.

The term "alkynyl" shall mean straight or branched chain alkynes of two to ten total carbon atoms, or any number within this range.

The term "cycloalkyl" shall mean cyclic rings of alkanes of three to eight total carbon atoms, or any number within this range (i.e., cyclopropyl, cyclobutyl, cyclopentyl, cyclohexyl, cycloheptyl or cyclooctyl).

The term "cycloheteroalkyl," as used herein, shall mean a 3- to 8-membered fully saturated heterocyclic ring containing one or two heteroatoms chosen from N, O or S. Examples of cycloheteroalkyl groups include, but are not limited to piperidinyl, pyrrolidinyl, azetidinyl, morpholinyl, piperazinyl.

The term "alkoxy," as used herein, refers to straight or branched chain alkoxides of the number of carbon atoms specified (e.g., C₁₋₅ alkoxy), or any number within this range (i.e., methoxy, ethoxy, etc.).

The term "aryl," as used herein, refers to a monocyclic or polycyclic system comprising at least one aromatic ring, wherein the monocylic or polycyclic system contains 0, 1, 2, 3, or 4 heteroatoms chosen from N, O, or S, and wherein the monocylic or polycylic system is either unsubstituted or substituted with one or more groups independently selected from hydrogen, halogen, C₁₋₁₀ alkyl, C₃₋₈ cycloalkyl, aryl, aryl C₁₋₈ alkyl, amino, amino C₁₋₈ alkyl, C₁₋₃ acylamino, C₁₋₃ acylamino C₁₋₈ alkyl, C₁₋₆ alkylamino, C₁₋₆ alkylamino C₁₋₈ alkyl, C₁₋₆ dialkylamino, C₁₋₆ dialkylamino-C₁₋₈ alkyl, C₁₋₄ alkoxy, C₁₋₄ alkoxy C₁₋₆ alkyl, hydroxycarbonyl, hydroxycarbonyl C₁₋₆ alkyl, C₁₋₅ alkoxycarbonyl, C₁₋₃ alkoxycarbonyl C₁₋₆ alkyl, hydroxycarbonyl C₁₋₆ alkyloxy, hydroxy, hydroxy C₁₋₆ alkyl, cyano, trifluoromethyl, oxo or C₁₋₅ alkylcarbonyloxy. Examples of aryl include, but are not limited to, phenyl, naphthyl, pyridyl, pyrazinyl, pyrimidinyl, imidazolyl, benzimidazolyl, benzthiazolyl, benzoxazolyl, indolyl, thienyl, furyl, pyrryl, pyrazolyl, dihydrobenzofuryl, benzo(1,3) dioxolane, oxazolyl, isoxazolyl and thiazolyl, which are either unsubstituted or substituted with one or more groups independently selected from hydrogen, halogen, C₁₋₁₀ alkyl, C₃₋₈ cycloalkyl, aryl, aryl C₁₋₈ alkyl, amino, amino C₁₋₈ alkyl, C₁₋₃ acylamino, C₁₋₃ acylamino C₁₋₈ alkyl, C₁₋₆ alkylamino, C₁₋₆ alkylamino-C₁₋₈ alkyl, C₁₋₆ dialkylamino, C₁₋₆ dialkylamino C₁₋₈ alkyl, C₁₋₄ alkoxy, C₁₋₄ alkoxy C₁₋₆ alkyl, hydroxycarbonyl, hydroxycarbonyl C₁₋₆ alkyl, C₁₋₅ alkoxycarbonyl, C₁₋₃ alkoxycarbonyl C₁₋₆ alkyl, hydroxycarbonyl C₁₋₆ alkyloxy, hydroxy, hydroxy C₁₋₆ alkyl, cyano, trifluoromethyl, oxo or C₁₋₅ alkylcarbonyloxy. Preferably, the aryl group is unsubstituted, mono-, di-, tri- or tetra-substituted with one to four of the above-named substituents; more preferably, the aryl group is unsubstituted, mono-, di- or tri-substituted with one to three of the above-named substituents; most preferably, the aryl group is unsubstituted, mono- or di-substituted with one to two of the above-named substituents.

Whenever the term "alkyl" or "aryl" or either of their prefix roots appear in a name of a substituent (e.g., aryl C₀₋₈ alkyl) it shall be interpreted as including those limitations given above for "alkyl" and "aryl." Designated numbers of carbon atoms (e.g., C₁₋₁₀) shall refer independently to the number of carbon atoms in an alkyl or cyclic alkyl moiety or to the alkyl portion of a larger substituent in which alkyl appears as its prefix root.

The terms "arylalkyl" and "alkylaryl" include an alkyl portion where alkyl is as defined above and to include an aryl portion where aryl is as defined above. Examples of arylalkyl include, but are not limited to, benzyl, fluorobenzyl, chlorobenzyl, phenylethyl, phenylpropyl, fluorophenylethyl, chlorophenylethyl, thienylmethyl, thienylethyl, and thienylpropyl. Examples of alkylaryl include, but are not limited to, toluene, ethylbenzene, propylbenzene, methylpyridine, ethylpyridine, propylpyridine and butylpyridine.

The term "halogen" shall include iodine, bromine, chlorine, and fluorine.

The term "oxy" means an oxygen (O) atom. The term "thio" means a sulfur (S) atom. The term "oxo" means "=O". The term "carbonyl" means "C=O."

The term "substituted" shall be deemed to include multiple degrees of substitution by a named substitutent. Where multiple substituent moieties are disclosed or claimed, the substituted compound can be independently substituted by one or more of the disclosed or claimed substituent moieties, singly or plurally. By independently substituted, it is meant that the (two or more) substituents can be the same or different.

Under standard nonmenclature used throughout this disclosure, the terminal portion of the designated side chain is described first, followed by the adjacent functionality toward the point of attachment. For example, a C₁₋₅ alkylcarbonylamino C₁₋₆ alkyl substituent is equivalent to

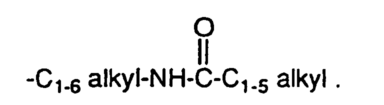

In choosing compounds of the present invention, one of ordinary skill in the art will recognize that the various substituents, i.e. W, X, Y, Z, R¹, R³, R⁴ R⁵, R⁶, R⁷, R⁸, R⁹, R¹⁰. and the subscripts m, n, p, t, and v are to be chosen in conformity with well-known principles of chemical structure connectivity.

Representative compounds of the present invention typically display submicromolar affinity for the integrin receptors, particularly the ανβ3, ανβ5, and/or ανβ6 receptors. Compounds of this invention are therefore useful for treating mammals suffering from a bone condition caused or mediated by increased bone resorption, who are in need of such therapy. Pharmacologically effective amounts of the compounds, including pharamaceutically acceptable salts thereof, are administered to the mammal, to inhibit the activity of mammalian osteoclasts.

The compounds of the present invention are administered in dosages effective to antagonize the αvβ3 receptor where such treatment is needed, as, for example, in the prevention or treatment of osteoporosis.

Further exemplifying the invention is the method wherein the integrin receptor antagonizing effect is an ανβ3 antagonizing effect. An illustration of the invention is the method wherein the ανβ3 antagonizing effect is selected from inhibition of bone resorption, restenosis, angiogenesis, diabetic retinopathy, macular degeneration, inflammation, viral disease, tumor growth, or metastasis. Preferably, the ανβ3 antagonizing effect is the inhibition of bone resorption.

An example of the invention is the method wherein the integrin receptor antagonizing effect is an ανβ5 antagonizing effect. More specifically, the ανβ5 antagonizing effect is selected from inhibition of restenosis, angiogenesis, diabetic retinopathy, macular degeneration, inflammation, tumor growth, or metastasis.

Illustrating the invention is the method wherein the integrin receptor antagonizing effect is a dual ανβ3/ανβ5 antagonizing effect. More particularly, the dual ανβ3/ανβ5 antagonizing effect is selected from inhibition of bone resorption, restenosis, angiogenesis, diabetic retinopathy, macular degeneration, inflammation, viral disease, tumor growth, or metastasis.

Illustrating the invention is the method wherein the integrin receptor antagonizing effect is an αvβ6 antagonizing effect. More particularly, the αvβ6 antagonizing effect is selected from inhibition of angiogenesis, inflammatory response, or wound healing.

Illustrating the invention is the method wherein the ανβ3 antagonizing effect is selected from inhibition of bone resorption, inhibition of restenosis, inhibition of angiogenesis, inhibition of diabetic retinopathy, inhibition of macular degeneration, inhibition of atherosclerosis, inflammation, viral disease, or inhibition of tumor growth or metastasis. Preferably, the αvβ3 antagonizing effect is the inhibition of bone resorption.

More particularly illustrating the invention is a pharmaceutical composition comprising any of the compounds described above and a pharmaceutically acceptable carrier. Another example of the invention is a pharmaceutical composition made by combining any of the compounds described above and a pharmaceutically acceptable carrier. Another illustration of the invention is a process for making a pharmaceutical composition comprising combining any of the compounds described above and a pharmaceutically acceptable carrier.

Further illustrating the invention is a method of treating and/or preventing a condition mediated by antagonism of an integrin receptor in a mammal in need thereof, comprising administering to the mammal a therapeutically effective amount of any of the compounds described above. Preferably, the condition is selected from bone resorption, osteoporosis, restenosis, diabetic retinopathy, macular degeneration, angiogenesis, atherosclerosis, inflammation, viral disease, cancer, tumor growth, and metastasis. More preferably, the condition is selected from osteoporosis and cancer. Most preferably, the condition is osteoporosis.

More specifically exemplifying the invention is a method of eliciting an integrin antagonizing effect in a mammal in need thereof, comprising administering to the mammal a therapeutically effective amount of any of the compounds or any of the pharmaceutical compositions described above. Preferably, the integrin antagonizing effect is an ανβ3 antagonizing effect; more specifically, the ανβ3 antagonizing effect is selected from inhibition of bone resorption, inhibition of restenosis, inhibition of atherosclerosis, inhibition of angiogenesis, inhibition of diabetic retinopathy, inhibition of macular degeneration, inhibition of inflammation, inhibition of viral disease, or inhibition of tumor growth or metastasis. Most preferably, the ανβ3 antagonizing effect is inhibition of bone resorption. Alternatively, the integrin antagonizing effect is an ανβ5 antagonizing effect, an ανβ6 antagonizing effect, or a mixed ανβ3, ανβ5, and ανβ6 antagonizing effect. Examples of ανβ5 antagonizing effects are inhibition of restenosis, atherosclerosis, angiogenesis, diabetic retinopathy, macular degeneration, inflammation, viral disease, or tumor growth. Examples of dual ανβ6 antagonizing effects are inhibition of angiogenesis, inflammatory response and wound healing.

Additional examples of the invention are methods of inhibiting bone resorption and of treating and/or preventing osteoporosis in a mammal in need thereof, comprising administering to the mammal a therapeutically effective amount of any of the compounds or any of the pharmaceutical compositions described above.

Additional illustrations of the invention are methods of treating hypercalcemia of malignancy, osteopenia due to bone metastases, periodontal disease, hyperparathyroidism, periarticular erosions in rheumatoid arthritis, Paget's disease, immobilization-induced osteopenia, and glucocorticoid treatment in a mammal in need thereof, comprising administering to the mammal a therapeutically effective amount of any of the compounds or any of the pharmaceutical compositions described above.

More particularly exemplifying the invention is the use of any of the compounds described above in the preparation of a medicament for the treatment and/or prevention of osteoporosis in a mammal in need thereof. Still further exemplifying the invention is the use of any of the compounds described above in the preparation of a medicament for the treatment and/or prevention of bone resorption, tumor growth, cancer, restenosis, atherosclerosis, diabetic retinopathy, macular degeneration, inflammation, viral disease, and/or angiogenesis.

Also exemplifying the invention are compositions further comprising an active ingredient selected from the group consisting of
a.) an organic bisphosphonate or a pharmaceutically acceptable salt or ester thereof,
b.) an estrogen receptor modulator,
c.) a cytotoxic/antiproliferative agent,
d.) a matrix metalloproteinase inhibitor,
e.) an inhibitor of epidermal-derived, fibroblast-derived, or platelet-derived growth factors,
f.) an inhibitor of VEGF,
g.) an inhibitor of Flk-1/KDR, Flt-1, Tck/Tie-2, or Tie-1,
h.) a cathepsin K inhibitor, and
i.) a prenylation inhibitor, such as a farnesyl transferase inhibitor or a geranylgeranyl transferase inhibitor or a dual farnesyl/geranylgeranyl transferase inhibitor; and mixtures thereof.
(See, B. Millauer et al., "Dominant-Negative Inhibition of Flk-1 Suppresses the Growth of Many Tumor Types in *Vivo*", Cancer Research, 56, 1615-1620 (1996), which is incorporated by reference herein in its entirety).

Preferably, the active ingredient is selected from the group consisting of:
a.) an organic bisphosphonate or a pharmaceutically acceptable salt or ester thereof,
b.) an estrogen receptor modulator, and
c.) a cathepsin K inhibitor, and mixtures thereof.

Nonlimiting examples of such bisphosphonates include alendronate, etidronate, pamidronate, risedronate, ibandronate, and pharmaceutically acceptable salts and esters thereof. A particularly preferred bisphosphonate is alendronate, especially alendronate monosodium trihydrate.

Nonlimiting examples of estrogen receptor modulators include estrogen, progesterin, estradiol, droloxifene, raloxifene, and tamoxifene.

Nonlimiting examples of cytotoxic/antiproliferative agents are taxol, vincristine, vinblastine, and doxorubicin.

Cathepsin K, formerly known as cathepsin O2, is a cysteine protease and is described in PCT International Application Publication No. WO 96/13523, published May 9, 1996; U.S. Patent No. 5,501,969, issued March 3, 1996; and U.S. Patent No. 5,736,357, issued April 7, 1998, all of which are incorporated by reference herein in their entirety. Cysteine proteases, specifically cathepsins, are linked to a number of disease conditions, such as tumor metastasis, inflammation, arthritis, and bone remodeling. At acidic pH's, cathepsins can degrade type-I collagen. Cathepsin protease inhibitors can inhibit osteoclastic bone resorption by inhibiting the degradation of collagen fibers and are thus useful in the treatment of bone resorption diseases, such as osteoporosis.

The present invention is also directed to combinations of the compounds of the present invention with one or more agents useful in the prevention or treatment of osteoporosis. For example, the compounds of the instant invention may be effectively administered in combination with effective amounts of other agents such as an organic bisphosphonate; an estrogen receptor modulator, or a cathepsin K inhibitor.

Additional illustrations of the invention are methods of treating tumor growth in a mammal in need thereof, comprising administering to the mammal a therapeutically effective amount of a compound described above and one or more agents known to be cytotoxic/antiproliferative. Also, the compounds of the present invention can be administered in combination with radiation therapy for treating tumor growth and metastasis.

In addition, the integrin ανβ3 antagonist compounds of the present invention may be effectively administered in combination with a growth hormone secretagogue in the therapeutic or prophylactic treatment of disorders in calcium or phosphate metabolism and associated diseases. These diseases include conditions which can benefit from a reduction in bone resorption. A reduction in bone resorption should improve the balance between resorption and formation, reduce bone loss or result in bone augmentation. A reduction in bone resorption can alleviate the pain associated with osteolytic lesions and reduce the incidence and/or growth of those lesions. These diseases include: osteoporosis (including estrogen deficiency, immobilization, glucocorticoid induced and senile), osteodystrophy, Paget's disease, myositis ossificans, Bechterew's disease, malignant hypercalcemia, metastatic bone disease, periodontal disease, cholelithiasis, nephrolithiasis, urolithiasis, urinary calculus, hardening of the arteries (sclerosis), arthritis, bursitis, neuritis and tetany. Increased bone resorption can be accompanied by pathologically high calcium and phosphate concentrations in the plasma, which would be alleviated by this treatment. Similarly, the present invention would be useful in increasing bone mass in patients with growth hormone deficiency. Thus, preferred combinations are simultaneous or alternating treatments of an ανβ3 receptor antagonist of the present invention and a growth hormone secretagogue, optionally including a third component comprising an organic bisphosphonate, preferably alendronate monosodium trihydrate.

In accordance with the method of the present invention, the individual components of the combination can be administered separately at different times during the course of therapy or concurrently in divided or single combination forms. The instant invention is therefore to be understood as embracing all such regimes of simultaneous or alternating treatment, and the term "administering" is to be interpreted accordingly. It will be understood that the scope of combinations of the compounds of this invention with other agents useful for treating integrin-mediated conditions includes in principle any combination with any pharmaceutical composition useful for treating osteoporosis.

As used herein, the term "composition" is intended to encompass a product comprising the specified ingredients in the specified amounts, as well as any product which results, directly or indirectly, from combination of the specified ingredients in the specified amounts.

The compounds of the present invention can be administered in such oral dosage forms as tablets, capsules (each of which includes sustained release or timed release formulations), pills, powders, granules, elixirs, tinctures, suspensions, syrups and emulsions. Likewise, they may also be administered in intravenous (bolus or infusion), intraperitoneal, topical (e.g., ocular eyedrop), subcutaneous, intramuscular or transdermal (e.g., patch) form, all using forms well known to those of ordinary skill in the pharmaceutical arts. An effective but non-toxic amount of the compound desired can be employed as an ανβ3 antagonist.

The dosage regimen utilizing the compounds of the present invention is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician, veterinarian or clinician can readily determine and prescribe the effective amount of the drug required to prevent, counter or arrest the progress of the condition.

Oral dosages of the present invention, when used for the indicated effects, will range between about 0.01 mg per kg of body weight per day (mg/kg/day) to about 100 mg/kg/day, preferably 0.01 to 10 mg/kg/day, and most preferably 0.1 to 5.0 mg/kg/day. For oral administration, the compositions are preferably provided in the form of tablets containing 0.01, 0.05, 0.1, 0.5, 1.0, 2.5, 5.0, 10.0, 15.0, 25.0, 50.0, 100 and 500 milligrams of the active ingredient for the symptomatic adjustment of the dosage to the patient to be treated. A medicament typically contains from about 0.01 mg to about 500 mg of the active ingredient, preferably, from about 1 mg to about 100 mg of active ingredient. Intravenously, the most preferred doses will range from about 0.1 to about 10 mg/kg/minute during a constant rate infusion. Advantageously, compounds of the present invention may be administered in a single daily dose, or the total daily dosage may be administered in divided doses of two, three or four times daily. Furthermore, preferred compounds for the present invention can be administered in intranasal form via topical use of suitable intranasal vehicles, or via transdermal routes, using those forms of transdermal skin patches well known to those of ordinary skill in the art. To be administered in the form of a transdermal delivery system, the dosage administration will, of course, be continuous rather than intermittent throughout the dosage regimen.

In the methods of the present invention, the compounds herein described in detail can form the active ingredient, and are typically administered in admixture with suitable pharmaceutical diluents, excipients or carriers (collectively referred to herein as 'carrier' materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, gelatin, natural sugars such as glucose or betalactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamide-phenol, polyhydroxy-ethylaspartamide-phenol, or polyethyleneoxide-polylysine substituted with palmitoyl residues. Furthermore, the compounds of the present invention may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and crosslinked or amphipathic block copolymers of hydrogels.

In the schemes and examples below, various reagent symbols and abbreviations have the following meanings:
- AcOH:: Acetic acid.
- BH₃•DMS:: Borane•dimethylsulfide.
- BOC(Boc):: t-Butyloxycarbonyl.
- BOP:: Benzotriazol-1-yloxytris(dimethylamino)-phosphonium hexafluorophosphate.
- CBZ(Cbz):: Carbobenzyloxy or benzyloxycarbonyl.
- CDI:: Carbonyldiimidazole.
- CH₂Cl₂:: Methylene chloride.
- CH3CN:: Acetonitrile
- CHCl₃:: Chloroform.
- DEAD:: Diethyl azodicarboxylate.
- DIAD:: Diisopropyl azodicarboxylate.
- DIBAH or DIBAL-H:: Diisobutylaluminum hydride.
- DIPEA:: Diisopropylethylamine.
- DMAP:: 4-Dimethylaminopyridine.
- DME:: 1,2-Dimethoxyethane.
- DMF:: Dimethylformamide.
- DMSO:: Dimethylsulfoxide.
- DPFN:: 3,5-Dimethyl-1-pyrazolylformamidine nitrate.
- EDC:: 1-(3-Dimethylaminopropyl)-3-ethylcarbodiimide • HCl
- EtOAc:: Ethyl acetate.
- EtOH:: Ethanol.
- HOAc:: Acetic acid.
- HOAT:: 1-Hydroxy-7-azabenzotriazole
- HOBT:: 1-Hydroxybenzotriazole.
- IBCF:: Isobutylchloroformate
- LDA:: Lithium diisopropylamide.
- MeOH:: Methanol.
- MMNG: 1,1-methyl-3-nitro-1-nitrosoguanidine
- NEt₃:: Triethylamine.
- NMM:: N-methylmorpholine.
- PCA•HCl:: Pyrazole carboxamidine hydrochloride.
- Pd/C:: Palladium on activated carbon catalyst.
- Ph:: Phenyl.
- pTSA: p-Toluenesulfonic acid.
- TEA:: Triethylamine.
- TFA:: Trifluoroacetic acid.
- THF:: Tetrahydrofuran.
- TLC:: Thin Layer Chromatography.
- TMEDA:: N,N,N',N'-Tetramethylethylenediamine.
- TMS:: Trimethylsilyl.

The novel compounds of the present invention can be prepared according to the procedure of the following schemes and examples, using appropriate materials and are further exemplified by the following specific examples. The compounds illustrated in the examples are not, however, to be construed as forming the only genus that is considered as the invention. The following examples further illustrate details for the preparation of the compounds of the present invention. Those skilled in the art will readily understand that known variations of the conditions and processes of the following preparative procedures can be used to prepare these compounds. All temperatures are degrees Celsius unless otherwise noted.

The following Schemes and Examples describe procedures for making representative compounds of the present invention. Moreover, by utilizing the procedures described in detail in PCT International Application Publication Nos. WO95/32710, published 7 December 1995, and WO95/17397, published 29 June 1995, both of which are incorporated by reference herein in their entirety, in conjunction with the disclosure contained herein, one of ordinary skill in the art can readily prepare additional compounds of the present invention claimed herein. Additionally, for a general review describing the synthesis of β-alanines which can be utilized as the C-terminus of the compounds of the present invention, see Cole, D.C., *Recent Stereoselective Synthetic Approaces to β-Amino Acids, Tetrahedron,* 1994, *50,* 9517-9582; Juaristi, E, *et al., Enantioselective Synthesis of β-Amino Acids, Aldrichimica Acta*, 1994, *27*, 3. In particular, synthesis of the 3-methyl-β-alanine is taught in Duggan, M.F. *et al., J. Med. Chem*., 1995, *38*, 3332-3341; the 3-ethynyl-β-alanine is taught in Zablocki, J.A., *et al., J. Med. Chem.,* 1995, *38,* 2378-2394; the 3-(pyridin-3-yl)-β-alanine is taught in Rico, J.G. *et al., J. Org. Chem.,* 1993, *58*, 7948-7951; and the 2-amino- and 2-tosylamino-β-alanines are taught in Xue, C-B, *et al., Biorg. Med. Chem. Letts.,* 1996, *6*, 339-344. The references described in this paragraph are all also incorporated by reference herein in their entirety.

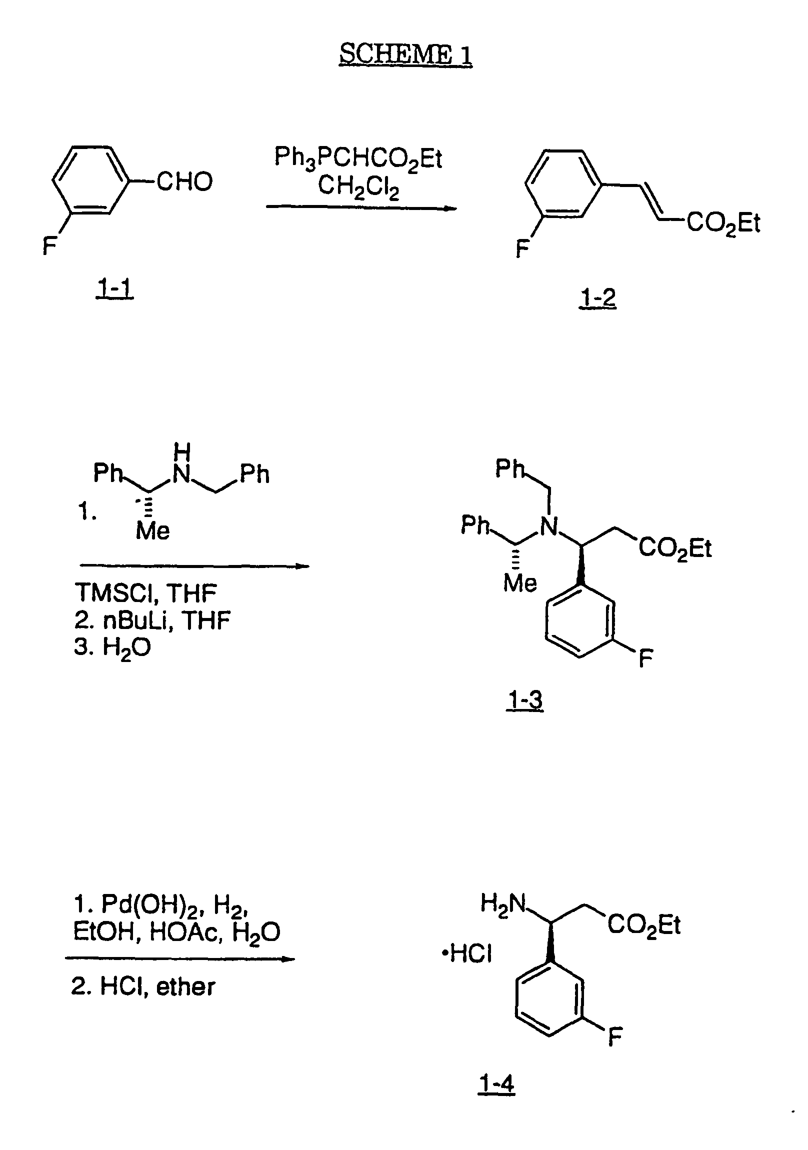

### Ethyl 3-fluorocinnamate (1-2)

To a solution of 3-fluorobenzaldehyde 1-1 (18.16 g, 146 mmol) in dichloromethane (500 mL) was added ethyl (triphenylphosphoranylidene)acetate (61.2 g; 176 mmol) and the resulting solution was stirred at room temperature for 18 hr. After evaporation of the solvent, the residue was swirled with ether/hexane and filtered. The filtrate was concentrated and then purified on a plug of silica gel eluting with hexane/EtOAc 9:1. Removal of the solvent afforded the title compound 1-2 as an oil (∼95% trans) which was used without further purification in the next step.
¹H NMR (CDCl₃): δ 1.36 (3H, t), 4.28 (2H, q), 6.43 (1H, d), 7.08 (1H, m), 7.2-7.4 (3H, m), 7.64 (1H, d).

### N-Benzyl-(R)-α-methylbenzyl-3(S)-fluorophenyl-β-alanine ethyl ester (1-3)

To a solution of N-benzyl-(R)-α-methylbenzylamine (33.4 g, 158 mmol) in THF (450 mL) at 0°C was added n-butyllithium (1.6M in hexanes; 99 mL, 158 mmol). The dark violet solution was stirred at 0°C for 30 minutes, cooled to -78°C and the ester
1-2 (29.2 g, 150 mmol) in THF (100 mL) was added over 5 minutes. The resulting solution was stirred at -78°C for 1 hr, then warmed to room temperature. After 2 hrs, the mixture was poured into water and extracted with EtOAc, washed with water, then brine, dried and concentrated *in vacuo* to give an oil. Column chromatography (silica gel; hexane/EtOAc 1:1 then pure EtOAc) gave the title compound 1-3.
¹H NMR (CDCl₃): δ 1.06 (3H, t), 1.28 (3H, d), 2.52 (1H, dd), 2.62 (1H, dd), 3.66 (1H, d), 3.72 (1H, d), 3.95 (2H, q), 4.44 (1H, dd), 6.95 (1H, m), 7.1-7.5 (13H, m).

### 3(S)-Fluorophenyl-β-alanine ethyl ester hydrochloride (1-4)

A solution of the N-benzyl-(R)-α-methylbenzylamine derivative 1-3 (28.2 g, 69.6 mmol) in ethanol (300 mL), acetic acid (30 mL) and water (3 mL) was degassed with argon for 30 minutes. Pd(OH)₂ on carbon (20% dry weight; 2.6 g) was added and the mixture then stirred under a hydrogen atmosphere (balloon) for 2 hours. The mixture was filtered through celite and the solvent removed *in vacuo* to give an oil. This oil was dissolved in 200 mL ether and to this solution was added 60 mL 1N HCl in ether to yield a precipitate. Filtration and washing the solid with ether/hexane then gave the title compound 1-4 as a white solid.
¹H NMR (CD₃OD): δ 1.21 (3H, t), 3.0-3.2 (2H, m), 4.16 (2H, q), 4.76 (1H, t), 7.2-7.35 (3H, m), 7.5 (1H, m).

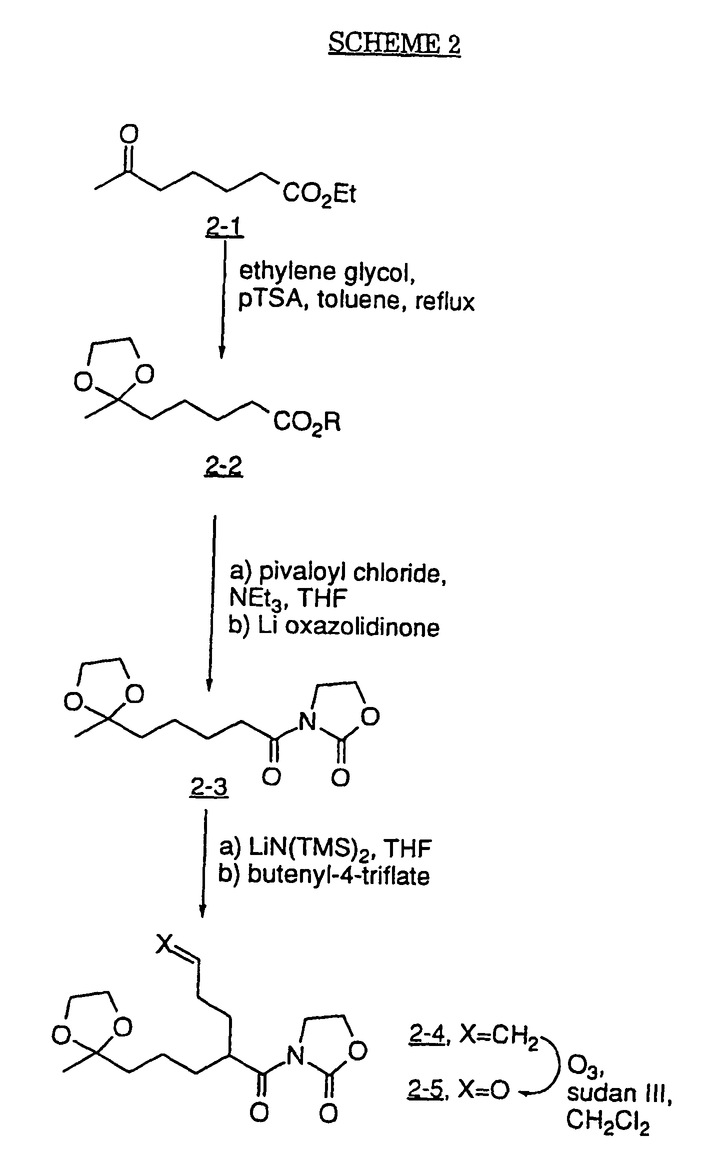

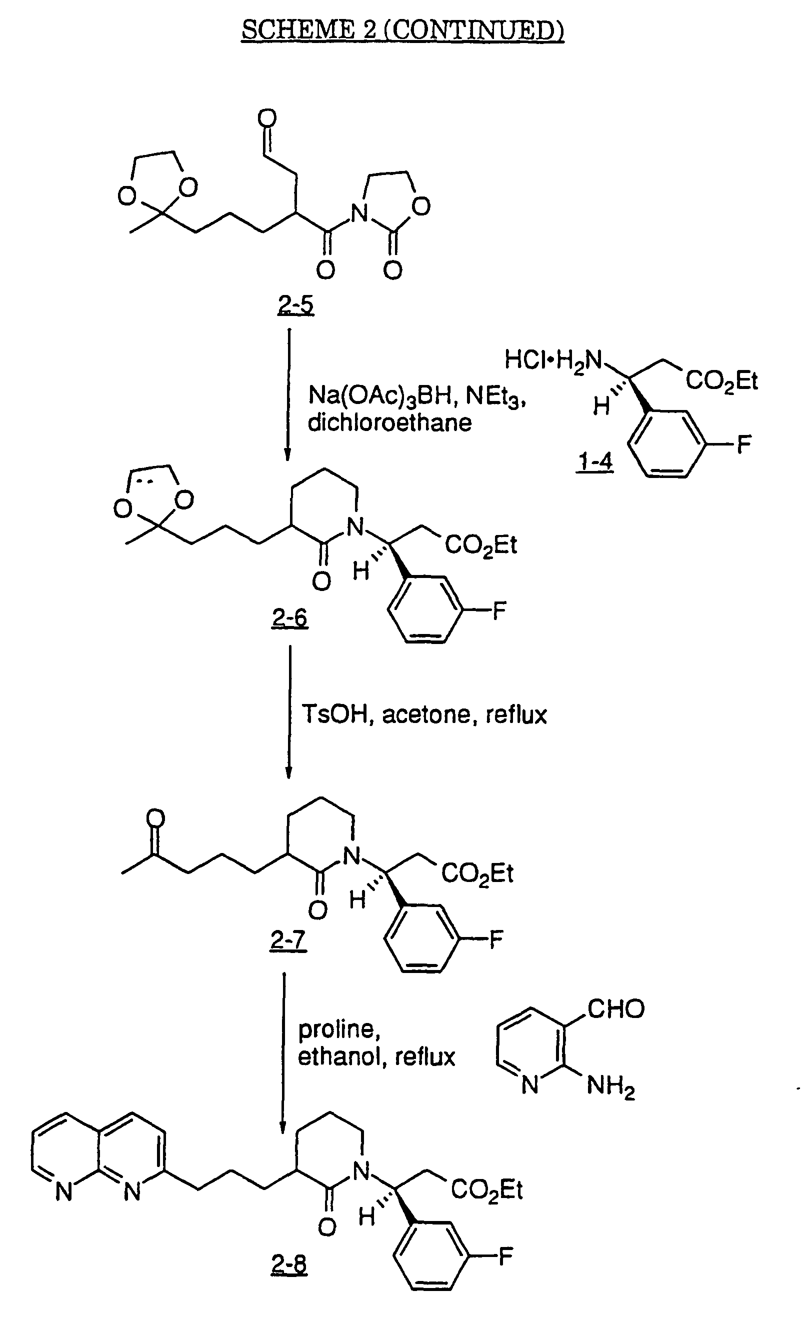

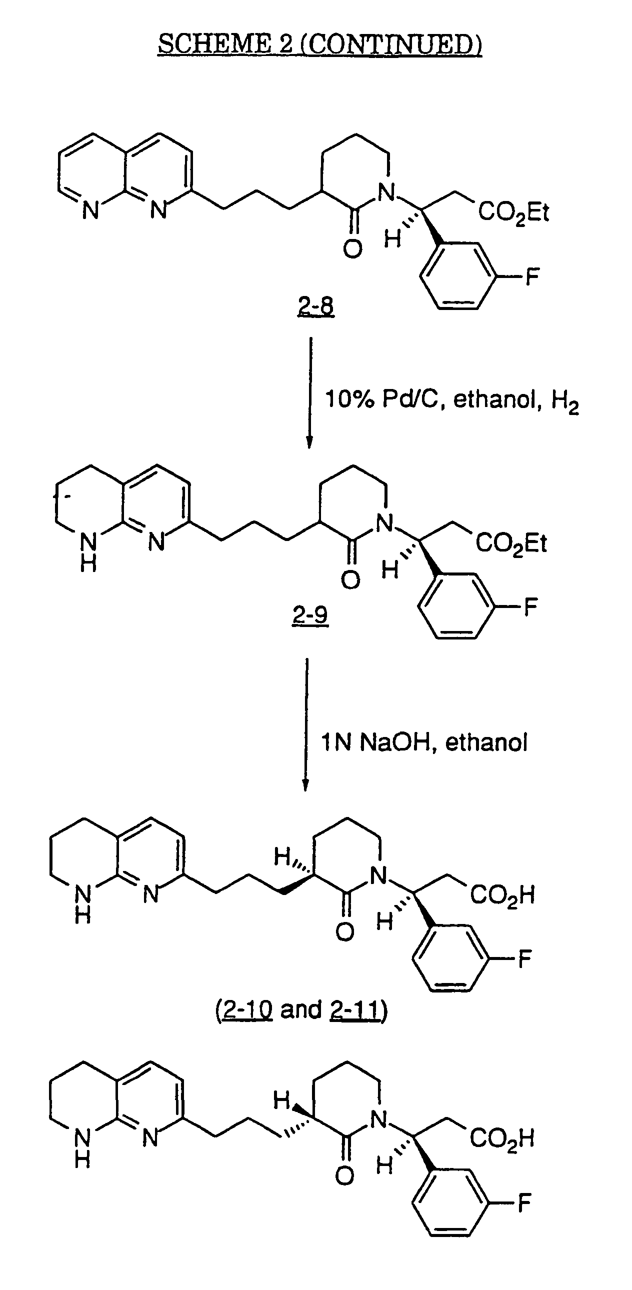

### 5-(2-Methyl-[1,3]dioxolan-2-yl)-pentanoic acid (2-2)

A mixture of ketone 2-1 (18 g, 105 mmol), ethylene glycol (3.2 ml, 110 mmol), p-TSA (50 mg, 0.2713 mmol) and toluene (300 mL) was heated to reflux with azeotropic removal of water for 24 hours. The reaction mixture was diluted with EtOAc and then washed with sat. NaHCO₃, brine, dried (MgSO₄), and concentrated. The residue was dissolved in EtOH (200 ml) and then treated with 1N NaOH (120 ml, 120 mmol). After 2 h, the reaction was poured into 600 mL 2:1 Et₂O/10% KHSO₄. The organic portion was separated, washed with brine, dried (MgSO₄) and concentrated to give acid 2-2 as a colorless oil.
¹H NMR (300 MHz, CDCl₃) δ 3.93 (m, 4H), 2.36 (m, 2H), 1.63 (m, 4H), 1.46 (m, 2H), 1.31 (s, 3H).

### 3-[5-(2-Methyl-[1,3]dioxolan-2-yl)-pentanoyl]-oxazolidin-2-one (2-3)

To a stirred solution of 2-2 (16.0 g, 85.5 mmol), NEt₃ (13.1 ml, 94.1 mmol) and THF (400 mL) at -78°C was added pivaloyl chloride (11.6 ml, 94.1 mmol). The mixture was warmed to 0°C for 1.0 h and then recooled to -78°C. To a stirred solution of 2-oxazolidinone (9.3g, 106.9 mmol) and THF (200 ml) at -78°C was added nBuLi (43.0ml, 106.9 mmol, 2.5M in hexanes) dropwise over 10 minutes. After 20 minutes, the lithium reagent was transferred to the mixed anhydride via cannula. After 10 minutes, the reaction was warmed to 0°C for 1.0h. The mixture was diluted with ethyl acetate, washed with sat. NaHCO₃, brine, and dried over MgSO₄. Following evaporative removal of the solvent, the residue was chromatographed (silica gel, 40%-50% EtOAc/hexanes) to give 2-3 as a colorless foam.
TLC R_{f} = 0.19 (silica, 40% EtOAc/hexanes)
¹H NMR (300 MHz, CDCl₃) δ 4.41 (t, J=8.1 Hz, 2H), 4.02 (t, J=8.1 Hz, 2H), 3.93 (m, 4H ), 3.93 (t, J=7.3 Hz, 2H), 1.66 (m, 4H), 1.48 (m, 2H), 1.31 (s, 3H).

### 3-(2-[3-(2-Methyl-[1,3]dioxolan-2-yl)-propyl]-hex-5-enoyl)-oxazolidin-2-one (2-4)

To a stirred solution of 2-3 (3.0 g, 11.7 mmol) and THF (125 mL) at -78°C was added NaN(TMS)₂ (15.2 mL, 15.2 mmol, 1.0 M in THF) dropwise over 20 minutes. Butenyl-4-triflate (5.0 mL, 29.2 mmol) was added dropwise; after 1 h, the reaction was warmed to 0°C. After 1 h, the reaction was diluted with EtOAc, washed with sat. NaHCO₃, brine, dried (MgSO₄) and concentrated. Flash chromatography (silica, 40% EtOAc/ hexanes) gave 2-4 as an yellow oil.
TLC R_{f} = 0.28 (silica, 50% EtOAc/hexanes)
¹H NMR (300 MHz, CDCl₃) δ 5.80 (m, 1H), 5.00 (m, 2H), 4.40 (m, 2H), 4.02 (m, 2H), 3.92 (m, 4H), 2.40-1.35 (m, 11H), 1.30 (s, 3H).

### 7-(2-Methyl-[1,3]dioxolan-2-yl)-4-(2-oxo-oxazolidine-3-carbonyl)-heptanal (2-5)

To a stirred solution of 2-4 (1.8 g, 5.8 mmol), sudan III (10 mg) and CH₂Cl₂ (150 mL) at -78°C under argon was bubbled ozone until the red solution changed to yellow-orange. The solution was purged with argon for 30 minutes. PPh₃ (2.26 g, 8.7 mmol) was added followed by the removal of the cooling bath. After 1.5 h, the reaction was concentrated. Flash chromatography (silica, 20%-50% EtOAc/hexanes) gave 2-5 as a yellow solid.
TLC R_{f} = 0.17 (silica, 50% EtOAc/hexanes)
¹H NMR (300 MHz, CDCl₃) δ 9.74 (s, 1H), 4.43 (m, 2H), 4.03 (m, 2H), 3.91 (m, 4H), 3.80 (1H), 3.04 (m, 1H), 2.48 (m, 2H), 2.10-1.40 (m, 8H), 1.29 (s, 3H).

### 3(S)-(3-Fluoro-phenyl)-3-{2-oxo-[3-(2-methyl-[1,3]dioxolan-2-yl)-propyl]-piperidin-1-yl}-propionic acid ethyl ester (2-6)

A mixture of 2-5 (600 mg, 1.0 mmol), 1-4 (240 mg, 1.0 mmol), Na(OAc)₃BH (313 mg, 1.5 mmol) and NEt₃ (0.28 mL, 2.0 mmol) in DCE (10 mL) was stirred for 24 h. The mixture was diluted with ethyl acetate, washed with 10% K₂CO₃, brine, and dried over MgSO₄. Following evaporative removal of the solvent, the residue was chromatographed (silica gel, 50:35:14:1 hexanes/ chloroform/ ethyl acetate/ MeOH) to give 2-6 as a yellow solid.
TLC R_{f} = 0.53 (silica, 70:25:5 chloroform/ ethyl acetate/ MeOH)
¹H NMR (300 MHz, CDCl₃) δ 7.30 (m, 1H), 7.07 (m, 1H), 6.98 (m 3H), 6.35 (m, 0.5H), 6.23 (m, 0.5H), 4.12 (q, J=7 Hz, 2H), 3.92 (m, 4H), 3.20 (m, 1H), 2.95 (m, 3H), 2.38 (m, 1H), 2.00-1.40 (m, 8H), 1.30 (s, 3H), 1.22 (t, J=7 Hz, 3H).

### 3(S)-(3-Fluorophenyl)-3-[2-oxo-3-(4-oxo-pentyl)-piperidin-1-yl]-propionic acid ethyl ester (2-7)

A solution of 2-6 (500 mg, 1.10 mmol), p-TSA (10 mg) and acetone (50 mL) was heated at reflux for 4 h. The cooled reaction mixture was diluted with EtOAc and then washed with sat. NaHCO₃ and brine, dried (MgSO₄), and concentrated to afford 2-7 as a yellow oil. ¹H NMR (300 MHz, CDCl₃) δ 7.30 (m, 1H), 7.06 (m, 1H), 6.98 (m, 2H), 6.32 (m, 0.5H), 6.20 (m, 0.5H), 4.12 (q, J=7Hz, 2H), 3.20 (m, 1H), 2.95 (m, 3H), 2.45 (m, 2H), 2.33 (m, 1H), 2.14 (s, 3H), 2.00-1.40 (m, 8H), 1.22 (t, J=7 Hz, 3H).

### 3(S)-(3-Fluorophenyl)-3-[3-(3-[1,8]naphthyridin-2-yl-propyl)-2-oxo-piperidin-1-yl]-propionic acid ethyl ester (2-8)

A mixture of 2-7 (450 mg, 1.2 mmol), 2-amino-3-formylpyridine (145 mg, 1.2 mmol; for prep., see *JOC* 1983,*48*, 3401) and proline (137 mg, 1.2 mmol) in absolute ethanol (20 mL) was heated at reflux for 12 h. Following evaporative removal of the solvent, the residue was chromatographed (silica gel, 50% ethyl acetate/ chloroform to 70:25:5 chloroform/ethyl acetate/MeOH) to give 2-8 as a yellow oil.
TLC Rf = 0.34 (70:25:5 chloroform/ethyl acetate/MeOH).
¹H NMR (300 MHz, CDCl₃) δ 9.08 (m, 1H), 8.16 (dd, J=2.0 Hz, 8.0 Hz 1H), 8.11 (d, J=8.3 Hz, 1H), 7.42 (m, 2H), 7.06 (m, 1H), 6.97 (m, 2H), 6.35 (m, 0.5H), 6.20 (m, 0.5H), 4.11 (m, 2H), 3.20-2.80 (m, 5H), 2.40 (m, 1H), 2.10-1.40 (m, 8H), 1.19 (m, 3H).

### 3(S)-(3-Fluorophenyl)-3-(2-oxo-3-[3-(5,6,7,8-tetrahydro-[1,8]naphthyridin-2-yl)-propyl]-piperidin-1-yl)-propionic acid ethyl ester (2-9)

A mixture of 2-8 (400 mg, 0.86 mmol) and 10% Pd/carbon (1300 mg) in EtOH (10 mL) was stirred under a balloon of hydrogen for 6 h. Following filtration and evaporative removal of the solvent, the residue was chromatographed (silica gel, 70:25:5 chloroform/ethyl acetate/MeOH) to give 2-9 as a yellow oil.
TLC Rf = 0.21 (70:25:5 chloroform/ethyl acetate/MeOH).
¹H NMR (300 MHz, CDCl₃) δ 7.29 (m, 1,), 7.05 (m, 2H), 6.98 (m, 2H), 6.33 (m, 1H), 6.30 (m, 0.5H), 6.20 (m, 0.5H), 4.76 (bs, 1H), 4.10 (m, 2H), 3.40 (m, 2H), 3.20 (m, 1H), 3.00-2.90 (m, 3H), 2.70 (t, J=6.3 Hz, 2H), 2.55 (m, 2H), 2.35 (m, 1H), 2.00-1.40 (m, 8H), 1.23 (t, J=7Hz, 3H).

### 3(S)-(3-Fluorophenyl)-3-(2-oxo-3(R or S)-[3-(5,6,7,8-tetrahydro-[1,8]naphthyridin-2-yl)-propyl]-piperidin-1-yl)-propionic acid and 3(S)-(3-fluorophenyl)-3-(2-oxo-3(S or R)-[3-(5,6,7,8-tetrahydro-[1,8]naphthyridin-2-yl)-propyl]-piperidin-1-yl)-propionic acid (2-10 and 2-11)

To a solution of 2-9 (300 mg, 0.6614 mmol) in EtOH (3 mL) was added 1N NaOH (0.725 ml, 0.725 mmol). After stirring for 1 h, the solvents were evaporated and the residue was chromatographed (silica gel, 25:10:1:1 - 15:10:1:1 ethyl acetate/EtOH/water/NH₄OH) to give 2-10 and 2-11 as pure diastereomeric white solids.
TLC Rf = 0.62 (isomer A) (10:10:1:1 ethyl acetate/EtOH/water/NH₄OH). TLC Rf = 0.51 (isomer B) (10:10:1:1 ethyl acetate/EtOH/water/NH₄OH). ¹H NMR (300 MHz, CD₃OD, Isomer A) δ 7.46 (d, J=7 Hz, 1H), 7.37 (m, 1H), 7.15 (m, 1H), 7.10 (m, 1H,), 7.03 (m, 1H), 6.50 (d, J=7 Hz, 1H,), 6.43 (m, 1H), 3.48 (m, 2H), 3.36 (m, 2H), 3.00 (m, 1H), 2.90 (m, 2H), 2.70 (m, 2H), 2.80-2.55 (m, 3H), 2.12-1.40 (m, 10H).
¹H NMR (300 MHz, CD₃OD, Isomer B) δ 7.40 (d, J=7 Hz, 1H), 7.34 (m, 1H), 7.15 (m, 1H), 7.10 (m, 1H,), 7.00 (m, 1H), 6.50 (d, J=7 Hz, 1H,), 5.63 (m, 1H), 3.45 (m, 2H), 3.36 (m, 2H), 3.15 (m, 2H), 2.82 (m, 1H), 2.78 (m, 2H), 2.63 (m, 2H), 2.47 (m, 1H), 2.00-1.50 (m, 10H).

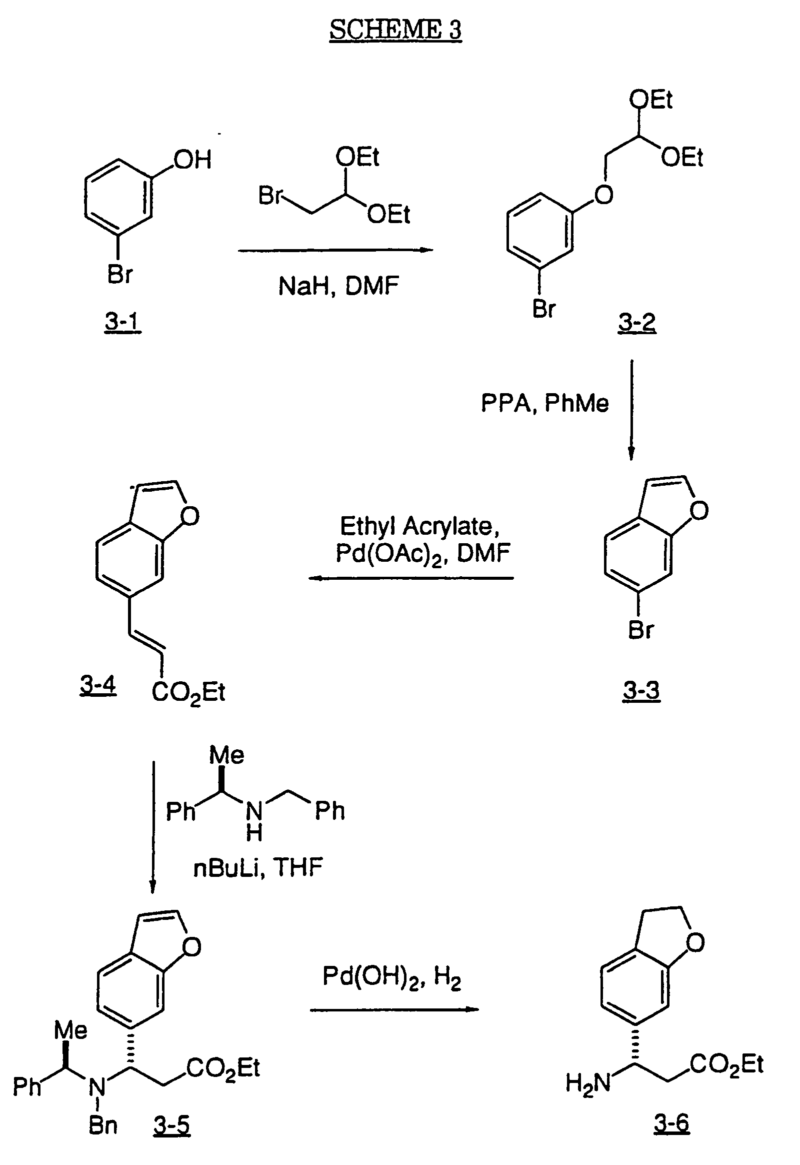

### 1-Bromo-3-(2,2-diethoxy-ethoxy)-benzene (3-2)

To a suspension of NaH (2.77 g, 115.6 mmol) in DMF (100 mL) at 0°C was added a solution of 3-bromophenol 3-1 in DMF (40 mL) over 40 min. After the addition was complete, the solution was stirred for an additional 30 min. The solution was then treated with neat bromoacetaldehyde diethyl acetal (17.36 g, 115.6 mmol). The solution was heated at 100°C for 8 h, cooled to room temperature, and extracted with Et₂O (3 x 200 mL). The combined organic extracts were washed with 10% aq. NaOH (100 mL) and brine (100 mL), dried over MgSO₄, filtered and concentrated to give 3-2 as a yellow oil.
TLC Rf = 0.4 (10% ethyl acetate/hexanes).
¹H NMR (300 MHz, CDCl₃) δ 7.19-7.05 (m, 3H), 6.85 (d, 1H), 4.81 (t, 1H, J=6.8 Hz), 3.99 (d, 2H, J=6.8 Hz), 3.71 (m, 4H), 1.22 (t, 6H, J=7.1 Hz) ppm.

### 6-Bromo-benzofuran (3-3)

To a solution of the acetal 3-2 in toluene (200 mL) was added polyphosphoric acid (20 g). The biphasic mixture was heated to 100°C and stirred at this temperature for 4 h. The mixture was cooled to room temperature, poured onto ice, and extracted with Et₂O (2 x 200 mL). The combined organic extracts were washed with saturated aq. NaHCO₃ and brine. The solution was dried over MgSO₄, filtered, and concentrated. The residue was purified by flash chromatography (100% hexanes) to give the product 3-3 as a yellow oil.
TLC Rf = 0.3 (100% hexanes).
¹H NMR (300 MHz, CDCl₃) δ 7.68 (s, 1H), 7.60 (d, 1H, J=2.1 Hz), 7.46 (d, 1H, J=8.4 Hz), 7.36 (dd, 1H, J=8.1, 1.5 Hz), 6.75 (dd, 1H, J=7.1, 0.9 Hz) ppm.

### 3-(Benzofuran-6-yl)-acrylic acid ethyl ester (3-4)

A mixture of the 6-bromo-benzofuran 3-3 (1.74 g, 8.79 mmol), ethyl acrylate (1.09 g, 10.98 mmol), Pd(OAc)₂ (0.099 g, 0.44 mmol), tri-*o*-tolylphosphine (0.268 g, 0.880 mmol), and sodium acetate (3.60 g, 43.9 mmol) in DMF (10 mL) was heated to 100°C in a sealed tube for 4 h. The mixture was cooled to room temperature, diluted with water, and extracted with Et₂O (2 x 40 mL). The combined organic extracts were washed with brine (30 mL), dried over MgSO₄, filtered, and concentrated. The residue was purified by flash chromatography (10% ethyl acetate/hexanes) to give the ester 3-4 as an off-white solid.
TLC Rf = 0.3 (10% ethyl acetate/hexanes).
¹H NMR (300 MHz, CDCl₃) δ 7.78 (d, 1H, J=15.9 Hz), 7.68 (d, 1H, J=2.4 Hz), 7.66 (s, 1H), 7.59 (d, 1H, J=8.4 Hz), 7.43 (dd, 1H, J=9.0, 1.5 Hz), 6.78 (m, 1H), 6.47 (d, 1H, J=15.9 Hz), 4.27 (q, 2H, J=7.2 Hz), 1.34 (t, 3H, J=7.2 Hz) ppm.

### 3(S)-Benzofuran-6-yl-3-[benzyl-(1(R)-phenyl-ethyl)-amino]-propionic acid ethyl ester (3-5)

A solution of N-benzyl-α-(R)-methylbenzylamine (1.32 g, 6.30 mmol) in THF (25 mL) at 0°C was treated with n-BuLi (2.52 mL of a 2.5 M soln in hexanes). The resulting solution was stirred at 0°C for 30 min and then cooled to -78°C. A solution of acrylate 3-4 (0.681 g, 3.15 mmol) in THF (5 mL) was added. After stirring for 15 min at -78 °C, satd. aq. NH₄Cl soln (5 mL) was added and the cold bath removed. The mixture was warmed to room temperature and extracted with Et₂O (2 x 40 mL). The combined organic extracts were washed with brine (30 mL), dried over MgSO₄, filtered, and concentrated. The residue was purified by flash chromatography (10% ethyl acetate/hexanes) to give the β-aminoester 3-5 as a yellow oil.
TLC Rf = 0.8 (10% ethanol/dichloromethane).
¹H NMR (300 MHz, CDCl₃) δ 7.58 (m, 3H), 7.41 (m, 2H), 7.22 (m, 9H), 7.59 (s, 1H), 4.58 (m, 1H), 4.05 (m, 1H), 3.91 (q, 2H, J=7.1 Hz), 3.72 (m, 2H), 2.62 (m, 2H), 1.21 (d, 3H, J=7.2 Hz), 1.03 (t, 3H, J=7.1 Hz) ppm.

### 3(S)-Amino-3-(2,3-dihydro-benzofuran-6-yl)-propionic acid ethyl ester (3-6)

A mixture of the dibenzylamine 3-5 (1.19 g, 2.78 mmol) in EtOH/H₂O/AcOH (26 mL/3 mL/1.0 mL) was degassed with argon and treated with Pd(OH)₂ (1.19 g). The mixture was placed under 1 atm of H₂. After stirring for 18 h, the mixture was diluted with EtOAc, and filtered through celite. The filtrate was concentrated and the residue purified by flash chromatography (10% ethyl acetate/dichloromethane) to give the ester 3-6 as a white solid.
TLC Rf = 0.25 (10% ethanol/dichloromethane).
¹H NMR (300 MHz, CD₃OD) as the trifluoroacetate salt: δ 7.25 (d, 1H, J=8.1 Hz), 6.88 (m, 1H), 7.66 (s, 1H), 6.82 (s, 1H), 4.58 (m, 3H), 4.12 (m, 2H), 3.30 (m, 1H), 3.19 (m, 2H), 2.98 (m, 2H), 1.11 (t, 3H, J=7.2 Hz) ppm.

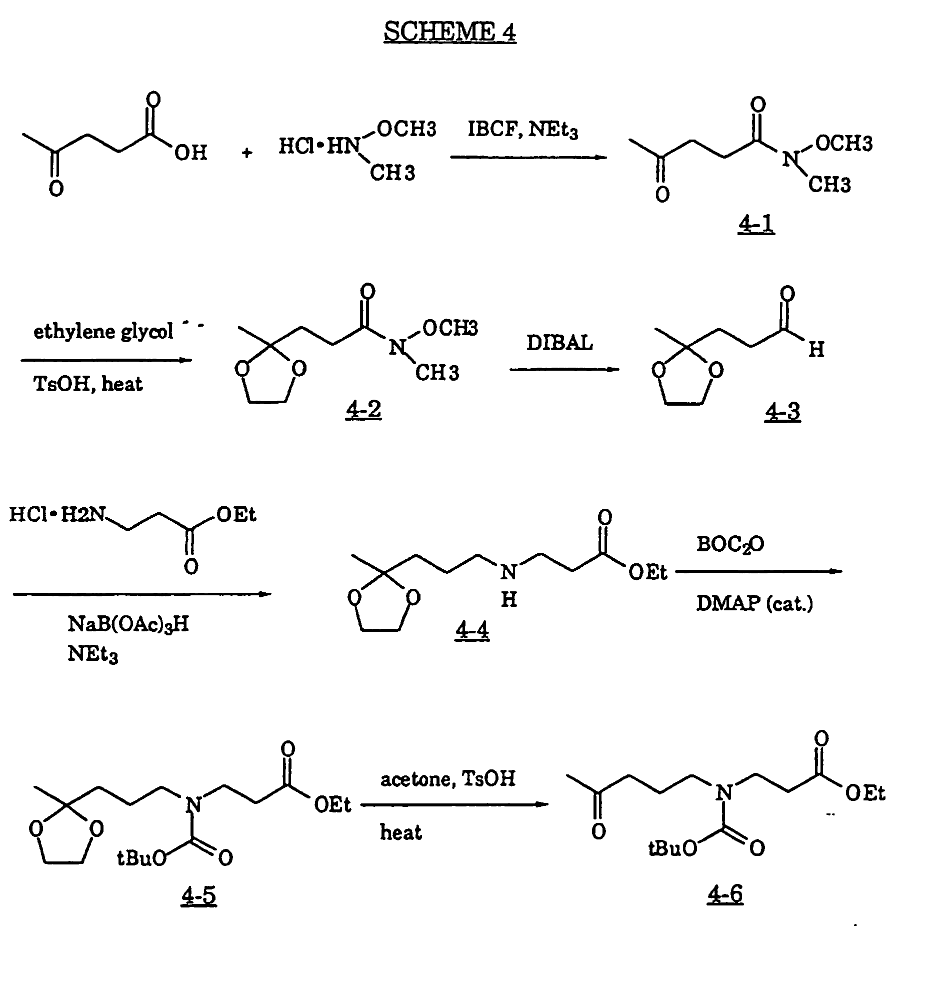

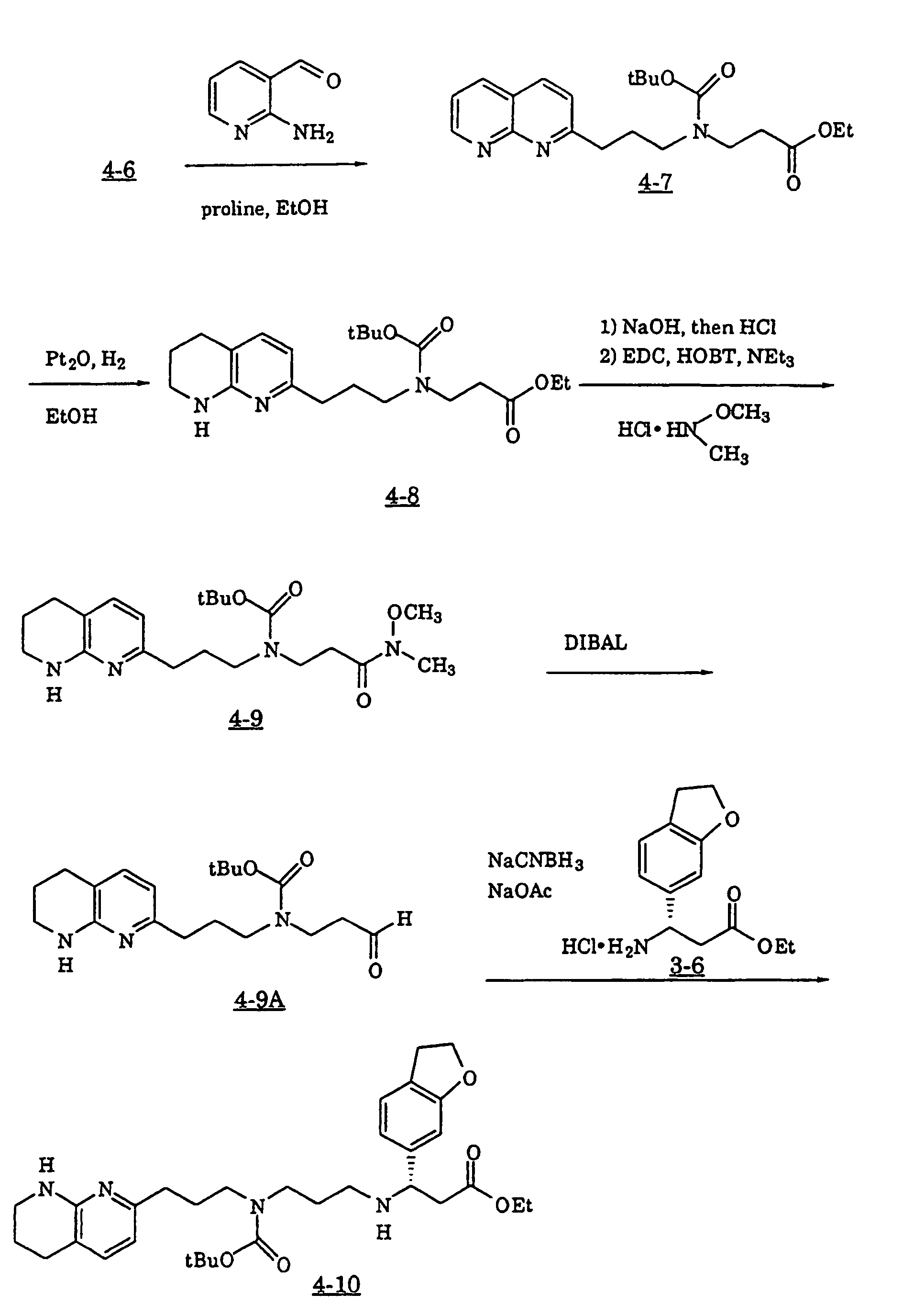

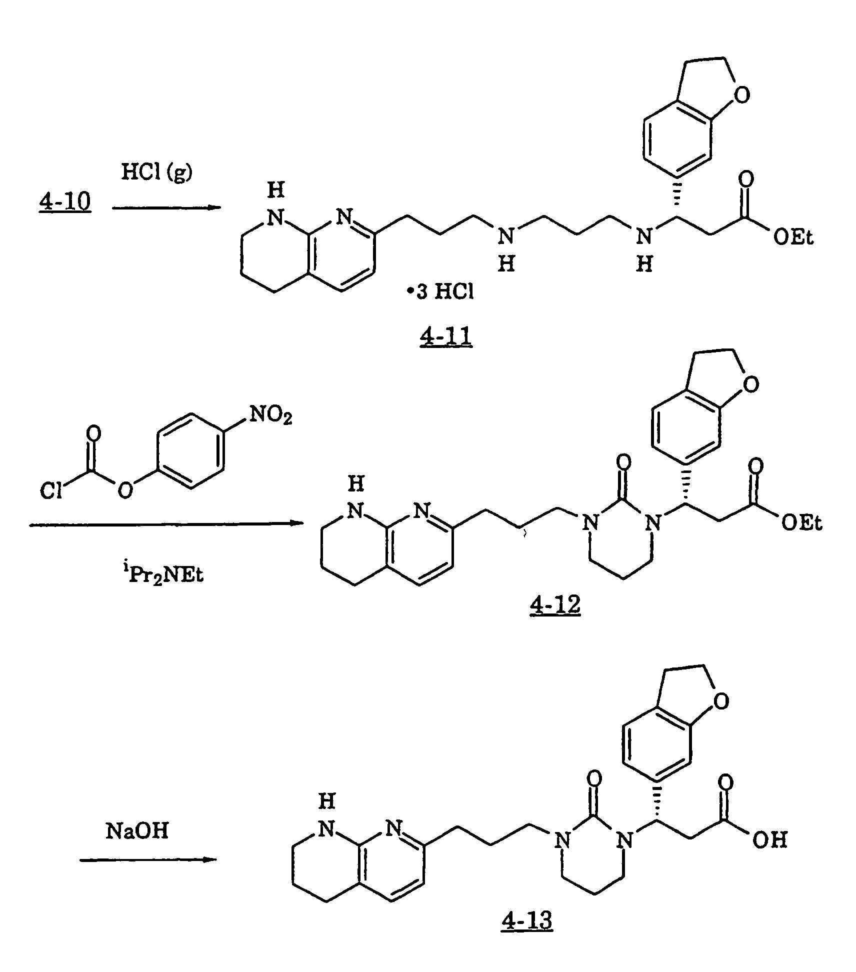

### 4-Oxo-pentanoic acid methoxy-methyl-amide (4-1)

To a stirred solution of levulinic acid (30 g, 0.258 mol) in 850 mL CHCl₃ at 0°C was added triethylamine (43.2 mL, 0.310 mol), followed by isobutyl chloroformate (37 mL, 0.284 mol) over 15 minutes. After 30 minutes, triethylamine (57.6 mL, 0.413 mol) was added, followed by N,O-dimethylhydroxylamine hydrochloride (37.8 g, 0.387 mol) in 5 portions over 5 minutes. Vigorous bubbling ensued, and the mixture was allowed to warm to RT and stir for 1 h. The mixture was reduced to a moist solid by rotary evaporation under reduced pressure, slurried in 500 mL EtOAc, washed with 10% K₂CO₃, brine, and dried over Na₂SO₄. Evaporative removal of the solvent gave 4-1 as a yellow oil.
TLC R_{f} = 0.42 (silica, 1:1 chloroform /ethyl acetate).
¹H NMR (300 MHz, CDCl₃) δ 3.74 (s, 3H), 3.18 (s, 3H), 2.65-2.95 (m, 4H), 2.21 (s, 3H).

### N-methoxy-N-methyl-3-(2-methyl-[1,3]dioxolan-2-yl)-propionamide (4-2)

To a solution of 4-1 (38 g, 0.239 mol) in 500 mL benzene was added ethylene glycol (17.3 mL, 0.310 mol) and p-toluenesulfonic acid (1 g). The mixture was heated at reflux for 2 h with azeotropic removal of water. After cooling, the solution was washed with 200 mL sat. NaHCO₃, brine, and dried over Na₂SO₄. Evaporative removal of the solvent gave 4-2 as a yellow oil.
TLC R_{f} = 0.62 (silica, ethyl acetate).
¹H NMR (300 MHz, CDCl₃) δ 3.95 (m, 4H), 3.68 (s, 3H), 3.17 (s, 3H), 2.51 (t, 2H, J=8 Hz), 2.00 (t, 3H, J=6 Hz) 1.33 (S, 3H).

### 3-(2-Methyl-[1,3]dioxolan-2-yl)-propionaldehyde (4-3)

To a solution of 5-2 (44.74 g, 0.22 mol) in 400 mL THF at -78°C was added DIBAL (264 mL 1 M in hexanes, 0.264 mol) over 10 minutes. After stirring for 1 h, 350 ml of 1.0 M Rochelle's salt and 300 ml ether was added followed by the removal of the cooling bath. After stirring for 1 h, the organic portion was separated and dried over Na₂SO₄. Evaporative removal of the solvent gave 4-3 as a colorless oil. TLC R_{f} = 0.80 (silica, ethyl acetate).
¹H NMR (300 MHz, CDCl₃) δ 9.73 (s, 1H), 3.50 (d, 1H, J=16 Hz), 2.61 (d, 1H, J=21 Hz), 2.48 (m, 1H), 2.07 (t, 1H, J= 7H), 1.33 (s, 3H).

### [3-(2-Methyl-[1,3]dioxolan-2-yl)-propylamino]-propionic acid ethyl ester (4-4)

To a solution of 4-3 (10 g, 0.69 mmol) in 200 mL 1,2-dichloroethane at 0°C was added beta-alanine ethyl ester hydrochloride (21 g, 0.138 mol), triethylamine (34 mL), and NaB(OAc)₃H (20.5 g, 0.097 mol). The mixture was allowed to warm to RT and stir for 15 h. The mixture was evaporated to one-third its initial volume, diluted with EtOAc and then washed with 10% K₂CO₃, brine, and dried over Na₂SO₄. Following evaporative removal of the solvent, the residue was chromatographed (silica gel, 70:25:5 chloroform /ethyl acetate /methanol) to give 4-4 as a yellow oil.
TLC R_{f} = 0.19 (silica, 70:25:5 chloroform /ethyl acetate /methanol).

### {Tert-butoxycarbonyl-[3-(2-methyl-[1,3]dioxolan-2-yl)-propyl]-amino)-propionic acid ethyl ester (4-5)

To a solution of 4-4 (10 g, 0.041 mol) in 200 mL THF was added a trace of DMAP, 20 drops of triethylamine, and BOC₂O (9.5 g, 0.045 mol). After 1 h, evaporative removal of the solvent gave 4-5 as a colorless oil.
TLC R_{f} = 0.91 (silica, 70:25:10 chloroform /ethyl acetate /methanol).
¹H NMR (300 MHz, CDCl₃) δ 4.15 (q, 2H, J=4 Hz), 3.93 (m, 4H), 3.46 (m, 2H), 3.22 (m, 2H), 2.58 (m, 2H), 1.61 (m, 4H), 1.48 (m, 9H), 1.28 (m, 6H).

### [Tert-butoxycarbonyl-(4-oxo-pentyl)-amino]-propionic acid ethyl ester (4-6)

To a solution of 4-5 (13.9 g, 0.040 mol) in 100 mL acetone was added p-toluenesulfonic acid (.05 g). The mixture was heated at reflux for 2 h. After cooling, the mixture was evaporated to one-fifth its initial volume, diluted with EtOAc and then washed with sat. NaHCO₃, brine, and dried over Na₂SO₄. Evaporative removal of the solvent gave 4-6 as a yellow oil.
TLC R_{f} = 0.36 (silica, 30% ethyl acetate/hexane).

### [Tert-butoxycarbonyl-(3-[1,8]naphthyridin-2-yl-propyl)-amino]-propionic acid ethyl ester (4-7)

To a solution of 4-6 (12 g, 40 mmol), 2-amino-3-formylpyridine (6.3 g, 52 mmol), proline (4.6 g, 40 mmol) and ethanol (300 mL) was heated at reflux for 15 h. After cooling and evaporation, the residue was chromatographed (silica gel, 1:1 chloroform /ethyl acetate) to give 4-7 as a yellow oil.
TLC R_{f} = 0.47 (silica, 70:25:5 chloroform /ethyl acetate /methanol)
¹H NMR (300 MHz, CDCl₃) δ 9.09 (m, 1H), 8.13 (m, 2H), 7.43 (m, 2H), 4.12 (q, 2H, 7 Hz), 3.42 (m, 4H), 3.03 (m, 2H), 2.48 (m, 2H), 2.16 (m, 2H), 1.42 (m, 9H), 1.22 (m, 3H).

### {Tert-butoxycarbonyl-[3-(5,6,7,8-tetrahydro-[1,8]naphthyridin-2-yl)-propyl]-amino}-propionic acid ethyl ester (4-8)

A solution of 5-7 (7.0 g, 18.1 mmol), 10% Pd/C (5 g) and ethanol (100 mL) was stirred under a balloon of hydrogen gas for 15 h. Following filtration and evaporation, the residue was chromatographed (silica gel, 70:28:2 chloroform /ethyl acetate /methanol) to give 4-8 as a yellow oil.
TLC R_{f} = 0.32 (silica, 70:28:2 chloroform /ethyl acetate /methanol)

### [(Methoxy-methyl-carbamoyl)-ethyl]-[3-(5,6,7,8-tetrahydro-[1,8]naphthyridin-2-yl)-propyl]-carbamic acid tert-butyl ester (4-9)

To a solution of 4-8 (5.6 g, 12.8 mmol) in ethanol (50 mL) was added NaOH (15 mL 1M solution in water,15 mmol). After stirring for 1 h, HCl (15 mL 1M solution in water,15 mmol) was added, and the mixture evaporated to give an oily residue. The residue was evaporated from ethanol three times, and then from acetonitrile three times, producing a yellow crusty solid which was dried under a vacuum of <2 mm Hg for 2 h. This residue was then slurried in chloroform (5 mL) and acetonitrile (50 mL), and NMM (8.5 mL), N,O-dimethylhydroxylamine hydrochloride (2.5 g, 26 mmol), HOBT (1.7g, 13 mmol) and EDC (2.4 g, 13 mmol) were added. After stirring for 15 h, the mixture was evaporated to dryness, the residue slurried in EtOAc, washed with sat. NaHCO₃, brine, and dried over Na₂SO₄. Evaporative removal of the solvent gave 4-9 as a yellow oil.
TLC R_{f} = 0.20 (silica, 70:20:10 chloroform /ethyl acetate /methanol)
¹H NMR (300 MHz, CDCl₃) δ 7.05 (d, 1H, J= 8 Hz), 6.28 (d, 1H, J=8 Hz), 4.83 (m, 1H), 3.69 (m, 3H), 3.51 (m, 2H), 3.39 (m, 2H), 3.14 (m, 2H), 3.18 (s, 3H), 2.63 (m, 4H), 2.48 (m, 2H), 1.83 (m, 9H).

### {Tert-butoxycarbonyl-[3-(5,6,7,8-tetrahydro-[1,8]naphthyridin-2-yl)-propyl]-amino}-propionaldehyde (4-9A)

To a stirred solution of 4-9 (12.8 mmol) and THF (50 ml) at -78°C was added DIBAL (1.0M/hexanes, 15 ml, 15 mmol) dropwise over 20 minutes. After 1.0 hour, 20 ml of 1.0 M Rochelle's salt was added followed by the removal of the cooling bath. The mixture was stirred for 1.0 hour and then diluted with Et₂O. After 30 minutes of stirring, the organic portion was separated and dried over MgSO₄. Evaporative removal of the solvent gave crude aldehyde 4-9A as a colorless oil.
TLC R_{f} = 0.13 (silica,75:20:10 chloroform /EtOAc/MeOH).

### 3(S)-(2-{Tert-butoxycarbonyl-[3-(5,6,7,8-tetrahydro-[1,8]naphthyridin-2-yl)-propyl]-amino}-propylamino)-3-(2,3-dihydro-benzofuran-6-yl)-propionic acid ethyl ester (4-10)

A mixture of the crude aldehyde 4-9A (0.4 g, 1.15 mmol), 3-6 (0.5 g, 1.5 mmol), powdered molecular sieves (1 g), sodium acetate (1 g) and 2-propanol (20 mL) was stirred for 30 minutes at 0°C. Then NaCNBH₃ (0.215 g, 3.5 mmol) was added. After 15 hours, to the reaction was added 10% KHSO₄ until pH~2, stirred 5 minutes, diluted with EtOAc and then adjusted to pH-12 with K₂CO₃. The mixture was extracted with EtOAc and dried over MgSO₄. Following evaporative removal of the solvent, the residue was chromatographed (silica gel, 1% [10:1:1 EtOH/ NH₄OH/ H₂O]/ 99% 75:25:5 chloroform /EtOAc/MeOH) to give 4-10 as a yellow oil.
TLC R_{f} = 0.19 (silica, 1% [10:1:1 EtOH/ NH₄OH/ H₂O]/ 99% 75:25:5 chloroform /EtOAc/MeOH)
¹H NMR (300 MHz, CDCl₃) δ 7.10 (m, 2H),6.78 (m, 2H), 6.37 (d, 1H, J=7 Hz), 4.69 (br s, 1H), 4.47 (t, 2H, J= 7 Hz), 4.11 (m, 2H), 3.95 (t, 1H, J= 7Hz), 3.38 (m, 2H), 3.17 (m, 6H), 2.7-2.4 (m, 8H), 1.82 (m, 5H), 1.61 (m, 2H), 1.40 (m, 9H), 1.21 (t, 3H, J= Hz).

### 3(S)-(2,3-dihydro-benzofuran-6-yl)-3-{2-oxo-3-[3-(5,6,7,8-tetrahydro-[1,8]naphthyridin-2-yl)-propyl]-tetrahydro-pyrimidin-1-yl)-propionic acid ethyl ester (4-12)

HCl gas was rapidly bubbled through a solution of 4-10 (0.3 g, 0.53 mmol) in dichloromethane (10 ml) at 0°C for 10 minutes. After 30 minutes, the solution was purged with argon for 30 minutes. The solution was concentrated to give the amine 4-11 as a yellow solid. To a stirred mixture of 4-11 (0.53 mmol) in CH₂Cl₂ (10 mL) and iPr₂NEt (0.56 mL, 3.3 mmol) was added para-nitrophenyl chloroformate (0.112 g, 0.55 mmol). After 30 minutes, dioxane (10 mL) was added, and the mixture heated to 100°C, allowing the dichloromethane to evaporate. After 12 h, the mixture was cooled, diluted with EtOAc, washed with 1 N NaOH, brine, and dried over MgSO₄. Evaporative removal of the solvent gave 4-12 as a yellow oil.
TLC R_{f} = 0.37 (silica, 70:20:10 chloroform /ethyl acetate /methanol)

### 3(S)-(2,3-dihydro-benzofuran-6-yl)-3-{2-oxo-3-[3-(5,6,7,8-tetrahydro-[1,8]naphthyridin-2-yl)-propyl]-tetrahydro-pyrimidin-1-yl}-propionic acid (4-13)

To a solution of 4-12 (0.20 g, 0.41 mmol) in EtOH (5 mL) was added 1N NaOH (0.5 ml, 0.5 mmol). After stirring for 2 h, the solvents were evaporated and the residue was chromatographed (silica gel, 25:10:1:1 followed by 15:10:1:1 ethyl acetate /EtOH /water /NH₄OH) to give 4-13 as a white solid.
TLC Rf = 0.13 (10:10:1:1 ethyl acetate/EtOH/water/NH₄OH).
¹H NMR (300 MHz, CD₃OD) δ 7.44 (d, 1H, J=7 Hz), 7.16 (d, 1H, J=7 Hz), 6.76 (d, 1H, J=7 Hz), 6.65 (s, 1H), 5.97 (d, 1H, J=7 Hz), 6.06 (m, 1H), 4.52 (t, 2H, J= 9 Hz), 3.95 (m, 1H), 3.46 (m, 2H), 3.38 (m, 1H), 3.18 (m, 3H), 2.75 (m, 8H), 1.91 (m, 6H), 1.76 (m, 2H).

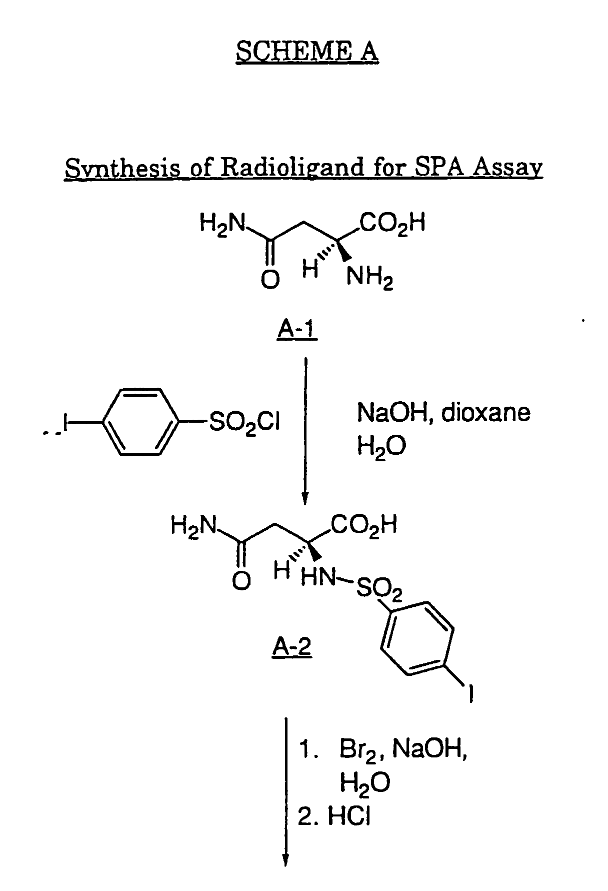

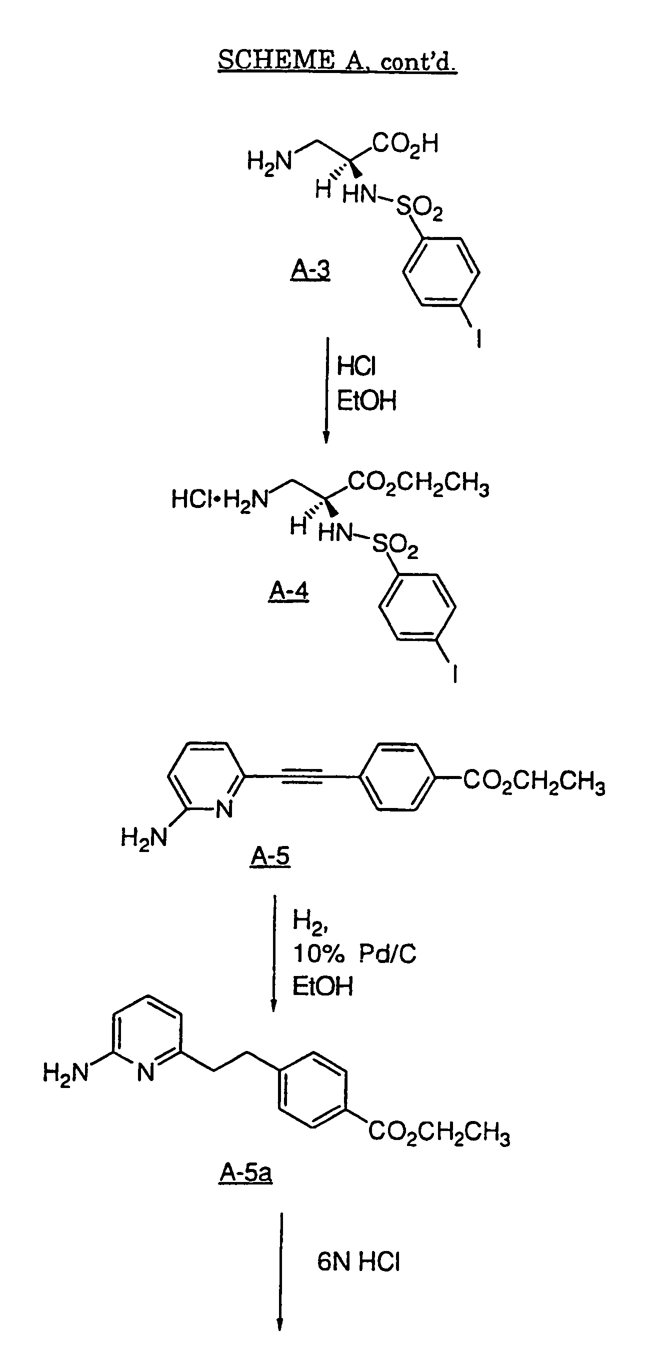

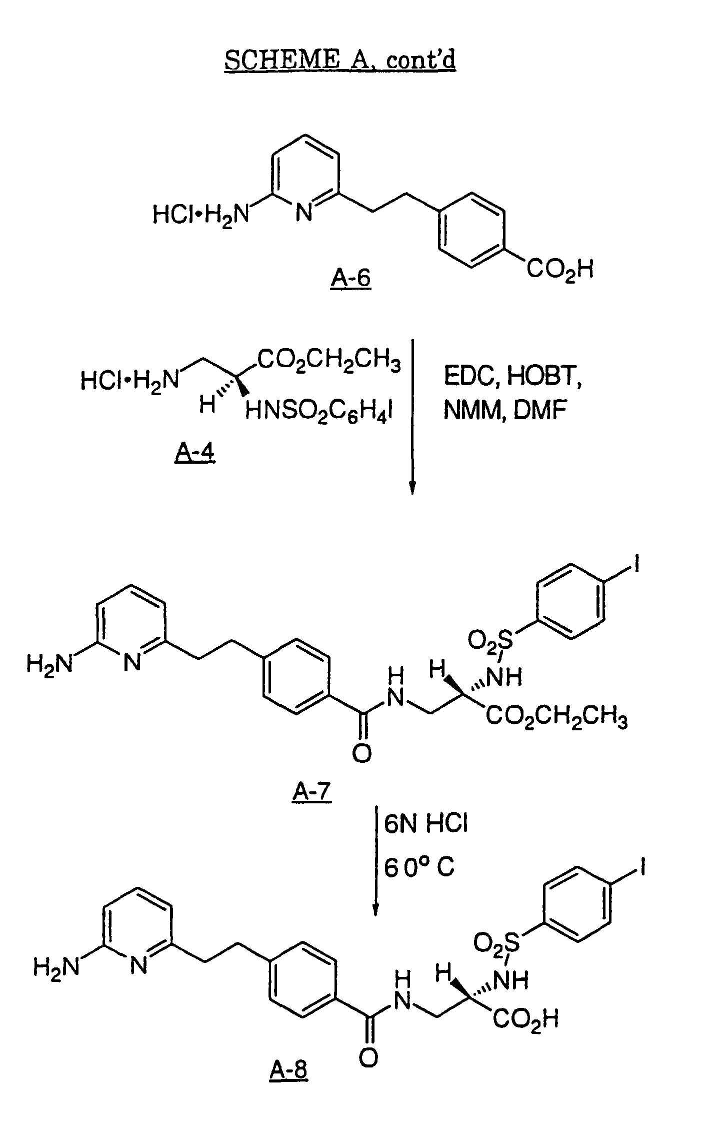

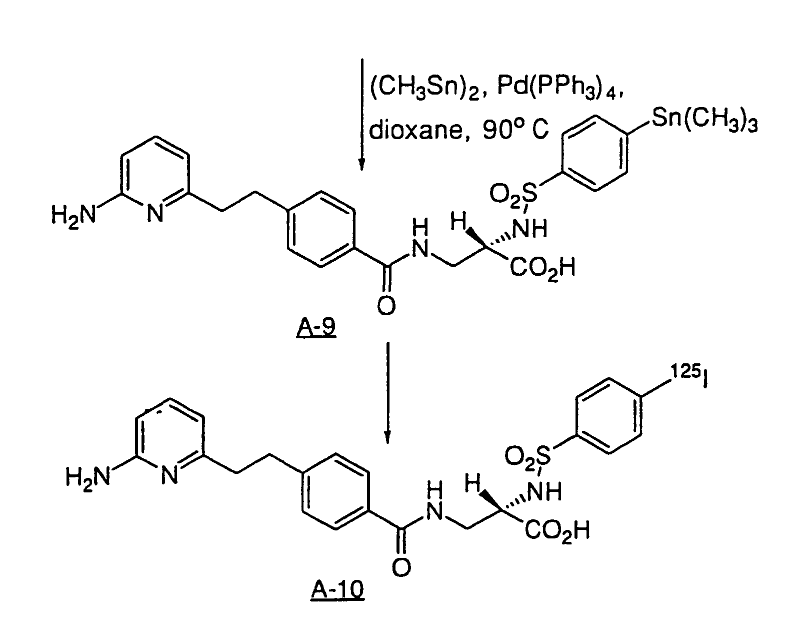

### N-(4-Iodo-phenylsulfonylamino)-L-asparagine (A-2)

To a stirred solution of acid A-1 (4.39 g, 33.2 mmol), NaOH (1.49 g, 37.2 mmol), dioxane (30 ml) and H₂O (30 ml) at 0°C was added pipsyl chloride (10.34 g, 34.2 mmol). After ~5 minutes, NaOH (1.49, 37.2 mmol), dissolved in 15 ml H₂O, was added followed by the removal of the cooling bath. After 2.0 h, the reaction mixture was concentrated. The residue was dissolved in H₂O (300 ml) and then washed with EtOAc. The aqueous portion was cooled to 0°C and then acidified with concentrated HCl. The solid was collected and then washed with Et₂O to provide acid A-2 as a white solid.
¹H NMR (300 MHz, D₂O) δ 7.86 (d, 2H, J=8Hz), 7.48 (d, 2H, J=8Hz) 3.70 (m, 1H), 2.39 (m, 2H).

### 2(S)-(4-Iodo-phenylsulfonylamino)-β-alanine (A-3)

To a stirred solution of NaOH (7.14 g, 181.8 mmol) and H₂O (40 ml) at 0°C was added Br₂ (1.30 ml, 24.9 mmol) dropwise over a ten minute period. After ∼5 minutes, acid A-2 (9.9 g, 24.9 mmol), NaOH (2.00 g, 49.8 mmol) and H₂O (35 ml) were combined, cooled to 0°C and then added in a single portion to the reaction. After stirring for 20 minutes at 0°C, the reaction was heated to 90°C for 30 minutes and then recooled to 0°C. The pH was adjusted to ~7 by dropwise addition of concentrated HCl. The solid was collected, washed with EtOAc, and then dried *in vacuo* to provide acid A-3 as a white solid.
¹H NMR (300 MHz, D₂O) δ 8.02 (d, 2H, J=8Hz), 7.63 (d, 2H, J=8Hz), 4.36 (m, 1H), 3.51 (dd, 1H, J=5Hz, 13Hz) 3.21 (m, 1H).

### Ethyl 2(S)-(4-iodo-phenylsulfonylamino)-β-alanine-hydrochloride (A-4)

HCl gas was rapidly bubbled through a suspension of acid A-3 (4.0 g, 10.81-mmol) in EtOH (50 ml) at 0°C for 10 minutes. The cooling bath was removed and the reaction was heated to 60°C. After 18 h, the reaction was concentrated to provide ester A-4 as a white solid.
¹H NMR (300 MHz, CD₃OD) δ 7.98 (d, 2H, J=8Hz), 7.63 (d, 2H, J=8Hz), 4.25 (q, 1H, J=5Hz), 3.92 (m, 2H), 3.33 (m, 1H), 3.06 (m, 1H), 1.01 (t, 3H, J=7Hz).

### Ethyl 4-[2-(2-Aminopyridin-6-yl)ethyl]benzoate (A-5a)

A mixture of ester A-5 (700 mg, 2.63 mmol), (for preparation, see: Scheme 29 of PCT International Application Publication No. WO 95/32710, published December 7, 1995) 10% Pd/C (350 mg) and EtOH were stirred under 1 atm H₂. After 20 h, the reaction was filtered through a celite pad and then concentrated to provide ester A-5a as a brown oil.
TLC R_{f} = 0.23 (silica, 40% EtOAc/hexanes)
¹H NMR (300 MHz, CDCl₃) δ 7.95 (d, 2H, J=8Hz), 7.26 (m, 3H), 6.43 (d, 1H, J=7Hz), 6.35 (d, 1H, J=8Hz), 4.37 (m, 4H), 3.05 (m, 2H), 2.91 (m, 2H), 1.39 (t, 3H, J=7Hz).

### 4-[2-(2-Aminopyridin-6-yl)ethyl]benzoic acid hydrochloride (A-6)

A suspension of ester A-5a (625 mg, 2.31 mmol) in 6N HCl (12 ml) was heated to 60°C. After ∼20 h, the reaction was concentrated to give acid A-6 as a tan solid.
¹H NMR (300 MHz, CD₃OD) δ 7.96 (d, 2H, J=8Hz), 7.80 (m, 1H), 7.33 (d, 2H, J=8Hz), 6.84 (d, 1H, J=9Hz), 6.69 (d, 1H, J=7Hz), 3.09 (m, 4H).

### Ethyl 4-[2-(2-Aminopyridin-6-yl)ethyl]benzoyl-2(S)-(4-iodo-phenylsulfonylamino)-β-alanine (A-7)

A solution of acid 15-6 (400 mg, 1.43 mmol), amine A-4 (686 mg, 1.57 mmol), EDC (358 mg, 1.86 mmol), HOBT (252 mg, 1.86 mmol), NMM (632 µl, 5.72 mmol) in DMF (10 ml) was stirred for ∼20 h. The reaction was diluted with EtOAc and then washed with sat. NaHCO₃, brine, dried (MgSO₄) and concentrated. Flash chromatography (silica, EtOAc then 5% isopropanol/EtOAc) provided amide A-7 as a white solid.
TLC R_{f} = 0.4 (silica, 10% isopropanol/EtOAc)
¹H NMR (300 MHz, CD₃OD) δ 7.79 (d, 2H, J=9Hz) 7.61 (d, 2H, J=8Hz), 7.52 (d, 2H, J=9Hz), 7.29 (m, 1H), 7.27 (d, 2H, J=8Hz), 4.20 (m, 1H), 3.95 (q, 2H, J=7Hz), 3.66 (dd, 1H, J=6Hz, 14Hz), 3.49 (dd, 1H, J=8Hz, 13Hz), 3.01 (m, 2H), 2.86 (m, 2H), 1.08 (t, 3H, J=7Hz).

### 4-[2-(2-Aminopyridin-6-yl)ethyl]benzoyl-2(S)-(4-iodophenylsulfonylamino)-β-alanine (A-8)

A solution of ester A-7 (200 mg, 0.3213 mmol) and 6N HCl (30 ml) was heated to 60°C. After ~20 h, the reaction mixture was concentrated. Flash chromatography (silica, 20:20:1:1 EtOAc/EtOH/NH₄OH/H₂O) provided acid A-8 as a white solid.
TLC R_{f} = 0.45 (silica, 20:20:1:1 EtOAc/EtOH/NH₄OH/H₂O)
¹H NMR (400 MHz, DMSO) δ 8.40 (m, 1H), 8.14 (Bs, 1H), 7.81 (d, 2H, J=8Hz), 7.62 (d, 2H, J=8Hz), 7.48 (d, 2H, J=8Hz), 7.27 (m, 3H), 6.34 (d, 1H, J=7Hz), 6.25 (d, 1H, J=8Hz), 5.85 (bs, 2H), 3.89 (bs, 1H), 3.35 (m, 2H), 2.97 (m, 2H), 2.79 (m, 2H).

### 4-[2-(2-Aminopyridin-6-yl)ethyl)benzoyl-2(S)-(4-trimethylstannylphenylsulfonylamino-β-alanine (A-9)

A solution of iodide A-8 (70 mg, 0.1178 mmol), [(CH₃)₃Sn]₂ (49 µl, 0.2356 mmol), Pd(PPh₃)₄ (5 mg) and dioxane (7 ml) was heated to 90°C. After 2 h, the reaction was concentrated and then purified by preparative HPLC (Delta-Pak C₁₈ 15 µM 100A°, 40 x 100 mm; 95:5 then 5:95 H₂O/CH₃CN) to provide the trifluoroacetate salt. The salt was suspended in H₂O (10 ml), treated with NH₄OH (5 drops) and then lyophilized to provide amide A-9 as a white solid.
¹H NMR (400 MHz, DMSO) δ 8.40 (m, 1H), 8.18 (d, 1H, J=8Hz), 7.67 (m, 5H), 7.56 (d, 2H, J=8Hz), 7.29 (d, 2H, J=8Hz), 6.95-7.52 (m, 2H), 6.45 (bs, 2H), 4.00 (m, 1H), 3.50 (m, 1H), 3.33 (m, 1H), 2.97 (m, 2H), 2.86 (m, 2H).

### 4-[2-(2-Aminopyridin-6-yl)ethyl]benzoyl-2(S)-4-¹²⁵iodophenylsulfonylamino-β-alanine (A-10)

An iodobead (Pierce) was added to a shipping vial of 5 mCi of Na¹²⁵I (Amérsham, IMS30) and stirred for five minutes at room temperature. A solution of 0.1 mg of A-9 in 0.05 mL of 10% H₂SO₄/MeOH was made and immediately added to the Na¹²⁵I/iodobead vial. After stirring for three minutes at room temperature, approximately 0.04-0.05 mL of NH₄OH was added so the reaction mixture was at pH 6-7. The entire reaction mixture was injected onto the HPLC for purification [Vydac peptide-protein C-18 column, 4.6 x 250 mm, linear gradient of 10% acetonitrile (0.1% (TFA):H₂O (0.1% TFA) to 90% acetonitrile (0.1% TFA):H₂O (0.1% TFA) over 30 minutes, 1 mL/min]. The retention time of A-10 is 17 minutes under these conditions. Fractions containing the majority of the radioactivity were pooled, lyophilized and diluted with ethanol to give approximately 1 mCi of A-10, which coeluted on HPLC analysis with an authentic sample of A-8.

Instrumentation: Analytical and preparative HPLC was carried out using a Waters 600E Powerline Multi Solvent Delivery System with 0.1 mL heads with a Rheodyne 7125 injector and a Waters 990 Photodiode Array Detector with a Gilson FC203 Microfraction collector. For analytical and preparative HPLC, a Vydac peptide-protein C-18 column, 4.6 x 250 mm was used with a C-18 Brownlee modular guard column. The acetonitrile used for the HPLC analyses was Fisher Optima grade. The HPLC radiodetector used was a Beckman 170 Radioisotope detector. A Vydac C-18 protein and peptide column, 3.9 x 250 mm was used for analytical and preparative HPLC. Solutions of radioactivity were concentrated using a Speedvac vacuum centrifuge. Calibration curves and chemical concentrations were determined using a Hewlett Packard Model 8452A UV/Vis Diode Array Spectrophotometer. Sample radioactivities were determined in a Packard A5530 gamma counter.

The test procedures employed to measure ανβ3 and ανβ5 binding and the bone resorption inhibiting activity of the compounds of the present invention are described below.

### BONE RESORPTION-PIT ASSAY

When osteoclasts engage in bone resorption, they can cause the formation of pits in the surface of bone that they are acting upon. Therefore, when testing compounds for their ability to inhibit osteoclasts, it is useful to measure the ability of osteoclasts to excavate these resorption pits when the inhibiting compound is present.

Consecutive 200 micron thick cross sections from a 6 mm cylinder of bovine femur diaphysis are cut with a low speed diamond saw (Isomet, Beuler, Ltd., Lake Bluff, Il). Bone slices are pooled, placed in a 10% ethanol solution and refrigerated until further use.

Prior to experimentation, bovine bone slices are ultrasonicated twice, 20 minutes each in H₂O. Cleaned slices are placed in 96 well plates such that two control lanes and one lane for each drug dosage are available. Each lane represents either triplicate or quadruplicate cultures. The bone slices in 96 well plates are sterilized by UV irradiation. Prior to incubation with osteoclasts, the bone slices are hydrated by the addition of 0.1 ml αMEM, pH 6.9 containing 5% fetal bovine serum and 1% penicillin/streptomycin.

Long bones from 7-14 day old rabbits (New Zealand White Hare) are dissected, cleaned of soft tissue and placed in αMEM containing 20 mM HEPES. The bones are minced using scissors until the pieces are <1 mm and transferred to a 50 ml tube in a volume of 25 ml. The tube is rocked gently by hand for 60 cycles, the tissue is sedimented for 1 min., and the supernatant is removed. Another 25 ml of medium is added to the tissue and rocked again. The second supernatant is combined with the first. The number of cells is counted excluding erythrocytes (typically ∼ 2 x 10⁷ cells/ml). A cell suspension consisting of 5 x 10⁶/ml in αMEM containing 5% fetal bovine serum, 10 nM 1,25(OH)₂D₃, and pencillin-streptomycin is prepared. 200 ml aliquots are added to bovine bone slices (200 mm x 6 mm) and incubated for 2 hrs. at 37°C in a humidified 5% CO₂ atmosphere. The medium is removed gently with a micropipettor and fresh medium containing test compounds is added. The cultures are incubated for 48 hrs., and assayed for c-telopeptide (fragments of the a1 chain of type I collagen) by Crosslaps for culture media (Herlev, Denmark).

Bovine bone slices are exposed to osteoclasts for 20-24 hrs and are processed for staining. Tissue culture media is removed from each bone slice: Each well is washed with 200 ml of H₂O, and the bone slices are then fixed for 20 minutes in 2.5% glutaraldehyde, 0.1 M cacodylate, pH 7.4. After fixation, any remaining cellular debris is removed by 2 min. ultrasonication in the presence of 0.25 M NH₄OH followed by 2 X 15 min ultrasonication in H₂O. The bone slices are immediately stained for 6-8 min with filtered 1% toluidine blue and 1% borax.

After the bone slices have dried, resorption pits are counted in test and control slices. Resorption pits are viewed in a Microphot Fx (Nikon) fluorescence microscope using a polarizing Nikon IGS filter cube. Test dosage results are compared with controls and resulting IC₅₀ values are determined for each compound tested.

The appropriateness of extrapolating data from this assay to mammalian (including human) disease states is supported by the teaching found in Sato, M., et al., Journal of Bone and Mineral Research, Vol. 5, No. 1, pp.31-40, 1990, which is incorporated by reference herein in its entirety. This article teaches that certain bisphosphonates have been used clinically and appear to be effective in the treatment of Paget's disease, hypercalcemia of malignancy, osteolytic lesions produced by bone metastases, and bone loss due to immobilization or sex hormone deficiency. These same bisphosphonates are then tested in the resorption pit assay described above to confirm a correlation between their known utility and positive performance in the assay.

### EIB ASSAY

Duong *et al., J. Bone Miner. Res.,* 8: S378 (1993) describes a system for expressing the human integrin ανβ3. It has been suggested that the integrin stimulates attachment of osteoclasts to bone matrix, since antibodies against the integrin, or RGD-containing molecules, such as echistatin (European Publication 382 451), can effectively block bone resorption.

### Reaction Mixture:

1. 175 µl TBS buffer (50 mM Tris •HCl pH 7.2, 150 mM NaCl, 1% BSA, 1 mM CaCl₂, 1 mM MgCl₂).
2. 25 µl cell extract (dilute with 100 mM octylglucoside buffer to give 2000 cpm/25 µl).
3. ¹²⁵I-echistatin (25 µl/50,000 cpm) (see EP 382 451).
4. 25 µl buffer (total binding) or unlabeled echistatin (non-specific binding).

The reaction mixture was then incubated for 1 h at room temp. The unbound and the bound ανβ3 were separated by filtration using a Skatron Cell Harvester. The filters (prewet in 1.5% polyethyleneimine for 10 mins) were then washed with the wash buffer (50 mM Tris HCl, 1mM CaCl₂/MgCl₂, pH 7.2). The filter was then counted in a gamma counter.

### SPA ASSAY

### MATERIALS:

1. Wheat germ agglutinin Scintillation Proximity Beads (SPA): Amersham
2. Octylglucopyranoside: Calbiochem
3. HEPES: Calbiochem
4. NaCl: Fisher
5. CaCl₂: Fisher
6. MgCl₂: SIGMA
7. Phenylmethylsulfonylfluoride (PMSF): SIGMA
8. Optiplate: PACKARD
9. Compound A-10 (specific activity 500-1000 Ci/mmole)
10. test compound
11. Purified integrin receptor: α_{ν}β₃ was purified from 293 cells overexpressing α_{ν}β₃ (Duong *et al., J*. *Bone Min. Res., 8*:S378, 1993) according to Pytela (*Methods in Enzymology, 144*:475, 1987)
12. Binding buffer: 50 mM HEPES, pH 7.8, 100 mM NaCl, 1 mM Ca^{2+/}Mg²⁺, 0.5 mM PMSF
13. 50 mM octylglucoside in binding buffer: 50-OG buffer

### PROCEDURE:

### 1. Pretreatment of SPA beads:

500 mg of lyophilized SPA beads were first washed four times with 200 ml of 50-OG buffer and once with 100 ml of binding buffer, and then resuspended in 12.5 ml of binding buffer.

### 2. Preparation of SPA beads and receptor mixture

In each assay tube, 2.5 µl (40 mg/ml) of pretreated beads were suspended in 97.5 µl of binding buffer and 20 µl of 50-OG buffer. 5 µl (~30 ng/µl) of purified receptor was added to the beads in suspension with stirring at room temperature for 30 minutes. The mixture was then centrifuged at 2,500 rpm in a Beckman GPR Benchtop centrifuge for 10 minutes at 4°C. The pellets were then resuspended in 50 µl of binding buffer and 25 µl of 50-OG buffer.

### 3. Reaction

The following were sequentially added into Optiplate in corresponding wells:
(i) Receptor/beads mixture (75 ml)
(ii) 25 µl of each of the following: compound to be tested, binding
   buffer for total binding or A-8 for non-specific binding (final concentration 1 µM)
(iii) A-10 in binding buffer (25 µl, final concentration 40 pM)
(iv) Binding buffer (125 µl)
(v) Each plate was sealed with plate sealer from PACKARD and
   incubated overnight with rocking at 4°C

### 4. Plates were counted using PACKARD TOPCOUNT

### 5. % inhibition was calculated as follows:

A = total counts
B = nonspecific counts
C = sample counts
% inhibition = [{(A-B)-(C-B)}/(A-B)]/(A-B) x 100

### OCFORM ASSAY

Osteoblast-like cells (1.8 cells), originally derived from mouse calvaria, were plated in CORNING 24 well tissue culture plates in αMEM medium containing ribo- and deoxyribonucleosides, 10% fetal bovine serum and penicillin-streptomycin. Cells were seeded at 40,000/well in the morning. In the afternoon, bone marrow cells were prepared from six week old male Balb/C mice as follows:

Mice were sacrificed, tibiae removed and placed in the above medium. The ends were cut off and the marrow was flushed out of the cavity into a tube with a 1 mL syringe with a 27.5 gauge needle. The marrow was suspended by pipetting up and down. The suspension was passed through >100 µm nylon cell strainer. The resulting suspension was centrifuged at 350 x g for seven minutes. The pellet was resuspended, and a sample was diluted in 2% acetic acid to lyse the red cells. The remaining cells were counted in a hemacytometer. The cells were pelleted and resuspended at 1 x 10⁶ cells/mL. 50 µL was added to each well of 1.8 cells to yield 50,000 cells/well and 1,25-dihydroxy-vitamin D₃ (D₃) was added to each well to a final concentration of 10 nM. The cultures were incubated at 37°C in a humidified, 5% CO₂ atmosphere. After 48 h, the medium was changed. 72 h after the addition of bone marrow, test compounds were added with fresh medium containing D₃ to quadruplicate wells. Compounds were added again after 48 h with fresh medium containing D₃. After an additional 48 h., the medium was removed, cells were fixed with 10% formaldehyde in phosphate-buffered saline for 10 minutes at room temperature, followed by a 1-2 minute treatment with ethanol:acetone (1:1) and air dried. The cells were then stained for tartrate resistant acid phosphatase as follows:

The cells were stained for 10-15 minutes at room temperature with 50 mM acetate buffer, pH 5.0 containing 30 mM sodium tartrate, 0.3 mg/mL Fast Red Violet LB Salt and 0.1 mg/mL Naphthol AS -MX phosphate. After staining, the plates were washed extensively with deionized water and air dried. The number of multinucleated, positive staining cells was counted in each well.

### ανβ5 ATTACHMENT ASSAY

Duong *et al., J. Bone Miner. Res., 11*: S290 (1996), describes a system for expressing the human ανβ5 integrin receptor.

### Materials:

1. Media and solutions used in this assay are purchased from BRL/Gibco, except BSA and the chemicals are from Sigma.
2. Attachment medium: HBSS with 1 mg/ml heat-inactivated fatty acid free BSA and 2 mM CaCl₂.
3. Glucosaminidase substrate solution: 3.75 mM p-nitrophenyl N-acetyl-beta-D-glucosaminide, 0.1 M sodium citrate, 0.25% Triton, pH 5.0.
4. Glycine-EDTA developing solution: 50 mM glycine, 5 mM EDTA, pH 10.5.

### Methods:

1. Plates (96 well, Nunc Maxi Sorp) were coated overnight at 4°C with human vitronectin (3 ug/ml) in 50 mM carbonate buffer (pH 9/.6), using 100 µl/well. Plates were then washed 2X with DPBS and blocked with 2% BSA in DPBS for 2h at room temperature. After additional washes (2X) with DPBS, plates were used for cell attachment assay.
2. 293 (αvβ5) cells were grown in MEM media in presence of 10% fetal calf serum to 90% confluence. Cells were then lifted from dishes with 1X Trypsin/EDTA and washed 3X with serum free MEM. Cells were resuspended in attachment medium (3 X 10⁵ cells/ml).
3. Test compounds were prepared as a series of dilutions at 2X concentrations and added as 50 µl/well. Cell suspension was then added as 50 µl/well. Plates were incubated at 37°C with 55 CO₂ for 1 hour to allow attachment.
4. Non-adherent cells were removed by gently washing the plates (3X) with DPBS and then incubated with glucosaminidase substrate solution (100 µl/well), overnight at room temperature in the dark. To quantitate cell numbers, standard curve of glucosaminidase activity was determined for each experiment by adding samples of cell suspension directly to wells containing the enzyme substrate solution.
5. The next day, the reaction was developed by addition of 185 µl/well of glycine/EDTA solution and reading absorbance at 405 nm using a Molecular Devices V-Max plate reader. Average test absorbance values (4 wells per test samples) were calculated. Then, the number of attached cells at each drug concentration was quantitated versus the standard curve of cells using the Softmax program.

### EXAMPLE OF A PHARMACEUTICAL FORMULATION

As a specific embodiment of an oral composition, 100 mg of a compound of the present invention are formulated with sufficient finely divided lactose to provide a total amount of 580 to 590 mg to fill a size O hard gel capsule.

Representative compounds of the present invention were tested and found to bind to human ανβ3 integrin. These compounds are generally found to have IC₅₀ values less than about 100 nM in the SPA assay.

Representative compounds of the present invention were tested and generally found to inhibit ≥ 50% the attachment of ανβ5 expressing cells-to plates coated with vitronectin at concentrations of about 1 µM.

For example, effective dosages other than the preferred doses as set forth hereinabove may be applicable as a consequence of variations in the responsiveness of the mammal being treated for severity of bone disorders caused by resorption, or for other indications for the compounds of the invention indicated above. Likewise, the specific pharmacological responses observed may vary according to and depending upon the particular active compound selected or whether there are present pharmaceutical carriers, as well as the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound of the formula 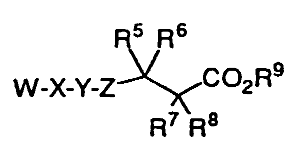 wherein W is selected from the group consisting of 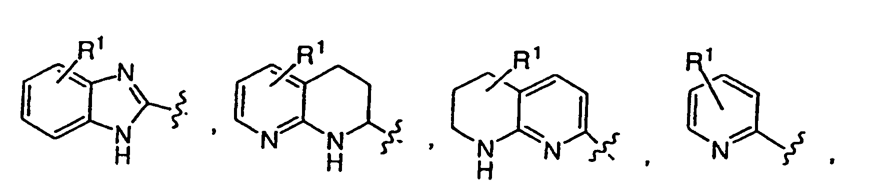 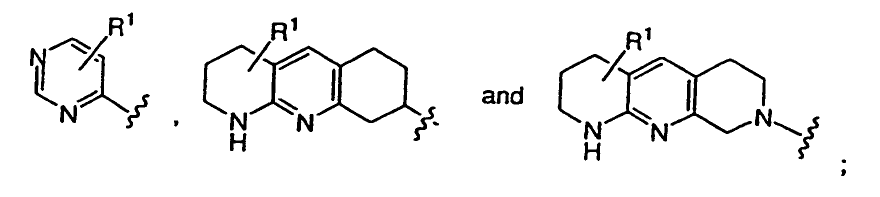
X is -(CH₂)ᵥ-, wherein any methylene (CH₂) carbon atom is either unsubstituted or substituted with one or two R¹ substitutents;
Y is selected from the group consisting of
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH₂)ₘ-O-(CH₂)ₙ-O-(CH₂)ₚ-,
-(CH₂)ₘ-O-(CH₂)ₙ-NR⁴-(CH₂)ₚ-,
-(CH₂)ₘ-NR⁴-(CH₂)ₙ-NR⁴-(CH₂)ₚ-,
-(CH₂)ₘ-O-(CH₂)ₙ-S-(CH₂)ₚ-,
-(CH₂)ₘ-S-(CH₂)ₙ-S-(CH₂)ₚ-,
-(CH₂)ₘ-NR⁴-(CH₂)ₙ-S-(CH₂)ₚ-,
-(CH₂)ₘ-NR⁴-(CH₂)ₙ-O-(CH₂)ₚ-,
-(CH₂)ₘ-S-(CH₂)ₙ-O-(CH₂)ₚ-,
and
-(CH₂)ₘ-S-(CH₂)ₙ-NR⁴-(CH₂)ₚ-,
wherein any methylene (CH₂) carbon atom in Y, other than in R⁴, can be substituted by one or two R³ substituents;
Z is selected from the group consisting of 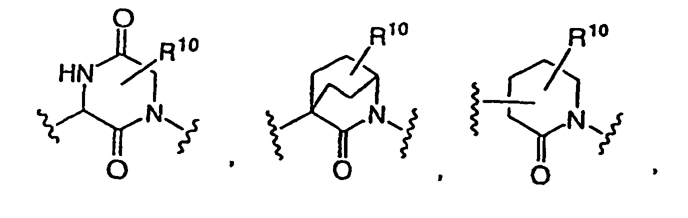 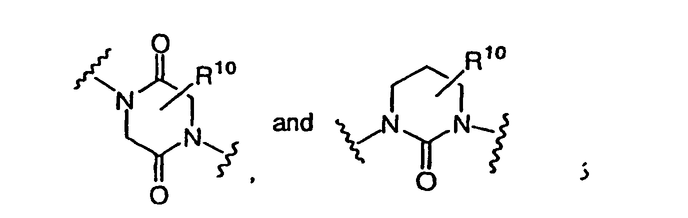
R¹ is selected from the group consisting of
hydrogen,
halogen,
C₁₋₁₀ alkyl,
C₃₋₈ cycloalkyl,
C₃₋₈ cycloheteroalkyl,
hydroxy,
nitro,
cyano,
trifluoromethyl, and
trifluoromethoxy;
each R³ is independently selected from the group consisting of
hydrogen,
fluoro,
trifluoromethyl,
aryl,
C₁₋₈ alkyl,
arylC₁₋₆ alkyl
hydroxyl,
oxo,
arylaminocarbonyl,
aryl C₁₋₅ alkylaminocarbonyl,
aminocarbonyl, and
aminocarbonyl C₁₋₆ alkyl;
each R⁴ is independently selected from the group consisting of
hydrogen,
aryl,
C₃₋₈ cycloalkyl,
C₁₋₈ alkyl,
C₁₋₈ alkylcarbonyl,
arylcarbonyl,
C₁₋₆ alkylsulfonyl,
arylsulfonyl,
arylC₁₋₆alkylsulfonyl,
arylC₁₋₆alkylcarbonyl,
C₁₋₈alkylaminocarbonyl,
arylC₁₋₅alkylaminocarbonyl,
arylC₁₋₈alkoxycarbonyl, and
C₁₋₈alkoxycarbonyl;
R⁵ and R⁶ are each independently selected from the group consisting of
hydrogen,
aryl,
C₁₋₈ alkyl,
aryl-C≡C-(CH₂)ₜ-,
aryl C₁₋₆ alkyl,
CH₂=CH-(CH₂)ₜ-, and
HC≡C-(CH₂)ₜ-;
R⁷ and R⁸ are each independently selected from the group consisting of
hydrogen,
aryl,
C₁₋₈ alkylcarbonylamino,
arylcarbonylamino,
C₁₋₈ alkylsulfonylamino,
arylsulfonylamino,
C₁₋₈ alkylsulfonylamino C₁₋₆ alkyl,
arylsulfonylamino C₁₋₆ alkyl,
aryl C₁₋₆ alkylsulfonylamino,
aryl C₁₋₆ alkylsulfonylamino C₁₋₆ alkyl,
C₁₋₈ alkoxycarbonylamino,
C₁₋₈ alkoxycarbonylamino C₁₋₈ alkyl,
aryloxycarbonylamino C₁₋₈ alkyl,
aryl C₁₋₈ alkoxycarbonylamino,
aryl C₁₋₈ alkoxycarbonylamino C₁₋₈ alkyl,
C₁₋₈ alkylcarbonylamino C₁₋₆ alkyl,
arylcarbonylamino C₁₋₆ alkyl,
aryl C₁₋₆ alkylcarbonylamino,
aryl C₁₋₆ alkylcarbonylamino C₁₋₆ alkyl,
aminocarbonylamino C₁₋₆ alkyl,
(C₁₋₈ alkyl)ₚaminocarbonylamino,
(C₁₋₈ alkyl)ₚaminocarbonylamino C₁₋₆ alkyl,
(aryl)ₚaminocarbonylamino C₁₋₆ alkyl,
(aryl C₁₋₈ alkyl)ₚaminocarbonylamino,
(aryl C₁₋₈ alkyl)ₚaminocarbonylamino C₁₋₆ alkyl,
aminosulfonylamino C₁₋₆ alkyl,
(C₁₋₈ alkyl)ₚaminosulfonylamino,
(C₁₋₈ alkyl)ₚaminosulfonylamino C₁₋₆ alkyl,
(aryl)ₚaminosulfonylamino C₁₋₆ alkyl,
(aryl C₁₋₈ alkyl)ₚaminosulfonylamino,
(aryl C₁₋₈ alkyl)ₚaminosulfonylamino C₁₋₆ alkyl,
C₁₋₆ alkylthiocarbonylamino,
C₁₋₆ alkylthiocarbonylamino C₁₋₆ alkyl,
arylthiocarbonylamino C₁₋₆ alkyl,
aryl C₁₋₆ alkylthiocarbonylamino, and
aryl C₁₋₆ alkylthiocarbonylamino C₁₋₆ alkyl;
R⁹ is selected from the group consisting of hydrogen, methyl, and ethyl;
R¹⁰ is selected from the group consisting of
hydrogen, and
C₁₋₈ alkyl;
each m is independently an integer from 0 to 6;
each n is independently an integer from 0 to 6;
each p is independently an integer from 0 to 2;
each t is an integer from 0 to 3;
v is independently an integer from 0 to 6; and
"aryl," as used herein, refers to a monocyclic or polycyclic system comprising at least one aromatic ring, wherein the monocylic or polycyclic system contains 0, 1, 2, 3, or 4 heteroatoms chosen from N, O, or S, and wherein the monocylic or polycylic system is either unsubstituted or substituted with one or more groups independently selected from hydrogen, halogen, C₁₋₁₀ alkyl, C₃₋₈ cycloalkyl, aryl, aryl C₁₋₈ alkyl, amino, amino C₁₋₈ alkyl, C₁₋₃ acylamino, C₁₋₃ acylamino C₁₋₈ alkyl, C₁₋₆ alkylamino, C₁₋₆ alkylamino C₁₋₈ alkyl, C₁₋₆ dialkylamino, C₁₋₆ dialkylamino-C₁₋₈ alkyl, C₁₋₄ alkoxy, C₁₋₄ alkoxy C₁₋₆ alkyl, hydroxycarbonyl, hydroxycarbonyl C₁₋₆ alkyl, C₁₋₅ alkoxycarbonyl, C₁₋₃ alkoxycarbonyl C₁₋₆ alkyl, hydroxycarbonyl C₁₋₆ alkyloxy, hydroxy, hydroxy C₁₋₆ alkyl, cyano, trifluoromethyl, oxo or C₁₋₅ alkylcarbonyloxy;
and the pharmaceutically acceptable salts thereof.

2. The compound of Claim 1 wherein W is 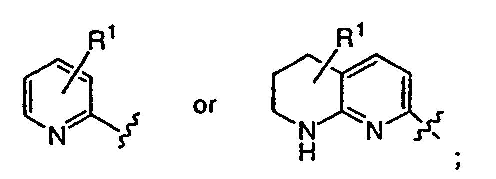
X is -(CH₂)ᵥ-, wherein any methylene (CH₂) carbon atom is either unsubstituted or substituted with one or two R¹ substitutents;
Y is selected from the group consisting of
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH2)m-O-(CH2)n-O-(CH2)p-,
-(CH2)m-O-(CH2)n-NR⁴-(CH2)p-,
-(CH2)m-NR⁴-(CH2)n-NR⁴-(CH2)p-,
and
-(CH2)m-NR⁴-(CH2)n-O-(CH2)p-;
wherein any methylene (CH₂) carbon atom in Y, other than in R⁴, can be substituted by one or two R³ substituents; and
Z is

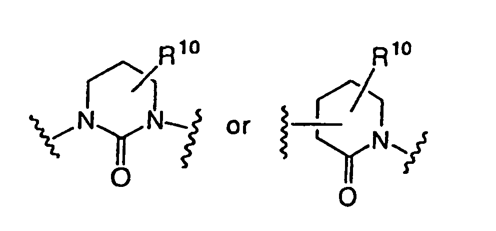

3. The compound of Claim 2 wherein Y is selected from the group consisting of
(CH₂)ₘ, (CH₂)ₘ-S-(CH₂)ₙ, and (CH₂)ₘ-NR⁴-(CH₂)ₙ,
wherein any methylene (CH₂) carbon atom in Y, other than in R⁴, can be substituted by one or two R³ substituents;
m and n are integers from 0-3;
and v is 0.

4. The compound of Claim 3 wherein R⁶, R⁷, and R⁸ are each hydrogen and R⁵ is selected from the group consisting of
hydrogen,
aryl,
C₁₋₈ alkyl,
aryl-C≡C-(CH₂)ₜ-,
aryl C₁₋₆ alkyl,
CH₂=CH-(CH₂)ₜ-, and
HC≡C-(CH₂)ₜ-.

5. The compound of Claim 4 wherein R⁹ is hydrogen.

6. The compound of Claim 5 wherein R⁵, R⁶, and R⁸ are each hydrogen and R⁷ is selected from the group consisting of
hydrogen,
aryl,
C₁₋₈ alkylcarbonylamino,
C₁₋₈ alkylsulfonylamino,
arylcarbonylamino,
arylsulfonylamino,
C₁₋₈ alkylsulfonylamino C₁₋₆ alkyl,
arylsulfonylamino C₁₋₆ alkyl,
aryl C₁₋₆ alkylsulfonylamino,
aryl C₁₋₆ alkylsulfonylamino C₁₋₆ alkyl,
C₁₋₈ alkoxycarbonylamino,
C₁₋₈ alkoxycarbonylamino C₁₋₈ alkyl,
aryloxycarbonylamino C₁₋₈ alkyl,
aryl C₁₋₈ alkoxycarbonylamino,
aryl C₁₋₈ alkoxycarbonylamino C₁₋₈ alkyl,
C₁₋₈ alkylcarbonylamino C₁₋₆ alkyl,
arylcarbonylamino C₁₋₆ alkyl,
aryl C₁₋₆ alkylcarbonylamino,
aryl C₁₋₆ alkylcarbonylamino C₁₋₆ alkyl,
aminocarbonylamino C₁₋₆ alkyl,
(C₁₋₈ alkyl)ₚaminocarbonylamino,
(C₁₋₈ alkyl)ₚaminocarbonylamino C₁₋₆ alkyl,
(aryl)ₚaminocarbonylamino C₁₋₆ alkyl,
arylaminocarbonylamino,
(aryl C₁₋₈ alkyl)ₚaminocarbonylamino,
(aryl C₁₋₈ alkyl)ₚaminocarbonylamino C₁₋₆ alkyl,
aminosulfonylamino C₁₋₆ alkyl,
(C₁₋₈ alkyl)ₚaminosulfonylamino,
(C₁₋₈ alkyl)ₚaminosulfonylamino C₁₋₆ alkyl,
(aryl)ₚaminosulfonylamino C₁₋₆ alkyl,
(aryl C₁₋₈ alkyl)ₚaminosulfonylamino,
(aryl C₁₋₈ alkyl)ₚaminosulfonylamino C₁₋₆ alkyl,
C₁₋₆ alkylthiocarbonylamino,
C₁₋₆ alkylthiocarbonylamino C₁₋₆ alkyl,
arylthiocarbonylamino C₁₋₆ alkyl,
aryl C₁₋₆ alkylthiocarbonylamino, and
aryl C₁₋₆ alkylthiocarbonylamino C₁₋₆ alkyl.

7. The compound of Claim 6 wherein R⁷ is selected from the group consisting of
hydrogen,
aryl,
C₁₋₈ alkylcarbonylamino,
aryl C₁₋₆ alkylcarbonylamino,
arylcarbonylamino,
C₁₋₈ alkylsulfonylamino,
aryl C₁₋₆ alkylsulfonylamino,
arylsulfonylamino,
C₁₋₈ alkoxycarbonylamino,
aryl C₁₋₈ alkoxycarbonylamino,
arylaminocarbonylamino,
(C₁₋₈ alkyl)ₚaminocarbonylamino,
(aryl C₁₋₈ alkyl)ₚaminocarbonylamino,
(C₁₋₈ alkyl)ₚaminosulfonylamino, and
(aryl C₁₋₈ alkyl)ₚaminosulfonylamino.

8. The compound of Claim 7 wherein R⁹ is hydrogen.

9. The compound of Claim 3 selected from the group consisting of:
ethyl 3(S)-(2,3-dihydro-benzofuran-6-yl)-3-{2-oxo-3-[3-(5,6,7,8-tetrahydro-[1,8]naphthyridin-2-yl)-propyl]-tetrahydro-pyrimidin-1-yl}-propionate;
ethyl 3(S)-(3-fluorophenyl)-3-(2-oxo-3(S or R)-[3-(5,6,7,8-tetrahydro-[1,8]naphthyridin-2-yl)-propyl]-piperidin-1-yl)-propionate;
ethyl 3(S)-(3-fluorophenyl)-3-(2-oxo-3(R or S)-[3-(5,6,7,8-tetrahydro-[1,8]naphthyridin-2-yl)-propyl]-piperidin-1-yl)-propionate;
3(S)-(2,3-dihydro-benzofuran-6-yl)-3-{2-oxo-3-[3-(5,6,7,8-tetrahydro-[1,8]naphthyridin-2-yl)-propyl]-tetrahydro-pyrimidin-1-yl}-propionic acid;
3(S)-(3-fluorophenyl)-3-(2-oxo-3(S or R)-[3-(5,6,7,8-tetrahydro-[1,8]naphthyridin-2-yl)-propyl]-piperidin-1-yl)-propionic acid;
3(S)-(3-fluorophenyl)-3-(2-oxo-3(R or S)-[3-(5,6,7,8-tetrahydro-[1,8]naphthyridin-2-yl)-propyl]-piperidin-1-yl)-propionic acid;
and the pharmaceutically acceptable salts thereof.

10. The compound of Claim 9 selected from the group consisting of:
3(S)-(2,3-dihydro-benzofuran-6-yl)-3-{2-oxo-3-[3-(5,6,7,8-tetrahydro-[1,8]naphthyridin-2-yl)-propyl]-tetrahydro-pyrimidin-1-yl}-propionic acid;
3(S)-(3-fluorophenyl)-3-(2-oxo-3(R or S)-[3-(5,6,7,8-tetrahydro-[1,8]naphthyridin-2-yl)-propyl]-piperidin-1-yl)-propionic acid;
3(S)-(3-fluorophenyl)-3-(2-oxo-3(S or R)-[3-(5,6,7,8-tetrahydro-[1,8]naphthyridin-2-yl)-propyl]-piperidin-1-yl)-propionic acid;
and the pharmaceutically acceptable salts thereof.

11. A pharmaceutical composition comprising a compound according to any previous Claim and a pharmaceutically acceptable carrier.

12. A process for making a pharmaceutical composition comprising combining a compound according to any of claims 1-10 and a pharmaceutically acceptable carrier.

13. A composition of Claim 11 which further comprises an active ingredient selected from the group consisting of
a) an organic bisphosphonate or a pharmaceutically acceptable salt or ester thereof,
b) an estrogen receptor modulator,
c) a cytotoxic/antiproliferative agent,
d) a matrix metalloproteinase inhibitor,
e) an inhibitor of epidermal-derived, fibroblast-derived, or platelet-derived growth factors,
f) an inhibitor of VEGF,
g) an inhibitor of Flk-1/KDR, Flt-1, Tck/Tie-2, or Tie-1,
h) a cathepsin K inhibitor, and
i) a prenylation inhibitor, such as a farnesyl transferase inhibitor or a geranylgeranyl transferase inhibitor or a dual famesyl/geranylgeranyl transferase inhibitor; and mixtures thereof.

14. The composition of Claim 13 wherein said active ingredient is selected from the group consisting of
a) an organic bisphosphonate or a pharmaceutically acceptable salt or ester thereof,
b) an estrogen receptor modulator, and
c) a cathepsin K inhibitor; and mixtures thereof.

15. The composition of Claim 14 wherein said organic bisphosphonate or pharmaceutically acceptable salt or ester thereof is alendronate monosodium trihydrate.

16. The composition of Claim 13 wherein said active ingredient is selected from the group consisting of
a) a cytotoxic/antiproliferative agent,
b) a matrix metalloproteinase inhibitor,
c) an inhibitor of epidermal-derived, fibroblast-derived, or platelet-derived growth factors,
d) an inhibitor of VEGF, and
e) an inhibitor of Flk-1/KDR, Flt-1, Tck/Tie-2, or Tie-1; and mixtures thereof.

17. Use of a compound according to any of claims 1-10 for the manufacture of a medicament for eliciting an integrin receptor antagonizing effect in a mammal.

18. Use according to claim 17 wherein the integrin receptor antagonizing effect is an αvβ3 antagonizing effect.

19. Use according to claim 18 wherein the αvβ3 antagonizing effect is selected from the group consisting of inhibition of bone resorption, restenosis, angiogenesis, diabetic retinopathy, macular degeneration, inflammation, viral disease, and tumor growth.

20. Use according to claim 19 wherein the αvβ3 antagonizing effect is the inhibition of bone resorption.

21. Use according to claim 17 wherein the integrin receptor antagonizing effect is an αvβ5 antagonizing effect.

22. Use according to claim 21 wherein the αvβ5 antagonizing effect is selected from the group consisting of inhibition of restenosis, angiogenesis, diabetic retinopathy, macular degeneration, inflammation, and tumor growth.

23. Use according to claim 17 wherein the integrin receptor antagonizing effect is a dual αvβ3/αvβ5 antagonizing effect.

24. Use according to claim 23 wherein the dual αvβ3/αvβ5 antagonizing effect is selected from the group consisting of inhibition of bone resorption, restenosis, angiogenesis, diabetic retinopathy, macular degeneration, inflammation, viral disease, and tumor growth.

25. Use according to claim 17 wherein the integrin antagonizing effect is an αvβ6 antagonizing effect.

26. Use according to claim 25 wherein the αvβ6 antagonizing effect is selected from the group consisting of angiogenesis, inflammatory response, and wound healing.

27. The composition of claim 11 for use in eliciting an integrin receptor antagonizing effect in a mammal.

28. The composition of claim 11 for use in treating or preventing a condition mediated by antagonism of an integrin receptor in a mammal.

29. The composition of claim 11 for use in inhibiting bone resorption in a mammal.

30. The composition of claim 14 for use in inhibiting bone resorption in a mammal.

31. The composition of claim 16 for use in treating tumor growth in a mammal.

32. A compound according to claim 1 for use in treating tumor growth in a mammal in combination with radiation therapy.

## Patentansprüche

1. Eine Verbindung der Formel 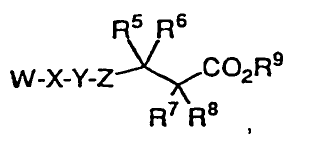 wobei
W ausgewählt ist aus der Gruppe, bestehend aus 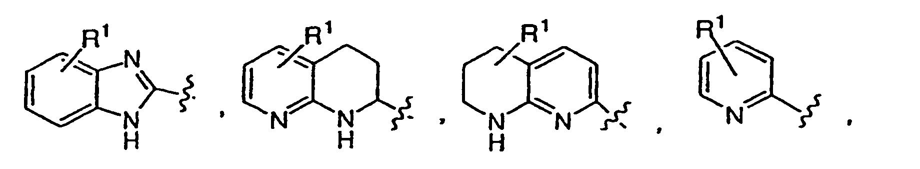 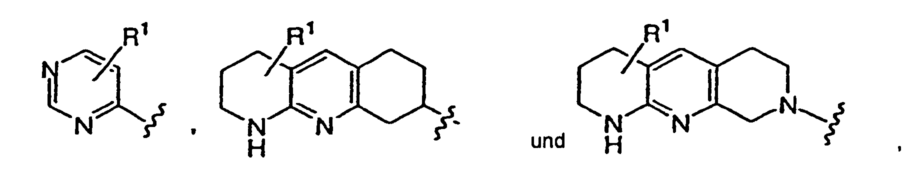
X -(CH₂)ᵥ- ist, wobei jedes beliebige Methylen(CH₂)-Kohlenstoffatom entweder unsubstituiert oder mit ein oder zwei R¹-Substituenten substituiert ist,
Y ausgewählt ist aus der Gruppe, bestehend aus
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH₂)ₘ-O-(CH₂)ₙ-O-(CH₂)ₚ-,
-(CH₂)ₘ-O-(CH₂)ₙ-NR⁴-(CH₂)ₚ -,
-(CH₂)ₘ-NR⁴-(CH₂)ₙ-NR⁴-(CH₂)ₚ -,
-(CH₂)ₘ-O-(CH₂)ₙ-S-(CH₂)ₚ -,
-(CH₂)ₘ-S-(CH₂)ₙ-S-(CH₂)ₚ -,
-(CH₂)ₘ-NR⁴-(CH₂)ₙ-S-(CH₂)ₚ -,
-(CH₂)ₘ-NR⁴ -(CH₂)ₙ-O-(CH₂)ₚ -,
-(CH₂)ₘ-S-(CH₂)ₙ-O-(CH₂)ₚ -
und
-(CH₂)ₘ-S-(CH₂)ₙ-NR⁴-(CH₂)ₚ -,
wobei jedes beliebige Methylen(CH₂)-Kohlenstoffatom in Y, anders als in R⁴, durch ein oder zwei
R³-Substituenten substituiert sein kann,
Z ausgewahlt ist aus der Gruppe, bestehend aus 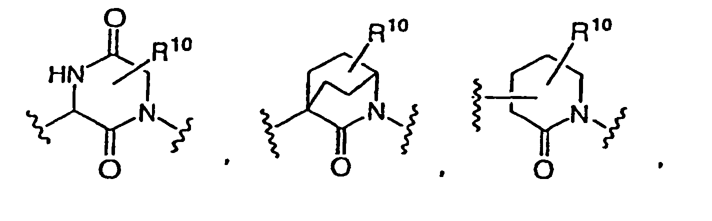 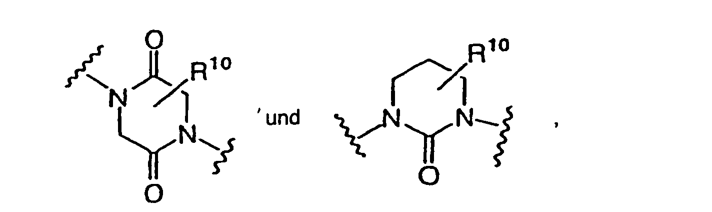
R¹ ausgewählt ist aus der Gruppe, bestehend aus
Wasserstoff,
Halogen,
C₁₋₁₀-Alkyl,
C₃₋₈-Cycloalkyl,
C₃₋₈-Cycloheteroalkyl,
Hydroxy,
Nitro,
Cyano,
Trifluormethyl und
Trifluormethoxy,
jedes R³ unabhängig ausgewählt ist aus der Gruppe, bestehend aus
Wasserstoff,
Fluor,
Trifluormethyl,
Aryl,
C₁₋₈-Alkyl,
Aryl-C₁₋₆-alkyl,
Hydroxyl,
Oxo,
Arylaminocarbonyl,
Aryl-C₁₋₅-alkylaminocarbonyl,
Aminocarbonyl und
Aminocarbonyl-C₁₋₆-alkyl,
jedes R⁴ unabhangig ausgewahlt ist aus der Gruppe, bestehend aus
Wasserstoff,
Aryl,
C₃₋₈-Cycloalkyl,
C₁₋₈-Alkyl,
C₁₋₈-Alkylcarbonyl,
Arylcarbonyl,
C₁₋₆-Alkylsulfonyl,
Arylsulfonyl,
Aryl-C₁₋₆-alkylsulfonyl,
Aryl-C₁₋₆-alkylcarbonyl,
C₁₋₈-Alkylaminocarbonyl,
Aryl-C₁₋₅-alkylaminocarbonyl,
Aryl-C₁₋₈-alkoxycarbonyl und
C₁₋₈-Alkoxycarbonyl,
R⁵ und R⁶ jeweils unabhängig ausgewählt sind aus der Gruppe, bestehend aus
Wasserstoff,
Aryl,
C₁₋₈-Alkyl,
Aryl-C≡C-(CH₂)ₜ-,
Aryl-C₁₋₆-alkyl,
CH₂=CH-(CH₂)ₜ- und
HC≡C-(CH₂)ₜ-,
R⁷ und R⁸ jeweils unabhängig ausgewählt sind aus der Gruppe, bestehend aus
Wasserstoff,
Aryl,
C₁₋₈-Alkylcarbonylamino,
Arylcarbonylamino,
C₁₋₈-Alkylsulfonylamino,
Arylsulfonylamino,
C₁₋₈-Alkylsulfonylamino-C₁₋₆-alkyl,
Arylsulfonylamino-C₁₋₆-alkyl,
Aryl-C₁₋₆-alkylsulfonylamino,
Aryl-C₁₋₆-alkylsulfonylamino-C₁₋₆-alkyl,
C₁₋₈-Alkoxycarbonylamino,
C₁₋₈-Alkoxycarbonylamino-C₁₋₈-alkyl,
Aryloxycarbonylamino-C₁₋₈-alkyl,
Aryl-C₁₋₈-alkoxycarbonylamino,
Aryl-C₁₋₈-alkoxycarbonylamino-C₁₋₈-alkyl,
C₁₋₈-Alkylcarbonylamino-C₁₋₆-alkyl,
Arylcarbonylamino-C₁₋₆-alkyl,
Aryl-C₁₋₆-alkylcarbonylamino,
ArylC₁₋₆-alkylcarbonylamino-C₁₋₆-alkyl,
Aminocarbonylamino-C₁₋₆-alkyl,
(C₁₋₈-Alkyl)ₚaminocarbonylamino,
(C₁₋₈-Alkyl)ₚaminocarbonylamino-C₁₋₆-alkyl,
(Aryl)ₚaminocarbonylamino-C₁₋₆-alkyl,
(Aryl-C₁₋₈-alkyl)ₚaminocarbonylamino,
(Aryl-C₁₋₈-alkyl)ₚaminocarbonylamino-C₁₋₆-alkyl,
Aminosulfonylamino-C₁₋₆-alkyl,
(C₁₋₈-Alkyl)ₚaminosulfonylamino,
(C₁₋₈-Alkyl)ₚaminosulfonylamino-C₁₋₆-alkyl,
(Aryl)ₚaminosulfonylamino-C₁₋₆-alkyl,
(Aryl-C₁₋₈-alkyl)ₚaminosulfonylamino,
(Aryl-C₁₋₈-alkyl)ₚaminosulfonylamino-C₁₋₆-alkyl,
C₁₋₆-Alkylthiocarbonylamino,
C₁₋₆-Alkylthiocarbonylamino-C₁₋₆-alkyl,
Arylthiocarbonylamino-C₁₋₆-alkyl,
Aryl-C₁₋₆-alkylthiocarbonylamino und
Aryl-C₁₋₆-alkylthiocarbonylamino-C₁₋₆-alkyl,
R⁹ ausgewählt ist aus der Gruppe, bestehend aus
Wasserstoff, Methyl und Ethyl,
R¹⁰ ausgewählt ist aus der Gruppe, bestehend aus
Wasserstoff und
C₁₋₈-Alkyl,
jedes m unabhängig eine ganze Zahl von 0 bis 6 ist,
jedes n unabhängig eine ganze Zahl von 0 bis 6 ist,
jedes p unabhängig eine ganze Zahl von 0 bis 2 ist,
jedes t eine ganze Zahl von 0 bis 3 ist,
v unabhängig eine ganze Zahl von 0 bis 6 ist und
"Aryl", so wie es hier verwendet wird, ein monocyclisches oder polycyclisches System bedeutet, das wenigstens 1 aromatischen Ring enthält, wobei das monocyclische oder polycyclische System 0, 1, 2, 3 oder 4 Heteroatome, ausgewählt aus N, O oder S, enthält, und wobei das monocyclische oder polycyclische System entweder unsubstituiert oder substituiert ist mit einer oder mehreren Gruppen, unabhängig ausgewählt aus Wasserstoff, Halogen, C₁₋₁₀-Alkyl, C₃₋₈-Cycloalkyl, Aryl, Aryl-C₁₋₈-alkyl, Amino, Amino-C₁₋₈-alkyl, C₁₋₃-Acylamino, C₁₋₃-Acylamino-C₁₋₈-alkyl, C₁₋₆-Alkylamino, C₁₋₆-Alkylamino-C₁₋₈-alkyl, C₁₋₆-Dialkylamino, C₁₋₆-Dialkylamino-C₁₋₈-alkyl, C₁₋₄-Alkoxy, C₁₋₄-Alkoxy-C₁₋₆-alkyl, Hydroxycarbonyl, Hydroxycarbonyl-C₁₋₆-alkyl, C₁₋₅-Alkoxycarbonyl, C₁₋₃-Alkoxycarbonyl-C₁₋₆-alkyl, Hydroxycarbonyl-C₁₋₆-alkyloxy, Hydroxy, Hydroxy-C₁₋₆-alkyl, Cyano, Trifluormethyl, Oxo oder C₁₋₅-Alkylcarbonyloxy,
und die pharmazeutisch annehmbaren Salze davon.

2. Die Verbindung nach Anspruch 1, wobei W 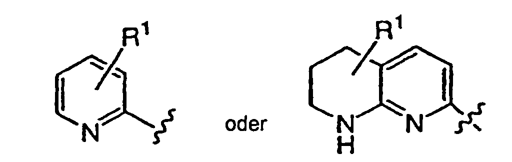 ist,
X -(CH₂)ᵥ- ist, wobei jedes beliebige Methylen(CH₂)-Kohlenstoffatom entweder unsubstituiert oder mit ein oder zwei R¹-Substituenten substituiert ist,
Y ausgewählt ist aus der Gruppe, bestehend aus
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH2)m-O-(CH2)n-O-(CH2)p-,
-(CH2)m-O-(CH2)n-NR⁴-(CH2)p-,
-(CH2)m-NR⁴-(CH2)n-NR⁴-(CH2)p-
und
-(CH2)m-NR⁴-(CH2)n-O-(CH2)p-,
wobei jedes beliebige Methylen(CH₂)-Kohlenstoffatom in Y, anders als in R⁴, durch ein oder zwei
R³-Substituenten substituiert sein kann, und
Z 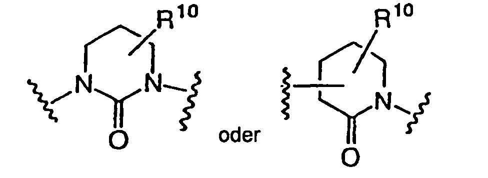 ist.

3. Die Verbindung nach Anspruch 2, wobei Y ausgewählt ist aus der Gruppe, bestehend aus
(CH₂)_{m'}(CH₂)ₘ-S-(CH₂)ₙ und (CH₂)ₘNR⁴-(CH₂)ₙ,
wobei jedes beliebige Methylen(CH₂)-Kohlenstoffatom in Y, anders als in R⁴, durch ein oder zwei
R³-Substituenten substituiert sein kann,
m und n ganze Zahlen von 0-3 sind
und v 0 ist.

4. Die Verbindung nach Anspruch 3, wobei R⁶, R⁷ und R⁸ jeweils Wasserstoff sind und R⁵ ausgewählt ist aus der Gruppe, bestehend aus
Wasserstoff,
Aryl,
C₁₋₈-Alkyl,
Aryl-C≡C-(CH₂)ₜ-,
Aryl-C₁₋₆-alkyl,
CH₂=CH-(CH₂)ₜ- und
HC≡C-(CH₂)ₜ-.

5. Die Verbindung nach Anspruch 4, wobei R⁹ Wasserstoff ist.

6. Die Verbindung nach Anspruch 5, wobei R⁵, R⁶ und R⁸ jeweils Wasserstoff sind und R⁷ ausgewahlt ist aus der Gruppe, bestehend aus
Wasserstoff,
Aryl,
C₁₋₈-Alkylcarbonylamino,
C₁₋₈-Alkylsulfonylamino,
Arylcarbonylamino,
Arylsulfonylamino,
C₁₋₈-Alkylsulfonylamino-C₁₋₆-alkyl,
Arylsulfonylamino-C₁₋₆-alkyl,
Aryl-C₁₋₆-alkylsulfonylamino,
Aryl-C₁₋₆-alkylsulfonylamino-C₁₋₆-alkyl,
C₁₋₈-Alkoxycarbonylamino,
C₁₋₈-Alkoxycarbonylamino-C₁₋₈-alkyl,
Aryloxycarbonylamino-C₁₋₈-alkyl,
Aryl-C₁₋₈-alkoxycarbonylamino,
Aryl-C₁₋₈-alkoxycarbonylamino-C₁₋₈-alkyl,
C₁₋₈-Alkylcarbonylamino-C₁₋₆-alkyl,
Arylcarbonylamino-C₁₋₆-alkyl,
Aryl-C₁₋₆-alkylcarbonylamino,
Aryl-C₁₋₆-alkylcarbonylamino-C₁₋₆-alkyl,
Aminocarbonylamino-C₁₋₆-alkyl,
(C₁₋₈-Alkyl)ₚaminocarbonylamino,
(C₁₋₈-Alkyl)ₚaminocarbonylamino-C₁₋₆-alkyl,
(Aryl)ₚaminocarbonylamino-C₁₋₆-alkyl,
Arylaminocarbonylamino,
(Aryl-C₁₋₈-alkyl)ₚaminocarbonylamino,
(Aryl-C₁₋₈-alkyl)ₚaminocarbonylamino-C₁₋₆-alkyl,
Aminosulfonylamino-C₁₋₆-alkyl,
(C₁₋₈-Alkyl)ₚaminosulfonylamino,
(C₁₋₈-Alkyl)ₚaminosulfonylamino-C₁₋₆-alkyl,
(Aryl)ₚaminosulfonylamino-C₁₋₆-alkyl,
(Aryl-C₁₋₈-alkyl)ₚaminosulfonylamino,
(Aryl-C₁₋₈-alkyl)ₚaminosulfonylamino-C₁₋₆-alkyl,
C₁₋₆-Alkylthiocarbonylamino,
C₁₋₆-Alkylthiocarbonylamino-C₁₋₆-alkyl,
Arylthiocarbonylamino-C₁₋₆-alkyl,
Aryl-C₁₋₆-alkylthiocarbonylamino und
Aryl-C₁₋₆-alkylthiocarbonylamino-C₁₋₆-alkyl.

7. Die Verbindung nach Anspruch 6, wobei R⁷ ausgewählt ist aus der Gruppe, bestehend aus
Wasserstoff,
Aryl,
C₁₋₈-Alkylcarbonylamino,
Aryl-C₁₋₆-alkylcarbonylamino,
Arylcarbonylamino,
C₁₋₈-Alkylsulfonylamino,
Aryl-C₁₋₆-alkylsulfonylamino,
Arylsulfonylamino,
C₁₋₈-Alkoxycarbonylamino,
Aryl-C₁₋₈-alkoxycarbonylamino,
Arylaminocarbonylamino,
(C₁₋₈-Alkyl)ₚaminocarbonylamino,
(Aryl-C₁₋₈-alkyl)ₚaminocarbonylamino,
(C₁₋₈-Alkyl)ₚaminosulfonylamino und
(Aryl-C₁₋₈-alkyl)ₚaminosulfonylamino.

8. Die Verbindung nach Anspruch 7, wobei R⁹ Wasserstoff ist.

9. Die Verbindung nach Anspruch 3, ausgewählt aus der Gruppe, bestehend aus:
Ethyl-3(S)-(2,3-dihydrobenzofuran-6-yl)-3-{2-oxo-3-[3-(5,6,7,8-tetrahydro[1,8]naphthyridin-2-yl)-propyl]tetrahydropyrimidin-1-yl}propionat,
Ethyl-3(S)-(3-fluorphenyl)-3-(2-oxo-3(S oder R)-[3-(5,6,7,8-tetrahydro[1,8]naphthyridin-2-yl)propyl]-pipendin-1-yl)propionat,
Ethyl-3(S)-(3-fluorphenyl)-3-(2-oxo-3(R oder S)-[3-(5,6,7,8-tetrahydro[1,8]naphthyridin-2-yl)propyl]-piperidin-1-yl)propionat,
3(S)-(2,3-Dihydrobenzofuran-6-yl)-3-{2-oxo-3-[3-(5,6,7,8-tetrahydro[1,8]naphthyridin-2-yl)propyl]-tetrahydropyrimidin-1-yl}propionsäure,
3(S)-(3-Fluorphenyl)-3-(2-oxo-3(S oder R)-[3-(5,6,7,8-tetrahydro[1,8]naphthyridin-2-yl)propyl]-pipendin-1-yl)propionsäure,
3(S)-(3-Fluorphenyl)-3-(2-oxo-3(R oder S)-[3-(5,6,7,8-tetrahydro[1,8]naphthyridin-2-yl)propyl]-piperidin-1-yl)propionsäure,
und die pharmazeutisch annehmbaren Salze davon.

10. Die Verbindung nach Anspruch 9, ausgewählt aus der Gruppe, bestehend aus:
3(S)-(2,3-Dihydrobenzofuran-6-yl)-3-{2-oxo-3-[3-(5,6,7,8-tetrahydro[1,8]naphthyridin-2-yl)propyl]-tetrahydropyrimidin-1-yl}propionsäure,
3(S)-(3-Fluorphenyl)-3-(2-oxo-3(R oder S)-[3-(5,6,7,8-tetrahydro[1,8]naphthyridin-2-yl)propyl]-piperidin-1-yl)propionsäure,
3(S)-(3-Fluorphenyl)-3-(2-oxo-3(S oder R)-[3-(5,6,7,8-tetrahydro[1,8]naphthyridin-2-yl)propyl]-piperidin-1-yl)propionsäure,
und die pharmazeutisch annehmbaren Salze davon.

11. Eine pharmazeutische Zusammensetzung, die eine Verbindung gemäß irgendeinem vorherigen Anspruch und einen pharmazeutisch annehmbaren Träger enthält.

12. Ein Verfahren zur Herstellung einer pharmazeutischen Zusammensetzung, welches das Kombinieren einer Verbindung gemäß irgendeinem der Ansprüche 1 bis 10 und eines pharmazeutisch annehmbaren Trägers umfaßt.

13. Eine Zusammensetzung nach Anspruch 11, die ferner einen Wirkstoff umfaßt, ausgewählt aus der Gruppe, bestehend aus
a) einem organischen Bisphosphonat oder einem pharmazeutisch annehmbaren Salz oder Ester davon,
b) einem Östrogenrezeptormodulator,
c) einem zytotoxischen/antiproliferativen Mittel,
d) einem Matrixmetalloproteinaseinhibitor,
e) einem Inhibitor der epidermalen, Fibroblasten- oder Thrombozyten-Wachstumsfaktoren,
f) einem VEGF-Inhibitor,
g) einem Flk-1/KDR-, Flt-1-, Tck/Tie-2- oder Tie-1-Inhibitor,
h) einem Kathepsin-K-Inhibitor und
i) einem Prenylierungsinhibitor, wie z.B. einem Farnesyltransferaseinhibitor oder einem Geranylgeranyltransferaseinhibitor oder einem doppelten Farnesyl/Geranylgeranyl-Transferaseinhibitor, und Mischungen davon.

14. Die Zusammensetzung nach Anspruch 13, wobei der Wirkstoff ausgewählt ist aus der Gruppe, bestehend aus
a) einem organischen Bisphosphonat oder einem pharmazeutisch annehmbaren Salz oder Ester davon,
b) einem Östrogenrezeptormodulator und
c) einem Kathepsin-K-Inhibitor und Mischungen davon.

15. Die Zusammensetzung nach Anspruch 14, wobei das organische Bisphosphonat oder das pharmazeutisch annehmbare Salz oder der pharmazeutisch annehmbare Ester davon Alendronat-Mononatrium-Tnhydrat ist.

16. Die Zusammensetzung nach Anspruch 13, wobei der Wirkstoff ausgewählt ist aus der Gruppe, bestehend aus
a) einem zytotoxischen/antiproliferativen Mittel,
b) einem Matrixmetalloproteinaseinhibitor,
c) einem Inhibitor der epidermalen, Fibroblasten- oder Thrombozyten-Wachstumsfaktoren,
d) einem VEGF-Inhibitor und
e) einem Flk-1/KDR-, Flt-1-, Tck/Tie-2- oder Tie-1-Inhibitor und Mischungen davon.

17. Die Verwendung einer Verbindung gemäß irgendeinem der Ansprüche 1 bis 10 zur Herstellung eines Medikaments zur Herbeiführung einer Integrinrezeptorantagonisierungswirkung bei einem Säugetier.

18. Die Verwendung gemäß Anspruch 17, wobei die Integrinrezeptorantagonisierungswirkung eine αvβ3-Antagonisierungswirkung ist.

19. Die Verwendung gemäß Anspruch 18, wobei die αvβ3-Antagonisierungswirkung ausgewählt ist aus der Gruppe, bestehend aus der Inhibierung von Knochenresorption, Restenose, Angiogenese, diabetischer Retinopathie, Makuladegeneration, Entzündung, Viruserkrankung und Tumorwachstum.

20. Die Verwendung gemäß Anspruch 19, wobei die αvβ3-Antagonisierungswirkung die Inhibierung der Knochenresorption ist.

21. Die Verwendung gemäß Anspruch 17, wobei die Integrinrezeptorantagonisierungswirkung eine αvβ5-Antagonisierungswirkung ist.

22. Die Verwendung gemäß Anspruch 21, wobei die αvβ5-Antagonisierungswirkung ausgewahlt ist aus der Gruppe, bestehend aus der Inhibierung von Restenose, Angiogenese, diabetischer Retinopathie, Makuladegeneration, Entzündung und Tumorwachstum.

23. Die Verwendung gemäß Anspruch 17, wobei die Integrinrezeptorantagonisierungswirkung eine doppelte αvβ3/αvβ5-Antagonisierungswirkung ist.

24. Die Verwendung gemäß Anspruch 23, wobei die doppelte αvβ3/αvβ5-Antagonisierungswirkung ausgewählt ist aus der Gruppe, bestehend aus der Inhibierung von Knochenresorption, Restenose, Angiogenese, diabetischer Retinopathie, Makuladegeneration, Entzündung, Viruserkrankung und Tumorwachstum.

25. Die Verwendung gemäß Anspruch 17, wobei die Integrinrezeptorantagonisierungswirkung eine αvβ6-Antagonisierungswirkung ist.

26. Die Verwendung gemäß Anspruch 25, wobei die αvβ6-Antagonisierungswirkung ausgewählt ist aus der Gruppe, bestehend aus Angiogenese, Entzündungsreaktion und Wundheilung.

27. Die Zusammensetzung nach Anspruch 11 zur Verwendung zur Herbeifuhrung einer Integrinrezeptorantagonisierungswirkung bei einem Säugetier.

28. Die Zusammensetzung nach Anspruch 11 zur Verwendung zur Behandlung oder Prävention eines Zustandes, der durch den Antagonismus eines Integrinrezeptors bei einem Säugetier vermittelt wird.

29. Die Zusammensetzung nach Anspruch 11 zur Verwendung zur Inhibierung von Knochenresorption bei einem Säugetier.

30. Die Zusammensetzung nach Anspruch 14 zur Verwendung zur Inhibierung von Knochenresorption bei einem Säugetier.

31. Die Zusammensetzung nach Anspruch 16 zur Verwendung zur Behandlung von Tumorwachstum bei einem Säugetier.

32. Eine Verbindung gemäß Anspruch 1 zur Verwendung zur Behandlung von Tumorwachstum bei einem Säugetier in Kombination mit einer Strahlentherapie.

## Revendications

1. Composé de formule 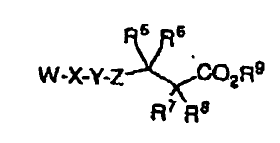 dans laquelle W est choisi dans l'ensemble consistant en 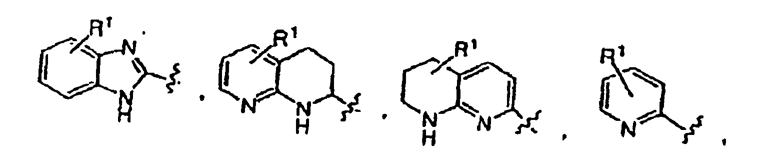 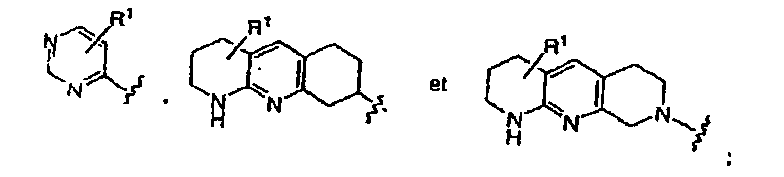
X représente
-(CH₂)ᵥ-, où l'atome de carbone de chaque groupe méthylène (CH₂) est non substitué ou porte un ou deux substituants R¹ ;
Y est choisi dans l'ensemble consistant en
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
- (CH₂)ₘ-O-(CH₂)ₙ-O-(CH₂)ₚ-,
- (CH₂)ₘ-O-(CH₂)ₙ-NR⁴-(CH₂)ₚ-,
-(CH₂)ₘ-NR⁴-(CH₂)ₙ-NR⁴-(CH₂)ₚ-,
-(CH₂)ₘ-O-(CH₂)ₙ-S-(CH₂)ₚ-,
- (CH₂)ₘ-S-(CH₂)ₙ-S-(CH₂)ₚ-,
- (CH₂)ₘ-NR⁴-(CH₂)ₙ-S-(CH₂)ₚ-,
- (CH₂)ₘ-NR⁴-(CH₂)ₙ-O-(CH₂)ₚ-,
-(CH₂)ₘ-S-(CH₂)ₙ-O-(CH₂)ₚ-,
et
-(CH₂)ₘ-S-(CH₂)ₙ-NR⁴-(CH₂)ₚ-,
où l'atome de carbone de chaque groupe méthylène (CH₂) dans Y, différent de R⁴, peut porter un ou deux substituants R³ ;
Z est choisi dans l'ensemble consistant en 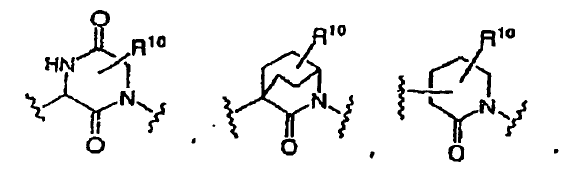 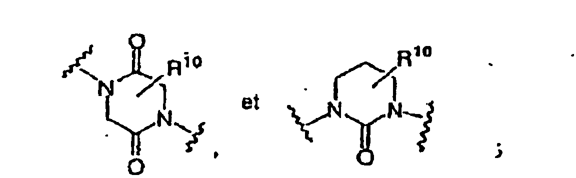
R¹ est choisi dans l'ensemble consistant en
un atome d'hydrogène,
un atome d'halogène,
un groupe alkyle en C₁₋₁₀,
un groupe cycloalkyle en C₃₋₈,
un groupe cyclohétéroalkyle en C₃₋₈,
un groupe hydroxy,
un groupe nitro,
un groupe cyano,
un groupe trifluorométhyle, et
un groupe trifluorométhoxy
chaque R³ est indépendant choisi dans l'ensemble consistant en
un atome d'hydrogène,
un atome de fluor,
un groupe trifluorométhyle,
un groupe aryle,
un groupe alkyle en C₁₋₈,
un groupe aryl(alkyle en C₁₋₆),
un groupe hydroxyle,
un groupe oxo,
un groupe arylaminocarbonyle,
un groupe aryl-(alkyle en C₁₋₅)aminocarbonyle,
un groupe aminocarbonyle, et
un groupe aminocarbonyl-(alkyl en C₁₋₆) ;
chaque R⁴ est indépendant choisi dans l'ensemble consistant en
un atome d'hydrogène,
un groupe aryle,
un groupe cycloalkyle en C₃₋₈,
un groupe alkyle en C₁₋₈,
un groupe (alkyle en C₁₋₈)carbonyle,
un groupe arylcarbonyle,
un groupe (alkyle en C₁₋₆)sulfonyle,
un groupe arylsulfonyle,
un groupe aryl-(alkyle en C₁₋₆)sulfonyle,
un groupe aryl-(alkyle en C₁₋₆)carbonyle,
un groupe (alkyle en C₁₋₈)aminocarbonyle,
un groupe aryl-(alkyle en C₁₋₅)aminocarbonyle,
un groupe aryl-(alcoxy en C₁₋₈)carbonyle, et
un groupe (alcoxy en C₁₋₈)carbonyle ;
R⁵ et R⁶ sont chacun indépendamment choisis dans l'ensemble consistant en
un atome d'hydrogène,
un groupe aryle,
un groupe alkyle en C₁₋₈,
un groupe aryl-C≡C-(CH₂)ₜ-,
un groupe aryl-(alkyle en C₁₋₆),
un groupe CH₂=CH-(CH₂)ₜ-, et
un groupe HC≡CH-(CH₂)ₜ- ;
R⁷ et R⁸ sont chacun indépendamment choisi dans l'ensemble consistant en
un atome d'hydrogène,
un groupe aryle,
un groupe (alkyle en C₁₋₈)carbonylamino,
un groupe arylcarbonylamino,
un groupe (alkyle en C₁₋₈)sulfonylamino,
un groupe arylsulfonylamino,
un groupe (alkyle en C₁₋₈)aulfonylamino-(alkyle en C₁₋₆),
un groupe arylsulfonylamino-(alkyle en C₁₋₆),
un groupe aryl-(alkyle en C₁₋₆)sulfonylamino,
un groupe aryl- (alkyle en C₁₋₆)sulfonylamino-(alkyle en C₁₋₆),
un groupe (alcoxy en C₁₋₈)carbonylamino,
un groupe (alcoxy en C₁₋₈)carbonylamino-(alkyle en C₁₋₈),
un groupe aryloxycarbonylamino-(alkyle en C₁₋₈),
un groupe aryl(alcoxy en C₁₋₈)carbonylamino,
un groupe aryl(alcoxy en C₁₋₈)carbonylamino-(alkyle en C₁₋₈),
un groupe (alkyle en C₁₋₈)carbonylamino-(alkyle en C₁₋₆),
un groupe arylcarbonylamino-(alkyle en C₁₋₆),
un groupe aryl-(alkyle en C₁₋₆)carbonylamino,
un groupe aryl-(alkyle en C₁₋₆)carbonylamino(alkyle en C₁₋₆),
un groupe aminocarbonylamino(alkyle en C₁₋₆),
un groupe (alkyle en C₁₋₈)ₚaminocarbonylamino,
un groupe (alkyle en C₁₋₈)ₚaminocarbonylamino(alkyle en C₁₋₆),
un groupe (aryl)ₚaminocarbonylamino(alkyle en C₁₋₆),
un groupe ((aryl)-(alkyle en C₁₋₈))ₚ-aminocarbonylamino,
un groupe ((aryl)-(alkyle en C₁₋₈))ₚ-aminocarbonylamino-(alkyle en C₁₋₆),
un groupe aminosulfonylamino(alkyle en C₁₋₆),
un groupe (alkyle en C₁₋₈)ₚaminosulfonylamino,
un groupe (alkyle en C₁₋₈)ₚaminosulfonylamino(alkyle en C₁₋₆),
un groupe (aryl)ₚaminoaulfonylamino(alkyle en C₁₋₆),
un groupe ((aryl)-(alkyle en C₁₋₈))ₚ-aminosulfonylamino,
un groupe ((aryl)-(alkyle en C₁₋₈))ₚ-aminosulfonylamino-(alkyle en C₁₋₆),
un groupe (alkyle en C₁₋₆)thiocarbonylamino,
un groupe (alkyle en C₁₋₆)thiocarbonylamino-(alkyle en C₁₋₆),
un groupe arylthiocarbonylamino(alkyle en C₁₋₆),
un groupe aryl(alkyle en C₁₋₆)thiocarbonylamino, et
un groupe aryl (alkyle en C₁₋₆)thiocarbonylamino(alkyle en C₁₋₆) ;
R⁹ est choisi dans l'ensemble consistant en
un atome d'hydrogène, un groupe méthyle et un groupe éthyle ;
R¹⁰ est choisi dans l'ensemble consistant en
un atome d'hydrogène et
un groupe alkyle en C₁₋₈ ;
chaque m est indépendamment un nombre entier de 0 à 6 ;
chaque n est indépendamment un nombre entier de 0 à 6 ;
chaque p est indépendamment un nombre entier de 0 à 2 ;
chaque t est un nombre entier de 0 à 3 ;
v est indépendamment un nombre entier de 0 à 6 ; et
un "groupe aryle", tel que le terme est utilisé ici, désigne un système monocyclique ou polycyclique comprenant au moins un cycle aromatique, lequel système monocyclique ou polycyclique contient 0, 1, 2, 3 ou 4 hétéroatomes choisis parmi N, O ou S, et lequel système monocycique ou polycyclique est non substitué ou substitué par un ou plusieurs groupes indépendamment choisis parmi un atome d'hydrogène, d'halogène, un groupe alkyle en C₁₋₁₀, cycloalkyle en C₃₋₈, aryle, aryl-(alkyle en C₁₋₈), amino, amino-(alkyle en C₁₋₈), acylamino en C₁₋₃, (acylamino en C₁₋₃)-(alkyle en C₁₋₈), (alkyle en C₁₋₆)amino, (alkyle en C₁₋₆)-amino-(alkyle en C₁₋₈), di-(alkyle en C₁₋₆)amino, di-(alkyle en C₁₋₆) amino-(alkyle en C₁₋₈), alcoxy en C₁₋₄, (alcoxy en C₁₋₄)-(alkyle en C₁₋₆), hydroxycarbonyle, hydroxycarbonyl-(alkyle en C₁₋₆), (alcoxy en C₁₋₅)carbonyle, (alcoxy en C₁₋₃)carbonyl-(alkyle en C₁₋₆), hydroxycarbonyl- (alkyloxy en C₁₋₆), hydroxy, hydroxy-(alkyle en C₁₋₆), cyano, trifluorométhyle, oxo ou (alkyle en C₁₋₅)carbonyloxy ;
et leurs sels pharmaceutiquement acceptables.

2. Composé de formule 1 dans laquelle W est 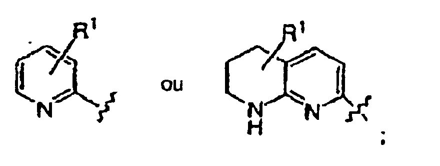
X représente-(CH₂)ᵥ-, où l'atome de carbone de chaque groupe méthylène (CH₂) est non substitué ou porte un ou deux substituants R¹ ;
Y est choisi dans l'ensemble consistant en
-(CH₂)ₘ-,
-(CH₂)ₘ-O-(CH₂)ₙ-,
- (CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH₂)ₘ-O-(CH₂)ₙ-O-(CH₂)ₚ-,
-(CH₂)ₘ-O-(CH₂)ₙ-NR⁴-(CH₂)ₚ-,
- (CH₂)ₘ-NR⁴-(CH₂)ₙ-NR⁴-(CH₂)ₚ-,
et
-(CH₂)ₘ-NR⁴-(CH₂)ₙ-O-(CH₂)ₚ-,
où l'atome de carbone de chaque groupe méthylène (CH₂) dans Y, différent de R⁴, peut porter un ou deux substituants R³ ; et
Z est

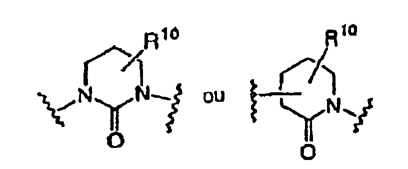

3. Composé de la revendication 2, dans lequel Y est choisi dans l'ensemble consistant en
-(CH₂)ₘ-, -(CH₂)ₘ-S-(CH₂)ₙ-, et -(CH₂)ₘ-NR₄-(CH₂)ₙ,
où l'atome de carbone de chaque groupe méthylène (CH₂) dans Y, différent de R⁴, peut porter un ou deux substituants R³ ;
m et n sont des nombres entiers de 0 à 3 ;
et v vaut 0.

4. Composé de la revendication 3, dans lequel R⁶, R⁷ et R⁸ représentent chacun un atome d'hydrogène et R⁵ est choisi dans l'ensemble consistant en
un atome d'hydrogène,
un groupe aryle,
un groupe alkyle en C₁₋₈,
un groupe aryl-C≡C-(CH₂)ₜ-,
un groupe aryl-(alkyle en C₁₋₆),
un groupe CH₂=CH-(CH₂)ₜ-, et
un groupe HC≡CH-(CH₂)ₜ-.

5. Composé de la revendication 4, dans lequel R⁹ représente un atome d'hydrogène.

6. Composé de la revendication 5, dans lequel R⁵, R⁶ et R⁸ représentent chacun un atome d'hydrogène et R⁷ est choisi dans l'ensemble consistant en
un atome d'hydrogène,
un groupe aryle,
un groupe (alkyle en C₁₋₈)carbonylamino,
un groupe (alkyle en C₁₋₈)sulfonylamino,
un groupe arylcarbonylamino,
un groupe arylsulfonylamino,
un groupe (alkyle en C₁₋₈)sulfonylamino-(alkyle en C₁₋₆),
un groupe arylsulfonylamino-(alkyle en C₁₋₆),
un groupe aryl-(alkyle en C₁₋₆)sulfonylamino,
un groupe aryl(alkyle en C₁₋₆)sulfonylamino-(alkyle en C₁₋₆),
un groupe (alcoxy en C₁₋₈)carbonylamino,
un groupe (alcoxy en C₁₋₈)carbonylamino-(alkyle en C₁₋₈),
un groupe aryloxycarbonylamino-(alkyle en C₁₋₈),
un groupe aryl-(alcoxy en C₁₋₈)carbonylamino,
un groupe aryl- (alcoxy en C₁₋₈)carbonylamino- (alkyle en C₁₋₈),
un groupe (alkyle en C₁₋₈)carbonylamino-(alkyle en C₁₋₆),
un groupe arylcarbonylamino-(alkyle en C₁₋₆),
un groupe aryl-(alkyle en C₁₋₆)carbonylamino,
un groupe aryl-(alkyle en C₁₋₆)carbonylamino- (alkyle en C₁₋₆),
un groupe aminocarbonylamino(alkyle en C₁₋₆),
un groupe (alkyle en C₁₋₈)ₚ-aminocarbonylamino,
un groupe (alkyle en C₁₋₈)ₚ-aminocarbonylamino (alkyle en C₁₋₆)
un groupe (aryl)ₚ-aminocarbonylamino(alkyle en C₁₋₆),
un groupe arylaminocarbonylamino,
un groupe ((aryl)-(alkyle en C₁₋₈))ₚ-aminocarbonylamino,
un groupe ((aryl)-(alkyle en C₁₋₈))ₚ-aminocarbonylamino-(alkyle en C₁₋₆),
un groupe aminosulfonylamino-(alkyle en C₁₋₆),
un groupe (alkyle en C₁₋₈)ₚ-aminosulfonylamino,
un groupe (alkyle en C₁₋₈)ₚ-aminosulfonylamino (alkyle en C₁₋₆),
un groupe (aryl)ₚ-aminosulfonylamino (alkyle en C₁₋₆),
un groupe ((aryl)-(alkyle en C₁₋₈))ₚ-aminosulfonylamino,
un groupe ((aryl)-(alkyle en C1-8))p-aminosulfonylaminoalkyle en C₁₋₆),
un groupe (alkyle en C₁₋₆)thiocarbonylamino,
un groupe (alkyle en C₁₋₆)thiocarbonylamino-(alkyle en C₁₋₆),
un groupe arylthiocarbonylamino(alkyle en C₁₋₆),
un groupe aryl-(alkyle en C₁₋₆)thiocarbonylamino, et
un groupe aryl-(alkyle en C₁₋₈))thiocarbonylamino-(alkyle en C₁₋₆).

7. Composé de la revendication 6, dans lequel R⁷ est choisi dans l'ensemble consistant en
un atome d'hydrogène,
un groupe aryle,
un groupe (alkyle en C₁₋₈)carbonylamino,
un groupe aryl-(alkyle en C₁₋₆)carbonylamino,
un groupe arylcarbonylamino,
un groupe (alkyle en C₁₋₈)sulfonylamino,
un groupe aryl(alkyle en C₁₋₆)sulfonylamino,
un groupe arylsulfonylamino,
un groupe (alcoxy en C₁₋₈)carbonylamino,
un groupe aryl(alcoxy en C₁₋₈)carbonylamino,
un groupe arylaminocarbonylamino,
un groupe (alkyle en C₁₋₈)ₚ-aminocarbonylamino,
un groupe ((aryl)-(alkyle en C₁₋₈))ₚ-aminocarbonylamino,
un groupe (alkyle en C₁₋₈)ₚ-aminosulonylamino, et
un groupe ((aryl)-(alkyle en C₁₋₈))ₚ-aminosulfonylamino.

8. Composé de la revendication 7, dans lequel R⁹ représente un atome d'hydrogène.

9. Composé de la revendication 3, choisi dans l'ensemble consistant en :
3(S)-(2,3-dihydro-benzofuran-6-yl)-3-{2-oxo-3-[3-(5,6,7,8-tétrahydro[1,8]naphtyridin-2-yl)-propyl]-tétrahydropyrimidin-1-yl}-propionate d'éthyle ;
3(S)-(3-fluorophényl)-3-(2-oxo-3(S ou R)-[3-(5,6,7,8-tétrahydro[1,8]naphtyridin-2-yl)-propyl]-pipéridin-1-yl)-propionate d'éthyle ;
3(S)-(3-fluorophényl)-3-(2-oxo-3(R ou S)-[3-(5,6,7,8-tétrahydro[1,8]naphtyridin-2-yl)-propyl]-pipéridin-1-yl)-propionate d'éthyle ;
acide 3(S)-(2,3-dihydro-benzofuran-6-yl)-3-{2-oxo-3-[3-(5,-6,7,8-tétrahydro[1,8]naphtyridin-2-yl)-propyl]-tétrahydropyrimidin-1-yl}-propionique ;
acide 3(S)-(3-fluorophényl)-3-(2-oxo-3(S ou R)-[3-(5,6,7,8-tétrahydro[1,8]naphtyridin-2-yl)-propyl]-pipéridin-1-yl)-propionique ;
acide 3(S)-(3-fluorophényl)-3-(2-oxo-3(R ou S)-[3-(5,6,7,8-tétrahydro[1,8]naphtyridin-2-yl)-propyl]-pipéridin-1-yl)-propionique ;
et leurs sels pharmaceutiquement acceptables.

10. Composé de la revendication 9, choisi dans l'ensemble consistant en
acide 3(S)-(2,3-dihydro-benzofuran-6-yl)-3-{2-oxo-3-[3-(5, 6,7,8-tétrahydro[1,8]naphtyridin-2-yl)-propyl]-tétrahydropyrimidin-1-yl}-propionique ;
acide 3(S)-(3-fluorophényl)-3-(2-oxo-3(S ou R)-[3-(5,6,7,8-tétrahydro[1,8]naphtyridin-2 - yl) -propyl]-pipéridin-1-yl) - propionique ;
acide 3(S)-(3-fluorophényl)-3-(2-oxo-3(R ou S)-[3-(5,6,7,8-tétrahydro[1,8]naphtyridin-2-yl)-propyl]-pipéridin-1-yl)-propionique ;
et leurs sels pharmaceutiquement acceptables.

11. Composition pharmaceutique comprenant un composé selon l'une quelconque des revendications précédentes et un support pharmaceutiquement acceptable.

12. Procédé de préparation d'une composition pharmaceutique comprenant la combinaison d'un composé selon l'une quelconque des revendications 1 à 10 et d'un support pharmaceutiquement acceptable.

13. Composition de la revendication 11, qui comprend en outre un ingrédient actif choisi dans l'ensemble consistant en
a) un bisphosphonate organique ou l'un de ses sels ou esters pharmaceutiquement acceptables,
b) un modulateur de récepteur d'oestrogène,
c) un agent cytotoxique/antiprolifératif,
d) un inhibiteur de matrice métalloprotéinase,
e) un inhibiteur de facteurs de croissance dérivé de l'épiderme, dérivé des fibroblastes ou dérivé des plaquettes,
f) un inhibiteur de VEGF,
g) un inhibiteur de Flk-1/KDR, Flt-1, Tck/Tie-2 ou Tie-1,
h) un inhibiteur de cathepsine K, et
i) un inhibiteur de prénylation, tel qu'un inhibiteur de farnésyl transférase ou un inhibiteur géranylgéranyl transférase ou un inhibiteur double de farnésyl/géranylgéranyl transférase ; et des mélanges de ceux-ci.

14. Composition de la revendication 13 dans laquelle ledit ingrédient actif est choisi dans l'ensemble consistant en
a) un bisphosphonate organique ou l'un de ses sels ou esters pharmaceutiquement acceptables,
b) un modulateur de récepteur d'oestrogène,
c) un inhibiteur de cathepsine K ;
et des mélanges de ceux-ci.

15. Composition de la revendication 14, dans laquelle ledit bisphosphonate organique ou l'un de ses sels ou esters pharmaceutiquement acceptables est l'alendronate de monosodium trihydraté.

16. Composition de la revendication 13, dans laquelle ledit ingrédient actif choisi dans l'ensemble consistant en
a) un agent cytotoxique/antiprolifératif,
b) un inhibiteur de matrice métalloprotéinase,
c) un inhibiteur de facteurs de croissance dérivé de l'épiderme, dérivé des fibroblastes ou dérivé des plaquettes,
d) un inhibiteur de VEGF,
e) un inhibiteur de Flk-1/KDR, Flt-1, Tck/Tie-2 ou Tie-1 ;
et des mélanges de ceux-ci.

17. Utilisation d'un composé selon l'une quelconque des revendications 1-10 pour la fabrication d'un médicament pour induire un effet antagoniste de récepteur d'intégrine chez un mammifère.

18. Utilisation selon la revendication 17, dans laquelle l'effet antagoniste de récepteur d'intégrine est un effet antagoniste d'αvβ3.

19. Utilisation selon la revendication 18, dans laquelle l'effet antagoniste d'αvβ3 est choisi dans l'ensemble consistant en une inhibition de la résorption osseuse, d'une resténose, d'une angiogénèse, d'une rétinopathie diabétique, d'une dégénérescence maculaire, d'une inflammation, d'une maladie virale et d'une croissance tumorale.

20. Utilisation selon la revendication 19, dans laquelle l'effet antagoniste d'αvβ3 est l'inhibition de la résorption osseuse.

21. Utilisation selon la revendication 17, dans laquelle l'effet antagoniste de récepteur d'intégrine est un effet antagoniste d'αvβ5.

22. Utilisation selon la revendication 21, dans laquelle l'effet antagoniste d'αvβ5 est choisi dans l'ensemble consistant en une inhibition d'une resténose, d'une angiogénèse, d'une rétinopathie diabétique, d'une dégénérescence maculaire, d'une inflammation, et d'une croissance tumorale.

23. Utilisation selon la revendication 17, dans laquelle l'effet antagoniste de récepteur d'intégrine est un effet antagoniste double d'αvβ3/αvβ5.

24. Utilisation selon la revendication 21, dans laquelle l'effet antagoniste double d'αvβ3/αvβ5 est choisi dans l'ensemble consistant en une inhibition de la résorption osseuse, d'une resténose, d'une angiogénèse, d'une rétinopathie diabétique, d'une dégénérescence maculaire, d'une inflammation, d'une maladie virale et d'une croissance tumorale.

25. Utilisation selon la revendication 17, dans laquelle l'effet antagoniste de récepteur d'intégrine est un effet antagoniste d'αvβ6.

26. Utilisation selon la revendication 25, dans laquelle l'effet antagoniste d'αvβ6 est choisi dans l'ensemble consistant en une angiogénèse, une réponse inflammatoire, et la cicatrisation de plaies

27. Composition de la revendication 11 pour une utilisation dans l'induction d'un effet antagoniste de récepteur d'intégrine chez un mammifère.

28. Composition de la revendication 11 pour une utilisation dans le traitement ou la prévention d'une condition médiée par un antagonisme de récepteur d'intégrine chez un mammifère.

29. Composition de la revendication 11 pour une utilisation dans l'inhibition de la résorption osseuse chez un mammifère.

30. Composition de la revendication 14 pour une utilisation dans l'inhibition de la résorption osseuse chez un mammifère.

31. Composition de la revendication 16 pour une utilisation dans le traitement d'une croissance tumorale chez un mammifère.

32. Composé de la revendication 1 pour une utilisation dans le traitement d'une croissance tumorale chez un mammifère en combinaison avec une radiothérapie.
